# EUROPEAN PATENT SPECIFICATION

(11) **EP 1 005 365 B1**
(45) Date of publication and mention of the grant of the patent: **02.01.2008**
(21) Application number: 98903550.6
(22) Date of filing: 15.01.1998
(51) Int. Cl.: A61K 39/04, C07K 14/35, C12N 15/31

(54) **EPITOPES OF AN EXTRACELLULAR ANTIGEN**
EPITOPEN EINES EXTRAZELLULÄREN ANTIGENS
EPITOPES D'UN ANTIGENE EXTRACELLULAIRE

(30) Priority: 21.01.1997 US 786533
(43) Date of publication of application: 07.06.2000
(73) Proprietor: The Regents of the University of California, Oakland, CA 94612-3550 (US)
(72) Inventor: HORWITZ, Marcus, A., Los Angeles, CA 90077 (US); HARTH, Günter, Los Angeles, CA 90025 (US); LEE, Bai-Yu, Los Angeles, CA 90064 (US)
(74) Representative: Gowshall, Jonathan Vallance
(86) International application number: PCT/US1998/000942
(87) International publication number: WO 1998/031388

(56) References cited:
- EP-A- 0 419 355
- WO-A-95/14713
- WO-A-96/37219
- US-A- 5 504 005
- US-A- 5 571 515
- BORREMANS M ET AL: "Cloning, sequence determination, and expression of a 32-kilodalton-protein gene of Mycobacterium tuberculosis" INFECTION AND IMMUNITY, vol. 57, no. 10, October 1989 (1989-10), pages 3123-3130, XP000572207 ISSN: 0019-9567
- GATELY M K & MULQUEEN M J: "Interleukin-12: potential clinical applications in the treatment and prevention of infectious diseases." DRUGS, vol. 52, no. Suppl. 2, 1996, pages 18-25 + 25 - 26, XP008009664 ISSN: 0012-6667
- HARTH G ET AL: "High-level heterologous expression and secretion in rapidly growing nonpathogenic mycobacteria of four major Mycobacterium tuberculosis extracellular proteins considered to be leading vaccine candidates and drug targets" INFECTION AND IMMUNITY, vol. 65, no. 6, June 1997 (1997-06), pages 2321-2328, XP002185377 ISSN: 0019-9567
- GUPTA R.K. et al.: "Adjuvants for Human Vaccines-Current Status, Problems and Future Prospects", VACCINE, 1995, Vol. 13, No. 14, pages 1263-1276, XP002913279
- BORREMANS M. et al.: "Cloning, Sequence Determination and Expression of a 32- Kilodalton-Protein Gene of Mycobacterium tuberculosis", INFECTION AND IMMUNITY, October 1989, Vol. 57, No. 10, pages 3123-3130, XP002913280

### SEQUENCE LISTING

(1) GENERAL INFORMATION:
   (i)APPLICANTS: Horwitz, Marcus A.; Harth, Günter; and Lee, Bai-Yu
   (ii) TITLE OF INVENTION: Abundant Extracellular Products and Methods for Their Production and Use
   (iii) NUMBER OF SEQUENCES: 159
   (iv) CORRESPONDENCE ADDRESS:
      (A) ADDRESSEE: Kurt A. MacLean
      (B) STREET: 2029 Century Park East, Suite 3800
      (C) CITY: Los Angeles
      (D) STATE: California
      (E) COUNTRY: U.S.A.
      (F) ZIP: 90067-3024
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: US 08/568,357
      (B) FILING DATE: 06-DEC-1995
   (vii) PRIOR APPLICATION DATA:
      (A) APPLICATION NUMBER: US 08/551,149
      (B) FILING DATE: 31-OCT-1995
   (vii) PRIOR APPLICATION DATA:
      (A) APPLICATION NUMBER: US 08/447,398
      (B) FILING DATE: 23-MAY-1995
   (vii) PRIOR APPLICATION DATA:
      (A) APPLICATION NUMBER: US 08/289,667
      (B) FILING DATE: 12-AUG-1994
   (vii) PRIOR APPLICATION DATA:
      (A) APPLICATION NUMBER: US 08/156,358 (B) FILING DATE: 23-NOV-1993
   (viii) ATTORNEY/AGENT INFORMATION:
      (A) NAME: MacLean, Kurt A.
      (B) REGISTRATION NUMBER: 31,118
      (C) REFERENCE/DOCKET NUMBER: 118-119
   (ix) TELECOMMUNICATION INFORMATION:
      (A) TELEPHONE: (714) 263-8250
      (B) TELEFAX: (714) 263-8260
(2) INFORMATION FOR SEQ ID NO:1:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:1:
   Asn Ser Lys Ser Val
   1               5
(2) INFORMATION FOR SEQ ID NO:2:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:2:
   Thr Asp Arg Val Ser
   1               5
(2) INFORMATION FOR SEQ ID NO:3:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:3:
   Ala Arg Ala Val Gly
   1               5
(2) INFORMATION FOR SEQ ID NO:4:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:4:
   Thr Glu Lys Thr Pro
   1               5
(2) INFORMATION FOR SEQ ID NO:5:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:5:
   Asp Pro Glu Pro Ala
   1               5
(2) INFORMATION FOR SEQ ID NO:6:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:6:
   Phe Ser Arg Pro Gly
   1               5
(2) INFORMATION FOR SEQ ID NO:7:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:7:
   Phe Ser Arg Pro Gly
   1               5
(2) INFORMATION FOR SEQ ID NO:8:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:8:
   Phe Ser Arg Pro Gly
   1               5
(2) INFORMATION FOR SEQ ID NO:9:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:9:
   Ala Pro Tyr Glu Asn
   1               5
(2) INFORMATION FOR SEQ ID NO:10:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:10:
   Ala Pro Lys Thr Tyr
   1               5
(2) INFORMATION FOR SEQ ID NO:11:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:11:
   Ala Glu Thr Tyr Leu
   1               5
(2) INFORMATION FOR SEQ ID NO:12:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:12:
   Ala Tyr Pro Ile Thr
   1               5
(2) INFORMATION FOR SEQ ID NO:13:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:13:
   Ala Asp Pro Arg Leu
   1               5
(2) INFORMATION FOR SEQ ID NO:14:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:14:
   Phe Asp Thr Arg Leu
   1               5
(2) INFORMATION FOR SEQ ID NO:15:
   (i) SEQUENCE CHARACTERISTICS:
      (A) LENGTH: 40 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:15:
(2) INFORMATION FOR SEQ ID NO:16:
   (i) SEQUENCE CHARACTERISTICS:
      (A) LENGTH: 40 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:16:
(2) INFORMATION FOR SEQ ID NO:17:
   (i) SEQUENCE CHARACTERISTICS:
      (A) LENGTH: 22 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:17:
(2) INFORMATION FOR SEQ ID NO:18:
   (i) SEQUENCE CHARACTERISTICS:
      (A) LENGTH: 48 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:18:
(2) INFORMATION FOR SEQ ID NO:19:
   (i) SEQUENCE CHARACTERISTICS:
      (A) LENGTH: 30 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:19:
(2) INFORMATION FOR SEQ ID NO:20:
   (i) SEQUENCE CHARACTERISTICS:
      (A) LENGTH: 30 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:20:
(2) INFORMATION FOR SEQ ID NO:21:
   (i) SEQUENCE CHARACTERISTICS:
      (A) LENGTH: 47 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:21:
(2) INFORMATION FOR SEQ ID NO:22:
   (i) SEQUENCE CHARACTERISTICS:
      (A) LENGTH: 47 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:22:
(2) INFORMATION FOR SEQ ID NO:23:
   (i) SEQUENCE CHARACTERISTICS:
      (A) LENGTH: 22 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:23:
(2) INFORMATION FOR SEQ ID NO:24:
   (i) SEQUENCE CHARACTERISTICS:
      (A) LENGTH: 13 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:24:
   Ala Pro Lys Thr Tyr Xaa Glu Glu Leu Lys Gly Thr Asp
   1               5                   10
(2) INFORMATION FOR SEQ ID NO:25:
   (i) SEQUENCE CHARACTERISTICS:
      (A) LENGTH: 60 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:25:
(2) INFORMATION FOR SEQ ID NO:26:
   (i) SEQUENCE CHARACTERISTICS:
      (A) LENGTH: 60 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:26:
(2) INFORMATION FOR SEQ ID NO:27:
   (i) SEQUENCE CHARACTERISTICS:
      (A) LENGTH: 40 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:27:
(2) INFORMATION FOR SEQ ID NO:28:
   (i) SEQUENCE CHARACTERISTICS:
      (A) LENGTH: 40 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:28:
(2) INFORMATION FOR SEQ ID NO:29:
   (i) SEQUENCE CHARACTERISTICS:
      (A) LENGTH: 22 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:29:
(2) INFORMATION FOR SEQ ID NO:30:
   (i) SEQUENCE CHARACTERISTICS:
      (A) LENGTH: 30 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:30:
(2) INFORMATION FOR SEQ ID NO:31:
   (i) SEQUENCE CHARACTERISTICS:
      (A) LENGTH: 20 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:31:
(2) INFORMATION FOR SEQ ID NO:32:
   (i) SEQUENCE CHARACTERISTICS:
      (A) LENGTH: 6 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:32:
   Ala Arg Ala Val Gly Ile
   1 5
(2) INFORMATION FOR SEQ ID NO:33:
   (i) SEQUENCE CHARACTERISTICS:
      (A) LENGTH: 8 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:33:
   Thr Asp Arg Val Ser Val Gly Asn
   1 5
(2) INFORMATION FOR SEQ ID NO:34:
   (i) SEQUENCE CHARACTERISTICS:
      (A) LENGTH: 22 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:34:
(2) INFORMATION FOR SEQ ID NO:35:
   (i) SEQUENCE CHARACTERISTICS:
      (A) LENGTH: 978 base pairs
      (B) TYPE: nucleic acid
      (C) STRANDEDNESS: single
      (D) TOPOLOGY: not relevant
   (ii) MOLECULE TYPE: DNA (genomic)
   (xi) SEQUENCE DESCRIPTION: SEQ ID NO:35:
   ATGACAGACG TGAGCCGAAA GATTCGAGCT TGGGGACGCC GATTGATGAT 50
   CGGCACGGCA GCGGCTGTAG TCCTTCCGGG CCTGGTGGGG CTTGCCGGCG 100
   GAGCGGCAAC CGCGGGCGCG TTCTCCCGGC CGGGGCTGCC GGTCGAGTAC 150
   CTGCAGGTGC CGTCGCCGTC GATGGGCCGC GACATCAAGG TTCAGTTCCA 200
   GAGCGGTGGG AACAACTCAC CTGCGGTTTA TCTGCTCGAC GGCCTGCGCG 250
   CCCAAGACGA CTACAACGGC TGGGATATCA ACACCCCGGC GTTCGAGTGG 300
   TACTACCAGT CGGGACTGTC GATAGTCATG CCGGTCGGCG GGCAGTCCAG 350
   CTTCTACAGC GACTGGTACA GCCCGGCCTG CGGTAAGGCT GGCTGCCAGA 400
   CTTACAAGTG GGAAACCTTC CTGACCAGCG AGCTGCCGCA ATGGTTGTCC 450
   GCCAACAGGG CCGTGAAGCC CACCGGCAGC GCTGCAATCG GCTTGTCGAT 500
   GGCCGGCTCG TCGGCAATGA TCTTGGCCGC CTACCACCCC CAGCAGTTCA 550
   TCTACGCCGG CTCGCTGTCG GCCCTGCTGG ACCCCTCTCA GGGGATGGGG 600
   CCTAGCCTGA TCGGCCTCGC GATGGGTGAC GCCGGCGGTT ACAAGGCCGC 650
   AGACATGTGG GGTCCCTCGA GTGACCCGGC ATGGGAGCGC AACGACCCTA 700
   CGCAGCAGAT CCCCAAGCTG GTCGCAAACA ACACCCGGCT ATGGGTTTAT 750
   TGCGGGAACG GCACCCCGAA CGAGTTGGGC GGTGCCAACA TACCCGCCGA 800
   GTTCTTGGAG AACTTCGTTC GTAGCAGCAA CCTGAAGTTC CAGGATGCGT 850
   ACAACGCCGC GGGCGGGCAC AACGCCGTGT TCAACTTCCC GCCCAACGGC 900
   ACGCACAGCT GGGAGTACTG GGGCGCTCAG CTCAACGCCA TGAAGGGTGA 950
   CCTGCAGAGT TCGTTAGGCG CCGGCTGA 978
(2) INFORMATION FOR SEQ ID NO:36:
   (i) SEQUENCE CHARACTERISTICS:
      (A) LENGTH: 1017 base pairs
      (B) TYPE: nucleic acid
      (C) STRANDEDNESS: single
      (D) TOPOLOGY: not relevant
   (ii) MOLECULE TYPE: DNA (genomic)
   (xi) SEQUENCE DESCRIPTION: SEQ ID NO:36:
   ATGCAGCTTG TTGACAGGGT TCGTGGCGCC GTCACGGGTA TGTCGCGTCG   50
   ACTCGTGGTC GGGGCCGTCG GCGCGGCCCT AGTGTCGGGT CTGGTCGGCG  100
   CCGTCGGTGG CACGGCGACC GCGGGGGCAT TTTCCCGGCC GGGCTTGCCG  150
   GTGGAGTACC TGCAGGTGCC GTCGCCGTCG ATGGGCCGTG ACATCAAGGT  200
   CCAATTCCAA AGTGGTGGTG CCAACTCGCC CGCCCTGTAC CTGCTCGACG  250
   GCCTGCGCGC GCAGGACGAC TTCAGCGGCT GGGACATCAA CACCCCGGCG  300
   TTCGAGTGGT ACGACCAGTC GGGCCTGTCG GTGGTCATGC CGGTGGGTGG  350
   CCAGTCAAGC TTCTACTCCG ACTGGTACCA GCCCGCCTGC GGCAAGGCCG  400
   GTTGCCAGAC TTACAAGTGG GAGACCTTCC TGACCAGCGA GCTGCCGGGG  450
   TGGCTGCAGG CCAACAGGCA CGTCAAGCCC ACCGGAAGCG CCGTCGTCGG  500
   TCTTTCGATG GCTGCTTCTT CGGCGCTGAC GCTGGCGATC TATCACCCCC  550
   AGCAGTTCGT CTACGCGGGA GCGATGTCGG GCCTGTTGGA CCCCTCCCAG  600
   GCGATGGGTC CCACCCTGAT CGGCCTGGCG ATGGGTGACG CTGGCGGCTA  650
   CAAGGCCTCC GACATGTGGG GCCCGAAGGA GGACCCGGCG TGGCAGCGCA  700
   ACGACCCGCT GTTGAACGTC GGGAAGCTGA TCGCCAACAA CACCCGCGTC  750
   TGGGTGTACT GCGGCAACGG CAAGCCGTCG GATCTGGGTG GCAACAACCT  800
   GCCGGCCAAG TTCCTCGAGG GCTTCGTGCG GACCAGCAAC ATCAAGTTCC  850
   AAGACGCCTA CAACGCCGGT GGCGGCCACA ACGGCGTGTT CGACTTCCCG  900
   GACAGCGGTA CGCACAGCTG GGAGTACTGG GGCGCGCAGC TCAACGCTAT  950
   GAAGCCCGAC CTGCAACGGG CACTGGGTGC CACGCCCAAC ACCGGGCCCG 1000
   CGCCCCAGGG CGCCTAG   1017
(2) INFORMATION FOR SEQ ID NO:92:
   (i) SEQUENCE CHARACTERISTICS:
      (A) LENGTH: 480 base pairs
      (B) TYPE: nucleic acid
      (C) STRANDEDNESS: single
      (D) TOPOLOGY: not relevant
   (ii) MOLECULE TYPE: DNA (genomic)
   (xi) SEQUENCE DESCRIPTION: SEQ ID NO:92:
   ATGAAGCTCA CCACAATGAT CAAGACGGCA GTAGCGGTCG TGGCCATGGC 50
   GGCCATCGCG ACCTTTGCGG CACCGGTCGC GTTGGCTGCC TATCCCATCA 100
   CCGGAAAACT TGGCAGTGAG CTAACGATGA CCGACACCGT TGGCCAAGTC 150
   GTGCTCGGCT GGAAGGTCAG TGATCTCAAA TCCAGCACGG CAGTCATCCC 200
   CGGCTATCCG GTGGCCGGCC AGGTCTGGGA GGCCACTGCC ACGGTCAATG 250
   CGATTCGCGG CAGCGTCACG CCCGCGGTCT CGCAGTTCAA TGCCCGCACC 300
   GCCGACGGCA TCAACTACCG GGTGCTGTGG CAAGCCGCGG GCCCCGACAC 350
   CATTAGCGGA GCCACTATCC CCCAAGGCGA ACAATCGACC GGCAAAATCT 400
   ACTTCGATGT CACCGGCCCA TCGCCAACCA TCGTCGCGAT GAACAACGGC 450
   ATGGAGGATC TGCTGATTTG GGAGCCGTAG 480
(2) INFORMATION FOR SEQ ID NO:93:
   (i) SEQUENCE CHARACTERISTICS:
      (A) LENGTH: 1437 base pairs
      (B) TYPE: nucleic acid
      (C) STRANDEDNESS: single
      (D) TOPOLOGY: not relevant
   (ii) MOLECULE TYPE: DNA (genomic)
   (xi) SEQUENCE DESCRIPTION: SEQ ID NO:93:
   GTGACGGAAA AGACGCCCGA CGACGTCTTC AAACTTGCCA AGGACGAGAA 50
   GGTCGAATAT GTCGACGTCC GGTTCTGTGA CCTGCCTGGC ATCATGCAGC 100
   ACTTCACGAT TCCGGCTTCG GCCTTTGACA AGAGCGTGTT TGACGACGGC 150
   TTGGCCTTTG ACGGCTCGTC GATTCGCGGG TTCCAGTCGA TCCACGAATC 200
   CGACATGTTG CTTCTTCCCG ATCCCGAGAC GGCGCGCATC GACCCGTTCC 250
   GCGCGGCCAA GACGCTGAAT ATCAACTTCT TTGTGCACGA CCCGTTCACC 300
   CTGGAGCCGT ACTCCCGCGA CCCGCGCACC ATCGCCCGCA AGGCCGAGAA 350
   CTACCTGATC AGCACTGGCA TCGCCGACAC CGCATACTTC GGCGCCGAGG 400
   CCGAGTTCTA CATTTTCGAT TCGGTGAGCT TCGACTCGCG CGCCAACGGC 450
   TCCTTCTACG AGGTGGACGC CATCTCGGGG TGGTGGAACA CCGGCGCGGC 500
   GACCGAGGCC GACGGCAGTC CCAACCGGGG CTACAAGGTC CGCCACAAGG 550
   GCGGGTATTT CCCAGTGGCC CCCAACGACC AATACGTCGA CCTGCGCGAC 600
   AAGATGCTGA CCAACCTGAT CAACTCCGGC TTCATCCTGG AGAAGGGCCA 650
   CCACGAGGTG GGCAGCGGCG GACAGGCCGA GATCAACTAC CAGTTCAATT 700
   CGCTGCTGCA CGCCGCCGAC GACATGCAGT TGTACAAGTA CATCATCAAG 750
   AACACCGCCT GGCAGAACGG CAAAACGGTC ACGTTCATGC CCAAGCCGCT 800
   GTTCGGCGAC AACGGGTCCG GCATGCACTG TCATCAGTCG CTGTGGAAGG 850
   ACGGGGCCCC GCTGATGTAC GACGAGACGG GTTATGCCGG TCTGTCGGAC 900
   ACGGCCCGTC ATTACATCGG CGGCCTGTTA CACCACGCGC CGTCGCTGCT 950
   GGCCTTCACC AACCCGACGG TGAACTCCTA CAAGCGGCTG GTTCCCGGTT 1000
   ACGAGGCCCC GATCAACCTG GTCTATAGCC AGCGCAACCG GTCGGCATGC 1050
   GTGCGCATCC CGATCACCGG CAGCAACCCG AAGGCCAAGC GGCTGGAGTT 1100
   CCGAAGCCCC GACTCGTCGG GCAACCCGTA TCTGGCGTTC TCGGCCATGC 1150
   TGATGGCAGG CCTGGACGGT ATCAAGAACA AGATCGAGCC GCAGGCGCCC 1200
   GTCGACAAGG ATCTCTACGA GCTGCCGCCG GAAGAGGCCG CGAGTATCCC 1250
   GCAGACTCCG ACCCAGCTGT CAGATGTGAT CGACCGTCTC GAGGCCGACC 1300
   ACGAATACCT CACCGAAGGA GGGGTGTTCA CAAACGACCT GATCGAGACG 1350
   TGGATCAGTT TCAAGCGCGA AAACGAGATC GAGCCGGTCA ACATCCGGCC 1400
   GCATCCCTAC GAATTCGCGC TGTACTACGA CGTTTAA 1437
(2) INFORMATION FOR SEQ ID NO:94:
   (i) SEQUENCE CHARACTERISTICS:
      (A) LENGTH: 687 base pairs
      (B) TYPE: nucleic acid
      (C) STRANDEDNESS: single
      (D) TOPOLOGY: not relevant
   (ii) MOLECULE TYPE: DNA (genomic)
   (xi) SEQUENCE DESCRIPTION: SEQ ID NO:94:
   GTGCGCATCA AGATCTTCAT GCTGGTCACG GCTGTCGTTT TGCTCTGTTG 50
   TTCGGGTGTG GCCACGGCCG CGCCCAAGAC CTACTGCGAG GAGTTGAAAG 100
   GCACCGATAC CGGCCAGGCG TGCCAGATTC AAATGTCCGA CCCGGCCTAC 150
   AACATCAACA TCAGCCTGCC CAGTTACTAC CCCGACCAGA AGTCGCTGGA 200
   AAATTACATC GCCCAGACGC GCGACAAGTT CCTCAGCGCG GCCACATCGT 250
   CCACTCCACG CGAAGCCCCC TACGAATTGA ATATCACCTC GGCCACATAC 300
   CAGTCCGCGA TACCGCCGCG TGGTACGCAG GCCGTGGTGC TCAAGGTCTA 350
   CCAGAACGCC GGCGGCACGC ACCCAACGAC CACGTACAAG GCCTTCGATT 400
   GGGACCAGGC CTATCGCAAG CCAATCACCT ATGACACGCT GTGGCAGGCT 450
   GACACCGATC CGCTGCCAGT CGTCTTCCCC ATTGTGCAAG GTGAACTGAG 500
   CAAGCAGACC GGACAACAGG TATCGATAGC GCCGAATGCC GGCTTGGACC 550
   CGGTGAATTA TCAGAACTTC GCAGTCACGA ACGACGGGGT GATTTTCTTC 600
   TTCAACCCGG GGGAGTTGCT GCCCGAAGCA GCCGGCCCAA CCCAGGTATT 650
   GGTCCCACGT TCCGCGATCG ACTCGATGCT GGCCTAG 687
(2) INFORMATION FOR SEQ ID NO:95:
   (i) SEQUENCE CHARACTERISTICS:
      (A) LENGTH: 900 base pairs
      (B) TYPE: nucleic acid
      (C) STRANDEDNESS: single
      (D) TOPOLOGY: not relevant
   (ii) MOLECULE TYPE: DNA (genomic)
   (xi) SEQUENCE DESCRIPTION: SEQ ID NO:95:
   ATGAAGGGTC GGTCGGCGCT GCTGCGGGCG CTCTGGATTG CCGCACTGTC 50
   ATTCGGGTTG GGCGGTGTCG CGGTAGCCGC GGAACCCACC GCCAAGGCCG 100
   CCCCATACGA GAACCTGATG GTGCCGTCGC CCTCGATGGG CCGGGACATC 150
   CCGGTGGCCT TCCTAGCCGG TGGGCCGCAC GCGGTGTATC TGCTGGACGC 200
   CTTCAACGCC GGCCCGGATG TCAGTAACTG GGTCACCGCG GGTAACGCGA 250
   TGAACACGTT GGCGGGCAAG GGGATTTCGG TGGTGGCACC GGCCGGTGGT 300
   GCGTACAGCA TGTACACCAA CTGGGAGCAG GATGGCAGCA AGCAGTGGGA 350
   CACCTTCTTG TCCGCTGAGC TGCCCGACTG GCTGGCCGCT AACCGGGGCT 400
   TGGCCCCCGG TGGCCATGCG GCCGTTGGCG CCGCTCAGGG CGGTTACGGG 450
   GCGATGGCGC TGGCGGCCTT CCACCCCGAC CGCTTCGGCT TCGCTGGCTC 500
   GATGTCGGGC TTTTTGTACC CGTCGAACAC CACCACCAAC GGTGCGATCG 550
   CGGCGGGCAT GCAGCAATTC GGCGGTGTGG ACACCAACGG AATGTGGGGA 600
   GCACCACAGC TGGGTCGGTG GAAGTGGCAC GACCCGTGGG TGCATGCCAG 650
   CCTGCTGGCG CAAAACAACA CCCGGGTGTG GGTGTGGAGC CCGACCAACC 700
   CGGGAGCCAG CGATCCCGCC GCCATGATCG GCCAAGCCGC CGAGGCGATG 750
   GGTAACAGCC GCATGTTCTA CAACCAGTAT CGCAGCGTCG GCGGGCACAA 800
   CGGACACTTC GACTTCCCAG CCAGCGGTGA CAACGGCTGG GGCTCGTGGG 850
   CGCCCCAGCT GGGCGCTATG TCGGGCGATA TCGTCGGTGC GATCCGCTAA 900
(2) INFORMATION FOR SEQ ID NO:96:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO: 96:
      F S R P G L P V E Y L Q V P S
(2) INFORMATION FOR SEQ ID NO:97:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:97:
      L P V E Y L Q V P S P S M G R
(2) INFORMATION FOR SEQ ID NO:98:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:98:
      L Q V P S P S M G R D I K V Q
(2) INFORMATION FOR SEQ ID NO:99:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:99:
      P S M G R D I K V Q F Q S G G
(2) INFORMATION FOR SEQ ID NO:100:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:100:
      D I K V Q F Q S G G A N S P
(2) INFORMATION FOR SEQ ID NO:101:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:101:
      F Q S G G A N S P A L Y L L D
(2) INFORMATION FOR SEQ ID NO:102:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:102:
      A N S P A L Y L L D G L R A Q
(2) INFORMATION FOR SEQ ID NO:103:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:103:
      L Y L L D G L R A Q D D F S G
(2) INFORMATION FOR SEQ ID NO:104:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:104:
      G L R A Q D D F S G W D I N T
(2) INFORMATION FOR SEQ ID NO:105:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:105:
      D D F S G W D I N T P A F E W
(2) INFORMATION FOR SEQ ID NO:106:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:106:
      W D I N T P A F E W Y D Q S G
(2) INFORMATION FOR SEQ ID NO:107:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:107:
      P A F E W Y D Q S G L S V V M
(2) INFORMATION FOR SEQ ID NO:108:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:108:
      Y D Q S G L S V V M P V G G Q
(2) INFORMATION FOR SEQ ID NO:109:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO : 109 :
      L S V V M P V G G Q S S F Y S
(2) INFORMATION FOR SEQ ID NO:110:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:110:
      P V G G Q S S F Y S D W Y Q P
(2) INFORMATION FOR SEQ ID NO:111:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:111:
      S S F Y S D W Y Q P A C G K A
(2) INFORMATION FOR SEQ ID NO:112:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:112:
      D W Y Q P A C G K A G C Q T Y
(2) INFORMATION FOR SEQ ID NO:113:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:113:
      A C G K A G C Q T Y K W E T F
(2) INFORMATION FOR SEQ ID NO:114:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis* (B): STRAIN: Erdman
   (xi) SEQUENCE DESCRIPTION: SEQ ID NO:114:
      G C Q T Y K W E T F L T S E LK Y K W E T F
(2) INFORMATION FOR SEQ ID NO:115:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:115:
      K W E T F L T S E L P G W L Q
(2) INFORMATION FOR SEQ ID NO:116:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:116:
      L T S E L P G W L Q A N R H V
(2) INFORMATION FOR SEQ ID NO:117:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:117:
      P G W L Q A N R H V K P T G S
(2) INFORMATION FOR SEQ ID NO:118:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:118:
      A N R H V K P T G S A V V G LK
(2) INFORMATION FOR SEQ ID NO:119:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:119:
      A P T G S A V V G L S M A A S
(2) INFORMATION FOR SEQ ID NO:120:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:120:
      A V V G L S M A A S S A L T L
(2) INFORMATION FOR SEQ ID NO:121:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:121:
      S M A A S S A L T L A I Y H P
(2) INFORMATION FOR SEQ ID NO:122:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:122:
      S A L T L A I Y H P Q Q F V Y
(2) INFORMATION FOR SEQ ID NO:123:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:123:
      A I Y H P Q Q F V Y A G A M S
(2) INFORMATION FOR SEQ ID NO:124:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:124:
      Q Q F V Y A G A M S G L L D P
(2) INFORMATION FOR SEQ ID NO:125:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:125:
      A G A M S G L L D P S Q A M G
(2) INFORMATION FOR SEQ ID NO:126:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:126:
      G L L D P S Q A M G P T L I G
(2) INFORMATION FOR SEQ ID NO:127:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:127:
      S Q A M G P T L I G L A M G DP
(2) INFORMATION FOR SEQ ID NO:128:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:128:
      S T L I G L A M G D A G G Y K
(2) INFORMATION FOR SEQ ID NO:129:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:129:
      L A M G D A G G Y K A S D M W
(2) INFORMATION FOR SEQ ID NO:130:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID N0:130:
      A G G Y K A S D M W G P K E D
(2) INFORMATION FOR SEQ ID NO:131:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:131:
      A S D M W G P K E D P A W Q R
(2) INFORMATION FOR SEQ ID NO:132:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:132:
      G P K E D P A W Q R N D P L L
(2) INFORMATION FOR SEQ ID NO:133:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:133:
      P A W Q R N D P L L N V G K L
(2) INFORMATION FOR SEQ ID NO:134:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:134:
      N D P L L N V G K L I A N N T
(2) INFORMATION FOR SEQ ID NO:135:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:135:
      N V G K L I A N N T R V W V Y
(2) INFORMATION FOR SEQ ID NO:136:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:136:
      I A N N T R V W V Y C G N G K
(2) INFORMATION FOR SEQ ID NO:137:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:137:
      R V W V Y C G N G K P S D L G
(2) INFORMATION FOR SEQ ID NO:138:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:138:
      C G N G K P S D L G G N N L P
(2) INFORMATION FOR SEQ ID NO:139:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:139:
      P S D L G G N N L P A K F L E
(2) INFORMATION FOR SEQ ID NO:140:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:140:
      G N N L P A K F L E G F V R TA
(2) INFORMATION FOR SEQ ID NO:141:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:141:
      G K F L E G F V R T S N I K F
(2) INFORMATION FOR SEQ ID NO:142:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:142:
      G F V R T S N I K F Q D A Y N
(2) INFORMATION FOR SEQ ID NO:143:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:143:
      S N I K F Q D A Y N A G G G H
(2) INFORMATION FOR SEQ ID NO:144:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:144:
      Q D A Y N A G G G H N G V F D
(2) INFORMATION FOR SEQ ID NO:145:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:145:
      A G G G H N G V F D F P D S G
(2) INFORMATION FOR SEQ ID NO:146:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:146:
      N G V F D F P D S G T H S W E
(2) INFORMATION FOR SEQ ID NO:147:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:147:
      F P D S G T H S W E Y W G A Q
(2) INFORMATION FOR SEQ ID NO:148:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:148:
      T H S W E Y W G A Q L N A M K
(2) INFORMATION FOR SEQ ID NO:149:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:149:
      Y W G A Q L N A M K P D L Q R
(2) INFORMATION FOR SEQ ID NO:150:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:150:
      L N A M K P D L Q R A L G A T
(2) INFORMATION FOR SEQ ID NO:151:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:151:
      P D L Q R A L G A T P N T G P
(2) INFORMATION FOR SEQ ID NO:152:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: C-Terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:152:
      A L G A T P N T G P A P Q G A
(2) INFORMATION FOR SEQ ID NO:153:
   (i) SEQUENCE CHARACTERISTICS:
      (A) LENGTH: 18 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: N-Terminal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:153:
      F S R P G L P V E Y L Q V P S P S M
(2) INFORMATION FOR SEQ ID NO:154:
   (i) SEQUENCE CHARACTERISTICS:
      (A) LENGTH: 16 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:154:
      D I K V Q F Q S G G A N S P A L
(2) INFORMATION FOR SEQ ID NO:155:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO: 155:
      P V G G Q S S F Y S D W Y Q P A C
(2) INFORMATION FOR SEQ ID NO:156:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:156:
      SMAASSALTLAIYHPQQ
(2) INFORMATION FOR SEQ ID NO:157:
   (i) SEQUENCE CHARACTERISTICS:
      (A) LENGTH: 18 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:157:
      P Q Q F V Y A G A M S G L L D P S Q
(2) INFORMATION FOR SEQ ID NO:158:
   (i) SEQUENCE CHARACTERISTICS:
      (A) LENGTH: 18 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:158:
      C G N G K P S D L G G N N L P A K
(2) INFORMATION FOR SEQ ID NO:159:
   (i) SEQUENCE CHARACTERISTICS:
      (A) LENGTH: 16 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:159:
      F Q D A Y N A G G G H N G V F D
(2) INFORMATION FOR SEQ ID NO:160:
   (i) SEQUENCE CHARACTERISTICS:
      (A) LENGTH: 14 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: Internal
   (vi) ORIGINAL SOURCE:
      (A) ORGANISM: *Mycobacterium tuberculosis*
      (B) STRAIN: Erdman
   (xi) SEQUENCE DESCRIPTION: SEQ ID NO:160:
      P D L Q R A L G A T P N T G

## Description

### FIELD OF THE INVENTION

The present invention generally relates to immunotherapeutic agents and vaccines against pathogenic bacteria. More specifically, unlike prior art vaccines and immunotherapeutic agents based upon pathogenic subunits or products which exhibit the greatest or most specific molecular immunogenicity, the present invention uses the most prevalent or majorly abundant immunogenic determinants released by the selected pathogen *Mycobacterium tuberculosis* to stimulate an effective immune response in mammalian hosts. Accordingly, the acquired immunity and immunotherapeutic activity produced through the present invention is directed to those antigenic markers which are displayed most often on infected host cells during the course of a pathogenic infection without particular regard to the relative or absolute immunogenicity of the administered compound.

### BACKGROUND OF THE INVENTION

It has long been recognized that parasitic microorganisms possess the ability to infect animals thereby causing disease and often the death of the host. Pathogenic agents have been a leading cause of death throughout history and continue to inflict immense suffering. Though the last hundred years have seen dramatic advances in the prevention and treatment of many infectious diseases, complicated host-parasite interactions still limit the universal effectiveness of therapeutic measures. Difficulties in countering the sophisticated invasive mechanisms displayed by many pathogenic vectors is evidenced by the resurgence of various diseases such as tuberculosis, as well as the appearance of numerous drug resistant strains of bacteria and viruses.

Among those pathogenic agents of major epidemiological concern, intracellular bacteria have proven to be particularly intractable in the face of therapeutic or prophylactic measures. Intracellular bacteria, including the genus *Mycobacterium* and the genus *Legionella,* complete all or part of their life cycle within the cells of the infected host organism rather than extracellularly. Around the world, intracellular bacteria are responsible for millions of deaths each year and untold suffering. Tuberculosis, caused by *Mycobacterium tuberculosis* is the leading cause of death from infectious disease worldwide, with 10 million new cases and 2.9 million deaths every year. In addition, intracellular bacteria are responsible for millions of cases of leprosy. Other debilitating diseases transmitted by intracellular agents include cutaneous and visceral leishmaniasis, American trypanosomiasis (Chagas disease), listeriosis, toxoplasmosis, histoplasmosis, trachoma, psittacosis, Q-fever, and Legionellosis including Legionnaires' disease. At this time, relatively little can be done to prevent debilitating infections in susceptible individuals exposed to these organisms.

Due to this inability to effectively protect populations from tuberculosis and the inherent human morbidity and mortality caused by tuberculosis, this is one of the most important diseases confronting mankind. More specifically, human pulmonary tuberculosis primarily caused by *M tuberculosis* is a major cause of death in developing countries. Capable of surviving inside macrophages and monocytes, *M tuberculosis* may produce a chronic intracellular infection. By concealing itself within the cells primarily responsible for the detection of foreign elements and subsequent activation of the immune system, *M tuberculosis* is relatively successful in evading the normal defenses of the host organism. These same pathogenic characteristics have heretofore prevented the development of an effective immunotherapeutic agent or vaccine against tubercular infections. At the same time tubercle bacilli are relatively easy to culture and observe under laboratory conditions. Accordingly, *M tuberculosis* is particularly well suited for demonstrating the principles and advantages of the present invention.

Currently it is believed that approximately half of the world's population is infected by *M tuberculosis* resulting in millions of cases of pulmonary tuberculosis annually. While this disease is a particularly acute health problem in the developing countries of Latin America, Africa, and Asia, it is also becoming more prevalent in the first world. In the United States specific populations are at increased risk, especially urban poor, immunocompromised individuals and immigrants from areas of high disease prevalence. Largely due to the AIDS epidemic the incidence of tuberculosis is presently increasing in developed countries, often in the form of multi-drug resistant *M tuberculosis*.

Recently, tuberculosis resistance to one or more drugs was reported in 36 of the 50 United States. In New York City, one-third of all cases tested in 1991 were resistant to one or more major drugs. Though nonresistant tuberculosis can be cured with a long course of antibiotics, the outlook regarding drug resistant strains is bleak. Patients infected with strains resistant to two or more major antibiotics have a fatality rate of around 50%. Accordingly, a safe and effective vaccine against such varieties of *M tuberculosis* is sorely needed.

Initial infections *of M. tuberculosis* almost always occur through the inhalation of aerosolized particles as the pathogen can remain viable for weeks or months in moist or dry sputum. Although the primary site of the infection is in the lungs, the organism can also cause infection of the bones, spleen, meninges and skin. Depending on the virulence of the particular strain and the resistance of the host, the infection and corresponding damage to the tissue may be minor or extensive. In the case of humans, the initial infection is controlled in the majority of individuals exposed to virulent strains of the bacteria. The development of acquired immunity following the initial challenge reduces bacterial proliferation thereby allowing lesions to heal and leaving the subject largely asymptomatic but possibly contagious.

When *M tuberculosis* is not controlled by the infected subject, it often results in the extensive degradation of lung tissue. In susceptible individuals lesions are usually formed in the lung as the tubercle bacilli reproduce within alveolar or pulmonary macrophages. As the organisms multiply, they may spread through the lymphatic system to distal lymph nodes and through the blood stream to the lung apices, bone marrow, kidney and meninges surrounding the brain. Primarily as the result of cell-mediated hypersensitivity responses, characteristic granulomatous lesions or tubercles are produced in proportion to the severity of the infection. These lesions consist of epithelioid cells bordered by monocytes, lymphocytes and fibroblasts. In most instances a lesion or tubercle eventually becomes necrotic and undergoes caseation.

While *M. tuberculosis* is a significant pathogen, other species of the genus *Mycobacterium* also cause disease in animals including man and are clearly within the scope of the present invention. For example, *M bovis* is closely related to *M tuberculosis* and is responsible for tubercular infections in domestic animals such as cattle, pigs, sheep, horses, dogs and cats. Further, *M bovis* may infect humans via the intestinal tract, typically from the ingestion of raw milk. The localized intestinal infection eventually spreads to the respiratory tract and is followed shortly by the classic symptoms of tuberculosis. Another important pathogenic vector of the genus *Mycobacterium* is *M. leprae* which causes millions of cases of the ancient disease leprosy. Other species of this genus which cause disease in animals and man include *M. kansasii, M. avium intracellulare, M. fortuitum, M. marinum, M. chelonei, M. africanum, M ulcerans, M microti* and *M. scrofulaceum.* The pathogenic mycobacterial species frequently exhibit a high degree of homology in their respective DNA and corresponding protein sequences and some species, such as *M. tuberculosis* and *M. bovis* are highly related.

For obvious practical and moral reasons, initial work in humans to determine the efficacy of experimental compositions with regard to such afflictions is infeasible. Accordingly, in the early development of any drug or vaccine it is standard procedure to employ appropriate animal models for reasons of safety and expense. The success of implementing laboratory animal models is predicated on the understandingthat immunodominantepitopes are frequently active in different host species. Thus, an immunogenic determinant in one species, for example a rodent or guinea pig, will generally be immunoreactive in a different species such as in humans. Only after the appropriate animal models are sufficiently developed will clinical trials in humans be carried out to further demonstrate the safety and efficacy of a vaccine in man.

With regard to alveolar or pulmonary infections by *M. tuberculosis,* the guinea pig model closely resembles the human pathology of the disease in many respects. Accordingly, it is well understood by those skilled in the art that it is appropriate to extrapolate the guinea pig model of this disease to humans and other mammals. As with humans, guinea pigs are susceptible to tubercular infection with low doses of the aerosolized human pathogen *M tuberculosis.* Unlike humans where the initial infection is usually controlled, guinea pigs consistently develop disseminated disease upon exposure to the aerosolized pathogen, facilitating subsequent analysis. Further, both guinea pigs and humans display cutaneous delayed-type hypersensitivity reactions characterized by the development of a dense mononuclear cell induration or rigid area at the skin test site. Finally, the characteristic tubercular lesions of humans and guinea pigs exhibit similar morphology including the presence of Langhans giant cells. As guinea pigs are more susceptible to initial infection and progression of the disease than humans, any protection conferred in experiments using this animal model provides a strong indication that the same protective immunity may be generated in man or other less susceptible mammals. Accordingly, for purposes of explanation only and not for purposes of limitation, the present invention will be primarily demonstrated in the exemplary context of guinea pigs as the mammalian host. Those skilled in the art will appreciate that the present invention may be practiced with other mammalian hosts including humans and domesticated animals.

Any animal or human infected with a pathogenic vector and, in particular, an intracellular organism presents a difficult challenge to the host immune system. While many infectious agents may be effectively controlled by the humoral response and corresponding production of protective antibodies, these mechanisms are primarily effective only against those pathogens located in the body's extracellular fluid. In particular, opsonizing antibodies bind to extracellular foreign agents thereby rendering them susceptible to phagocytosis and subsequent intracellular killing. Yet this is not the case for other pathogens. For example, previous studies have indicated that the humoral immune response does not appear to play a significant protective role against infections by intracellular bacteria such as *M tuberculosis* However, the present invention may generate a beneficial humoral response to the target pathogen and, as such, its effectiveness is not limited to any specific component of the stimulated immune response.

More specifically, antibody mediated defenses seemingly do not prevent the initial infection of intracellular pathogens and are ineffectual once the bacteria are sequestered within the cells of the host. As water soluble proteins, antibodies can permeate the extracellular fluid and blood, but have difficulty migrating across the lipid membranes of cells. Further, the production of opsonizing antibodies against bacterial surface structures may actually assist intracellular pathogens in entering the host cell. Accordingly, any effective prophylactic measure against intracellular agents, such as *Mycobacterium* should incorporate an aggressive cell-mediated immune response component leading to the rapid proliferation of antigen specific lymphocytes which activate the compromised phagocytes or cytotoxically eliminate them. However, as will be discussed in detail below, inducing a cell-mediated immune response does not equal the induction of protective immunity. Though cell-mediated immunity may be a prerequisite to protective immunity, the production of vaccines in accordance with the teachings of the present invention requires animal based challenge studies.

This cell-mediated immune response generally involves two steps. The initial step, signaling that the cell is infected, is accomplished by special molecules (major histocompatibility or MHC molecules) which deliver pieces of the pathogen to the surface of the cell. These MHC molecules bind to small fragments of bacterial proteins which have been degraded within the infected cell and present them at the surface of the cell. Their presentation to T-cells stimulates the immune system of the host to eliminate the infected host cell or induces the host cell to eradicate any bacteria residing within.

Unlike most infectious bacteria *Mycobacterium,* including *M*. *tuberculosis,* tend to proliferate in vacuoles which are substantially sealed off from the rest of the cell by a membrane. Phagocytes naturally form these protective vacuoles making them particularly susceptible to infection by this class of pathogen. In such vacuoles the bacteria are effectively protected from degradation, making it difficult for the immune system to present integral bacterial components on the surface of infected cells. However, the infected cell's MHC molecules will move to the vacuole and collect any free (released) bacterial products or move to other sites in the host cell to which the foreign extracellular bacterial products have been transported for normal presentation of the products at the cell surface. As previously indicated, the presentation of the foreign bacterial products will provoke the proper response by the host immune system.

The problems intracellular pathogens pose for the immune system also constitute a special challenge to vaccine development. Thus far, the production of an effective vaccine against *Mycobacterium* infections and, in particular, against *M tuberculosis* has eluded most researchers. At the present time the only widely available vaccine against intracellular pathogens is the live attenuated vaccine BCG, an avirulent strain of *M bovis,* which is used as a prophylactic measure against the tubercle bacillus. Yet in 1988, extensive World Health Organization studies from India determined that the efficacy of the best BCG vaccines was so slight as to be unmeasurable. Despite this questionable efficacy, BCG vaccine has been extensively employed in high incidence areas of tuberculosis throughout the world. Complicating the matter even further individuals who have been vaccinated with BCG will often develop sensitivity to tuberculin which negates the usefulness of the most common skin test for tuberculosis screening and control.

Another serious problem involving the use of a live, attenuated vaccine such as BCG is the possibility of initiating a life-threatening disease in immunocompromised patients. These vaccines pose a particular risk for persons with depressed cell-mediated immunity because of their diminished capacity to fight a rapidly proliferating induced infection. Such individuals include those weakened by malnourishment and inferior living conditions, organ transplant recipients, and persons infected with HIV. In the case of BCG vaccine, high risk individuals also include those suffering from lung disorders such as emphysema, chronic bronchitis, pneumoconiosis, silicosis or previous tuberculosis. Accordingly, the use of attenuated vaccines is limited in the very population where they have the greatest potential benefit.

The use of live attenuated vaccines may also produce other undesirable side effects. Because live vaccines reproduce in the recipient, they provoke a broader range of antibodies and a less directed cell-mediated immune response than noninfectious vaccines. Often this shotgun approach tends to occlude the immune response directed at the molecular structures most involved in cellular prophylaxis. Moreover, the use of live vaccines with an intact membrane may induce opsonizing antibodies which prepare a foreign body for effective phagocytosis. Thus, upon host exposure to virulent strains of the target organism, the presence of such antibodies could actually enhance the uptake of nonattenuated pathogens into host cells where they can survive and multiply. Further, an attenuated vaccine contains thousands of different molecular species and consequently is more likely to contain a molecular species that is toxic or able to provoke an adverse immune response in the patient. Other problems with live vaccines include virulence reversion, natural spread to contacts, contaminating viruses and viral interference, and difficulty with standardization.

Similarly, noninfectious vaccines, such as killed organisms or conventional second generation subunit vaccines directed at strongly antigenic membrane bound structures, are limited with respect to the inhibition of intracellular bacteria. Like attenuated vaccines, killed bacteria provoke an indiscriminate response which may inhibit the most effective prophylactic determinants. Further, killed vaccines still present large numbers of potentially antigenic structures to the immune system thereby increasing the likelihood of toxic reactions or opsonization by the immune system. Traditional subunit vaccines incorporating membrane bound structures, whether synthesized or purified, can also induce a strong opsonic effect facilitating the entry of the intracellular pathogen into phagocytes in which they multiply. By increasing the rate of bacterial inclusion, killed vaccines directed to intracellular surface antigens may increase the relative virulence of the pathogenic agent. Thus, conventional attenuated or killed vaccines directed against strongly antigenic bacterial surface components may be contraindicated in the case of intracellular pathogens.

In order to circumvent the problems associated with the use of traditional vaccines, developments have been made using extracellularproteins or their immunogenic analogs to stimulate protective immunity against specific intracellular pathogens. For example, this inventor's U.S. Patent No. 5,108,745, issued April 28, 1992 discloses vaccines and methods of producing protective immunity against *Legionella pneumophila* and *M. tuberculosis* as well as other intracellular pathogens. These prior art vaccines are broadly based on extracellular products originally derived from proteinaceous compounds released extracellularly by the pathogenic bacteria into broth culture *in vitro* and released extracellularly by bacteria within infected host cells *in vivo.* As disclosed therein, these vaccines are selectively based on the identification of extracellular products or their analogs which stimulate a strong immune response against the target pathogen in a mammalian host.

More specifically, these prior art candidate extracellularproteins were screened by determining their ability to provoke either a strong lymphocyte proliferative response or a cutaneous delayed-type hypersensitivity response in mammals which were immune to the pathogen of interest. Though this disclosed method and associated vaccines avoid many of the drawbacks inherent in the use of traditional vaccines, conflicting immunoresponsive results due to cross-reactivity and host variation may complicate the selection of effective immunizing agents. Thus, while molecular immunogenicity is one indication of an effective vaccine, other factors may complicate its use in eliciting an effective immune response *in vivo.*

More importantly, it surprisingly was discovered that, particularly with respect to *M. tuberculosis,* conventional prior art methods for identifying effective protective immunity inducing vaccines were cumbersome and potentially ineffective. For example, SDS-PAGE analysis of bulk *M tuberculosis* extracellular protein followed by conventional Western blot techniques aimed at identifying the most immunogenic of these extracellular components produced inconsistent results. Repeated testing failed to identify which extracellular product would produce the strongest immunogenic response and, consistent with prior art thinking, thereby function as the most effective vaccine. Many of the extracellular products of *M tuberculosis* are well known in the art, having been identified and, in some cases, sequenced. Further, like any foreign protein, it can be shown that these known compounds induce an immune response. However, nothing in the art directly indicates that any of these known compounds will induce protective immunity as traditionally identified.

Accordingly, it is a principal object of the present invention to provide vaccines or immunotherapeutic agents for mounting an effective an effective immune response against intracellularpathogens including *M. tuberculosis, M. bovis, M. kansasii, M. avium-intracellulare, M. fortuitum, M chelonei, M. marinum, M. scrofulaceum, M. leprae, M. africanum, M. ulcerans* and *M. microti.*

It is an additional object of the present invention to provide easily produced vaccines and immunotherapeuticagents exhibiting reduced toxicity relative to killed or attenuated vaccines.

### SUMMARY OF THE INVENTION

The present invention as defined in the claims accomplishes the above-described and other obj ects by providing compounds for use as vaccines and/or immunotherapeutic agents and methods for their production and their use to generate protective or therapeutic immune responses in mammalian hosts against infection by pathogens.

For purposes of the present disclosure, the term "majorly abundant" should be understood as a relative term identifying those extracellular products released in the greatest quantity by the pathogen of interest. For example, with respect to *M. tuberculosis* grown under various conditions of culture to an optical density of approximately 0.5, one skilled in the art should expect to obtain on the order of 10 µg/L or more of a majorly abundant extracellular product. Thus, out of the total exemplary 4 mg/L total output of extracellular product for *M. tuberculosis* grown under normal or heat shock conditions, approximately fifteen to twenty (alone or in combination) of the one hundred or so known extracellular products will constitute approximately ninety percent of the total quantity. These are the majorly abundant extracellularproducts contemplated as being within the scope of the present disclosure and are readily identifiable as the broad bands appearing in SDS/PAGE gels. In addition, the extracellular products of interest may further be characterized and differentiated by amino acid sequencing. The remaining extracellular products are minor. Those skilled in the art will also appreciate that the relative quantitative abundance of specific major extracellular products may vary depending upon conditions of culture. However, in most cases, the identification of an individual majorly abundant extracellular product will not change.

Accordingly, the compounds of the disclosure may be used to protect a mammalian host against infection by bacterial, pathogens.

The vaccines of the present invention are effective in generating protective immunity against various species and serogroups of the genus *Mycobacterium.* The vaccines of the present disclosure are effective as immunotherapeuticagents for the treatment of existing disease conditions.

Surprisingly, it has been found by this inventor that immunization with the most or majorly abundant products released extracellularly by bacterial pathogens or their immunogenic analogs can provoke an effective immune response irrespective of the absolute immunogenicity of the administered compound. Due to their release from the organism and hence their availability to host molecules involved in antigen processing and presentation and due to their naturally high concentration in tissue during infection, the majorly abundant extracellular products of a pathogenic bacterium are processed and presented to the host immune system more often than other bacterial components. In the case of intracellular pathogens, the majorly abundant extracellular products are the principal immunogenic determinants presented on the surface of the infected host cells and therefore exhibit a greater presence in the surrounding environment. Accordingly, acquired immunity against the majorly abundant extracellularproducts of a pathogenic bacterium allows the host defense system to swiftly detect pathogens sequestered inside host cells and effectively inhibit them.

More particularly, the principal or majorly abundant products released by pathogenic bacteria appear to be processed by phagocytes and other host immune system mechanisms at a greater rate than less prevalent or membrane bound pathogenic components regardless of their respective immunogenic activity or specificity. This immunoprocessingdisparity is particularly significant when the pathogenic agent is an intracellular bacteria sequestered from normal immune activity. By virtue of their profuse and continual presentation to the infected host's immune system, the most prevalent bacterial extracellular products or their immunogenic analogs provoke a vigorous immune response largely irrespective of their individual molecular immunogenic characteristics.

Majorly abundant extracellular products are the principal constituents of proteins and other molecular entities which are released by the target pathogen into the surrounding environment. Current research indicates that in some instances a single majorly abundant extracellular product may comprise up to 40% by weight of the products released by a microorganism. More often, individual majorly abundant extracellular products account for between from about 0.5% to about 25% of the total products released by the infectious pathogen. Moreover, the top five or six majorly abundant extracellular products may be found to comprise between 60% to 70% of the total mass released by a microorganism. Of course those skilled in the art will appreciate that the relative levels of extracellular products may fluctuate over time as can the absolute or relative quantity of products released. For example, pH, oxidants, osmolality, heat and other conditions of stress on the organism, stage of life cycle, reproduction status and the composition of the surrounding environment may alter the composition and quantity of products released. Further, the absolute and relative levels of extracellular products may differ greatly from species to species and even between strains within a species.

In the case of intracellular pathogens extracellular products appear to expand the population of specifically immune lymphocytes capable of detecting and exerting an antimicrobial effect against macrophages containing live bacteria. Further, by virtue of their repeated display on the surface of infected cells, the majorly abundant or principal extracellularproducts function as effective antigenic markers. Accordingly, vaccination and the inducement of protective immunity directed to the majorly abundant extracellular products of a pathogenic bacteria or their immunogenically equivalent determinants, prompts the host immune system to mount a rapid and efficient immune response with a strong cell-mediated component when subsequently infected by the target pathogen.

In direct contrast to prior art immunization activities which have primarily been focused on the production of vaccines and the stimulation of immune responses based upon the highly specific molecular antigenicity of individual screened pathogen components, the present disclosure advantageously exploits the relative abundance of bacterial extracellular products or their immunogenic analogs (rather than their immunogenic specificities) to establish or induce protective immunity with compounds which may actually exhibit lower immunogenic specificity than less prevalent extracellular products. For the purposes of this disclosure an immunogenic analog is any molecule or compound sufficiently analogous to at least one majorly abundant extracellular product expressed by the target pathogen, or any fraction thereof, to have the capacity to stimulate a protective immune response in a vaccinated mammalian host upon subsequent infection by the target pathogen. In short, the vaccines of the present disclosure are identified or produced by selecting the majorly abundant product or products released extracellularly by a specific pathogen (or molecular analogs capable of stimulating a substantially equivalent immune response) and isolating them in a relatively pure form or subsequently sequencing the DNA or RNA responsible for their production to enable their synthetic or endogenous production. The desired prophylactic immune response to the target pathogen may then be elicited by formulating one or more of the isolated immunoreactive products or the encoding genetic material using techniques well known in the art and immunizing a mammalian host prior to infection by the target pathogen.

The present invention produces consistent, standardized vaccines which may be developed, tested and administered with relative ease and speed. Further, the use of a few well defined molecules corresponding to the majorly abundant secretory or extracellular products greatly reduces the risk of adverse side effects associated with conventional vaccines and eliminates the possible occlusion of effective immunogenic markers. Similarly, because the present invention is not an attenuated or a killed vaccine the risk of infection during production, purification or upon administration is effectively eliminated. As such, the vaccines of the present invention may be administered safely to immunocompromised individuals, including asymptomatic tuberculosis patients and those infected with HIV. Moreover, as the humoral immune response is directed exclusively to products released by the target pathogen, there is little chance of generating a detrimental opsonic immune component. Accordingly, the present invention allows the stimulated humoral response to assist in the elimination of the target pathogen from antibody susceptible areas.

Another beneficial aspect of the present invention is the ease by which the vaccines may be harvested or produced and subsequently purified and sequenced. For example, the predominantly abundant extracellular products may be obtained from cultures of *M. tuberculosis* with little effort. As the desired compounds are released into the media during growth, they can readily be separated from the intrabacterial and membrane-bound components of the target pathogen utilizing conventional techniques. More preferably, the desired immunoreactive constituents of the vaccines of the present invention may be produced and purified from genetically engineered organisms into which the genes expressing the specific extracellular products of *M. tuberculosis* have been cloned. As known in the art, such engineered organisms can be modified to produce higher levels of the selected extracellular products or modified immunogenic analogs. Alternatively, the immunoprotective products, portions thereof or analogs thereof, can be chemically synthesized using techniques well known in the art or directly expressed in host cells injected with naked genes encoding therefor. Whatever production source is employed, the immunogenic components of the predominant or majorly abundant extracellular products may be separated and subsequently formulated into deliverable vaccines using common biochemical procedures such as fractionation, chromatography or other purification methodology and conventional formulation techniques or directly expressed in host cells containing directly introduced genetic constructs encoding therefor.

For example, in the present invention the target pathogen is *M. tuberculosis and* the majorly abundant products released extracellularly by *M. tuberculosis* into broth culture are separated from other bacterial components and used to elicit an immune response in mammalian hosts. Individual proteins or groups of proteins are then utilized in animal based challenge experiments to identify those which induce protective immunity making them suitable for use as vaccines in accordance with the teachings of the present invention. More specifically, following the growth and harvesting of the bacteria, by virtue of their physical abundance the principal extracellular products are separated from intrabacterial and other components through centrifugation and filtration. If desired, the resultant bulk filtrate is then subjected to fractionationusing ammonium sulfate precipitation with subsequent dialysis to give a mixture of extracellular products, commonly termed EP. Solubilized extracellular products in the dialyzed fractions are then purified to substantial homogeneity using suitable chromatographic techniques as known in the art and as described more fully below.

These exemplary procedures result in the production of fourteen individual proteinaceous major extracellularproducts of *M. tuberculosis* having molecular weights ranging from 110 kilo Daltons (KD) to 12 KD. Following purification each individual majorly abundant extracellular product exhibits one band corresponding to its respective molecular weight when subjected to polyacrylamide gel electrophoresis thereby allowing individual products or groups of products corresponding to the majorly abundant extracellular products to be identified and prepared for use as vaccines. The purified majorly abundant extracellularproducts may further be characterized and distinguished by determining all or part of their respective amino acid sequences using techniques common in the art. Sequencing may also provide information regarding possible structural relationships between the majorly abundant extracellular products.

Subsequently, immunization and the stimulation of acquired immunity in a mammalian host system may be accomplished utilizing a series of subcutaneous or intradermal injections of these purified extracellular products over a course of time. For example, injection with a purified majorly abundant bacterial extracellular product or products in incomplete Freund's adjuvant followed by a second injection in the same adjuvant approximately three weeks later can be used to elicit a protective response upon subsequent challenge with the virulent pathogen. Other exemplary immunization protocols may include a series of three or four injections of purified extracellular product or products or their analogs in Syntex Adjuvant Formulation (SAF) over a period of time. While a series of injections may generally prove more efficacious, the single administration of a selected majorly abundant extracellular product or its immunogenic subunits or analogs can impart the desired immune response.

Such exemplary protocols can be demonstrated using art accepted laboratory models such as guinea pigs. For example; as will be discussed in detail, immunization of several guinea pigs with a combination of five majorly abundant extracellular products (purified from *M. tuberculosisas* previously discussed) was accomplished with an immunization series of three injections of the bacterial products in SAF adjuvant with corresponding sham-immunization of control animals. Exemplary dosages of each protein ranged from 100 µg to 2 µg. Following the last vaccination all of the animals were simultaneously exposed to an infectious and potentially lethal dose of aerosolized *M*. *tuberculosis* and monitored for an extended period of time. The control animals showed a significant loss in weight when compared with the animals immunized with the combination of the majorly abundant extracellular products of *M. tuberculosis*. Moreover, half of the control animals died during the observation period while none of the immunized animals succumbed to tuberculosis. Autopsies conducted after this experiment revealed that the nonimmunized control animals had significantly more colony forming units (CFU) and corresponding damage in their lungs and spleens than the protected animals. Seventeen additional combinations of purified majorly abundant extracellular products provided immunoprophylaxis when tested.

However, it should be emphasized that the present disclosure is not restricted to combinations of secretory or extracellular products. For example, several alternative experimental protocols demonstrate the capacity of a single abundant extracellular product to induce mammalian protective immunity. In each experiment guinea pigs were immunized with a single majorly abundant extracellular product purified from *M. tuberculosis* EP.

Following their respective immunizations both sets of animals and the appropriate controls were exposed to lethal doses of aerosolized *M*. *tuberculosis* to determine vaccine effectiveness.

It is important to note that the formulation of the vaccine is not critical to the present invention and may be optimized to facilitate administration. Solutions of the purified immunogenic determinants derived from the majorly abundant pathogenic extracellular products may be administered alone or in combination in any manner designed to generate a protective immune response. The purified protein solutions may be delivered alone, or formulated with an adjuvant before being administered.

Alternatively, genetic material encoding the genes for one or more of the immunogenic determinants derived from the majorly abundant pathogenic extracellular products may be coupled with eucaryotic promoter and/or secretion sequences and injected directly into a mammalian host to induce endogenous expression of the immunogenic determinants and subsequent protective immunity.

Other objects, features and advantages of the present invention will be apparent to those skilled in the art from a consideration of the following detailed description of preferred exemplary embodiments thereof taken in conjunction with the figures which will first be described briefly.

### BRIEF DESCRIPTION OF THE DRAWINGS

Fig. 1 is a representation of 4 coomassie blue stained gels, labeled 1a to 1d, illustrating the purification of exemplary majorly abundant extracellular products of *M. tuberculosis* as identified by sodium deodecyl sulfate polyacrylamidegel electrophoresis (SDS-PAGE).
Fig. 2 is a tabular representation identifying the five N-terminal amino acids of fourteen exemplary majorly abundant extracellular products of *M*. *tuberculosis* (Sequence ID Nos. 1-14) and the apparent molecular weight for such products.
Fig. 3 is a tabular representation of the extended N-terminal amino acid sequence of three exemplary majorly abundant secretory products of *M*. *tuberculosis* (Sequence ID Nos. 15-17) which were not distinguished by the five N-terminal amino acids shown in Fig. 2.
Fig. 4 is a graphical comparison of mean guinea pig body weight of animals immunized with vaccine comprising a combination of extracellular products produced according to the teachings of the present invention and nonimmunized controls following exposure to an aerosolized lethal dose of *M. tuberculosis*
Fig. 5 is a graphical comparison of mean guinea pig body weight of animals immunized with three different dosages of a vaccine comprising a combination of extracellular products produced according to the teachings of the present invention and nonimmunized controls following exposure to an aerosolized lethal dose of *M*. *tuberculosis*
Fig. 6 is a graphical comparison of mean guinea pig body weight of animals immunized with vaccines comprising six different combinations of extracellular products produced according to the teachings of the present invention and nonimmunized controls following exposure to an aerosolized lethal dose of *M. tuberculosis*
Fig. 7 is a graphical illustration of the mapping of the immunodominant epitopes of the 32A KD protein of *M. tuberculosis*
Fig. 8 provides a diagrammatic representation of the constructs used for the expression of recombinant 32A KD protein. The diagram depicts the pSMT3 vector used for the expression of recombinant 32A KD protein. In (A) the DNA fragment carrying the gene for the 32A KD protein is arranged in the opposite direction from the hsp 60 promoter. In (B) the DNA fragment carrying the gene for the 32A KD protein is arranged in the same direction as the hsp 60 promoter.
Fig. 9 shows electrophoresis test results comparing secretion of recombinant mature *M. tuberculosis* 32A KD major extracellularprotein at 28°C (Lane 3) and 37°C (Lane 2).
Fig. 10 is a graphical comparison of the growth of guinea pig lymphoblasts in the presence of various concentrations of recombinant human and murine IL-12.
Fig. 11 is a graphical comparison of the growth of guinea pig lymphoblasts in the presence of various concentrations of recombinant human and murine IL-12 from a guinea pig different from that of Fig. 21.
Fig. 12 is a graphical comparison of mean guinea pig body weight gain or loss of animals immunized with a vaccine comprising a combination of purified 32A KD, 30 KD, and 16 KD extracellular proteins, MF 59 adjuvant, and IL-12 versus nonimmunized controls following exposure to an aerosolized lethal dose of *M. tuberculosis*.
Fig. 13 is a graphical comparison of mean guinea pig body weight gain or loss of animals immunized with a vaccine comprising a combination of purified 32A KD, 30 KD,: and 16 KD extracellular proteins with MF 59 adjuvant, with IL-12, and with a mixture of MF 59 and IL-12 versus nonimmunized controls following exposure to an aerosolized lethal dose of *M. tuberculosis*

### DETAILED DESCRIPTION OF EXEMPLARY EMBODIMENTS

The present invention is directed to compounds and method for their production and their use against pathogenic organisms as vaccines and immunotherapeutic agents as defined in the claims. More specifically, the present disclosure is directed to the production and use of majorly abundant extracellular products released by pathogenic organisms, their immunogenic analogs or the associated genetic material encoding therefor as vaccines or immunotherapeuticagents and to associated methods for generating protective immunity in mammalian hosts against infection. These compounds will be referred to as vaccines throughout this application for purposes of simplicity.

In exemplary embodiments, the majorly abundant extracellular products of *M. tuberculosis* were distinguished and subsequently purified. Guinea pigs were immunized with purified forms of these majorly prevalent extracellular products with no determination of the individual product's specific molecular immunogenicity. Further, the exemplary immunizations were carried out using the purified extracellular products alone or in combination and with various dosages and routes of administration. The present invention is limited to vaccines directed against *M*. *tuberculosis*.

In these exemplary embodiments, the majorly abundant extracellular products of *M. tuberculosis* were separated and purified using column chromatography. Determination of the relative abundance and purification of the extracellular products was accomplished using polyacrylamide gel electrophoresis. Following purification of the vaccine components, guinea pigs were vaccinated with the majorly abundant extracellular products alone or in combination and subsequently challenged with *M*. *tuberculosis.* As will be discussed in detail, in addition to developing the expected measurable responses to these extracellular products following immunization, the vaccines of the present invention unexpectedly conferred an effective immunity in these laboratory animals against subsequent lethal doses of aerosolized *M*. *tuberculosis.*

While these exemplary embodiments used purified forms of the extracellular products, those skilled in the art will appreciate that the present invention may easily be practiced using immunogenic analogs which are produced through recombinant means or other forms of chemical synthesis using techniques well known in the art. Further, immunogenic analogs, homologs or selected segments of the majorly abundant extracellular products may be employed in lieu of the naturally occurring products within the scope and teaching of the present invention.

A further understanding of the present invention will be provided to those skilled in the art from the following nonlimiting examples which illustrate exemplary protocols for the identification, isolation, production and use of majorly abundant extracellular products (alone and in combination)as vaccines.

### Example 1

### Isolation and Production of Bulk Extracellular Proteins (EP) from Mycobacterium tuberculosis

*M tuberculosis* Erdman strain (ATCC 35801) was obtained from the American Tissue Culture Collection (Rockville, Md.). The lyophilized bacteria were reconstituted in Middlebrook 7H9 culture medium (Difco Laboratories, Detroit, Mich.) and maintained on Middlebrook 7H11 agar. 7H11 agar was prepared using Bacto Middlebrook 7H10 agar (Difco), OADC Enrichment Medium (Difco), 0. 1 % casein enzymatic hydrolysate (Sigma), and glycerol as previously described by Cohn (Cohn, M.1., Am. Rev. Respir. Dis. 98:295-296). Following sterilization by autoclaving, the agar was dispensed into bacteriologic petri dishes (100 by 15 mm) and allowed to cool.

*M. tuberculosis* was then plated using sterile techniques and grown at 37°C in 5% CO₂-95% air, 100% humidity. After culture on 7H11 for 7 days, the colonies were scraped from the plates, suspended in 7H9 broth to 10⁸ CFU/ml and aliquoted into 1.8-ml Nunc cryotubes (Roskilde, Denmark). Each liter of the broth was prepared by rehydrating 4.7g of Bacto Middlebrook 7H9 powder with 998 ml of distilled water, and 2 ml of glycerol (Sigma Chemical Co., St. Louis, Mo.) before adjusting the mixture to a pH value of 6.75 and autoclaving the broth for 15 min at 121°C. The aliquoted cells were then slowly frozen and stored at -70°C. Cells stored under these conditions remained viable indefinitely and were used as needed.

Bulk extracellular protein (EP) preparations were obtained from cultures of *M. tuberculosis* grown in the Middlebrook 7H9 broth made as above. Following reconstitution, 150 ml aliquots of the broth were autoclaved for 15 min at 121°C and dispensed into vented Co-star 225 cm² tissue culture flasks. *M. tuberculosis* cells stored at -70°C as described in the previous paragraph were thawed and used to inoculate 7H11 gar plates. After culture for 7 days, the colonies were scraped from the plates, suspended in a few ml of 7H9 broth, and sonicated in a water bath to form a single cell suspension. The *M tuberculosis* cells were suspended in the sterile 150 ml aliquots at an initial optical density of 0.05, as determined by a Perkin-Elmer Junior model 35 spectrophotometer(Norwalk, Conn). The cells were then incubated at 37°C in 5% CO₂-95% air for 3 weeks until the suspension showed an optical density of 0.4 to 0.5. These cultures were used as stock bottles for subsequent cultures also in 7H9 broth. The stock bottles were sonicated in a water bath to form a single cell suspension. The *M tuberculosis* cells were then diluted in 7H9 broth to an initial optical density of 0.05 and incubated at 37°C in 5% CO²-95% air for 2½ to 3 weeks until the suspension showed an optical density of 0.4 to 0.5. Culture supernatant was then decanted and filter sterilized sequentially through 0.8 µm and 0.2 µm low-protein-binding filters (Gelman Sciences Inc., Ann Arbor, Mich.). The filtrate was then concentrated approximately 35 fold in a Filtron Minisette with an Omega membrane having a 10 KD cutoff and stored at 4°C. Analysis of the bulk extracellularprotein preparation by sodium deodecyl sulfate-polyacrylamidegel electrophoresis (SDS-PAGE) revealed a protein composition with multiple bands. Bulk extracellularprotein mixture (EP) was prepared by obtaining a 40-95% ammonium sulfate cut of the culture filtrate.

### Example 2

### Purification of Principal Majorly Abundant ExtracellularProducts of Mycobacterium tuberculosis

Ammonium sulfate (grade I, Sigma) was added to the sterile culture filtrate of Example 1 in concentrations ranging from 10% to 95% at 0°C and gently stirred to fractionate the proteins. The suspension was then transferred to plastic bottles and centrifuged in a swinging bucket rotor at 3,000 rpm on a RC3B Sorvall Centrifuge to pellet the resulting precipitate. The supernatant fluid was decanted and, depending on the product of interest, the supernatant fluid or pellet was subjected to further purification. When the product of interest was contained in the supernatant fluid a second ammonium sulfate cut was executed by increasing the salt concentration above that of the first cut. After a period of gentle stirring the solution was then centrifuged as previously described to precipitate the desired product and the second supernatant fluid was subjected to further purification.

Following centrifugation, the precipitated proteins were resolubilized in the appropriate cold buffer and dialyzed extensively in a Spectrapor dialysis membrane (Spectrum Medical Industries, Los Angeles, California) with a 6,000 to 8,000 molecular weight cut-off to remove the salt. Extracellularprotein concentration was determined by a bicinchoninic acid protein assay (Pierce Chemical Co., Rockford, Illinois) and fraction components were determined using SDS-PAGE. The fractions were then applied to chromatography columns for further purification.

Using the general scheme outlined immediately above fourteen extracellular products were purified from the bulk extracellularprotein filtrate obtained by the process detailed in Example 1. The exact ammonium sulfate precipitation procedure and chromatography protocol is detailed below for each extracellular product isolated.

### A. 110 KD Extracellular Product

1. A 50-100% ammonium sulfate precipitate was obtained as discussed above.
2. The resolubilized precipitate was dialyzed and applied to a DEAE Sepharose CL-6B or QAE Sepharose ion exchange column in column buffer consisting of 10% sorbitol, 10 mM potassium phosphate, pH 7,
   5 mM 2-mercaptoethanol, and 0.2 mM EDTA and eluted with a sodium chloride gradient. Fractions containing 110 KD protein elute at approximately 550 mM salt and were collected.
3. Collected fractions were applied to S200 Sepharose size fractionation column in PBS (phosphate buffered saline) buffer. The protein eluted as a homogeneous 110 KD protein.

### B. 80 KD Extracellular Product

1. The 0-25% ammonium sulfate cut (1 hour at 0°C) was discarded and the 25-60% ammonium sulfate cut (overnight at 0°C) was retained as discussed above.
2. A DEAE CL-6B column (Pharmacia) was charged with 25mM Tris, pH 8.7 containing 1M NaCl and equilibrated with 25mM Tris, pH 8.7, 10mM NaCl and the protein sample was dialyzed against 25mM Tris, pH 8.7, 10mM NaCl and applied to the column. The column was washed overnight with the same buffer. A first salt gradient of 10mM to 200 mM NaCl in 25mM Tris, pH 8.7 was run through the column to elute other proteins. A second salt gradient (200 to 300 mM NaCl) was run through the column and the 80 KD protein eluted at approximately 275 mM NaCl.
3. A Q-Sepharose HP column was charged with 25mM Tris, pH 8.7, 1M NaCl and re-equilibratedto 25mM Tris, pH 8.7, 10mM NaCl. The protein sample was dialyzed against 25mM Tris, ph 8.7, 10mM NaCl and applied to the column. The column was washed in the same buffer and then eluted with 200-300 mM NaCl in 25mM Tris, pH 8.7.
4. Fractions containing the 80 KD protein were collected and dialyzed against 25mM Tris, pH 8.7, 10mM NaCl, and then concentrated in a Speed-Vac concentratorto 1-2 ml. The protein sample was applied to a Superdex 75 column and eluted with 25 mM Tris, pH 8.7, 150 mM NaCl. The 80 KD protein eluted as a homogenous protein.

### C. 71 KD Extracellular Product

1. A 40-95% ammonium sulfate precipitate was obtained as discussed above with the exception that the 71 KD product was cultured in 7H9 broth at pH 7.4 and at 0% CO₂ and heat-shocked at 42°C for 3h once per week. The precipitate was dialyzed against Initial Buffer (20 mM Hepes, 2 mM MgAc, 25 mM KCl, 10 mM (NH4)₂SO₄, 0.8 mM DL-Dithiothreitol,pH 7.0).
2. The resolubilized precipitate was applied to an ATP Agarose column equilibrated with Initial Buffer. Effluent was collected and reapplied to the ATP Agarose column. The 71 KD protein bound to the column.
3. Subsequently the ATP Agarose column was washed, first with Initial Buffer, then 1 M KCl, then Initial Buffer.
4. Homogeneous 71 KD protein was eluted from the column with 10 mM ATP and dialyzed against phosphate buffer.

### D. 58 KD ExtracellularProduct

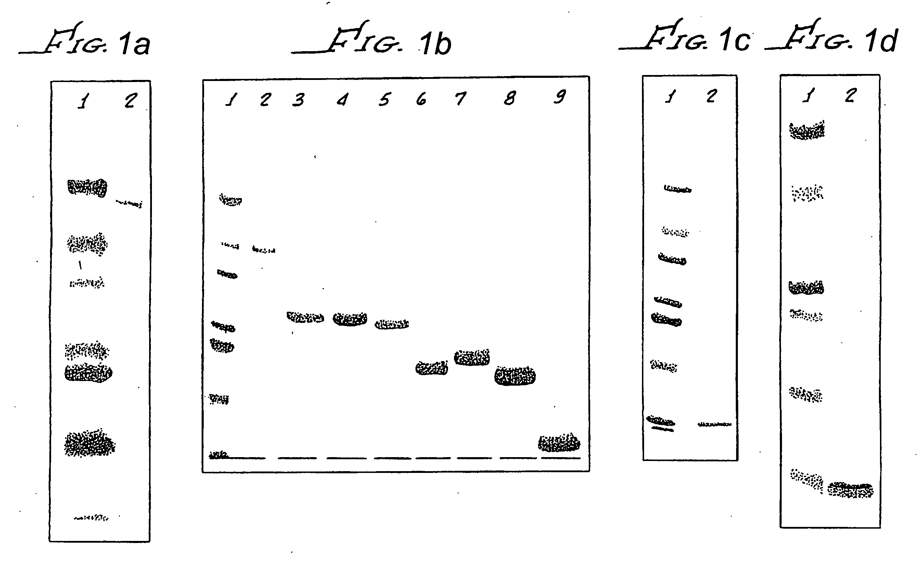

1. A 25-50% ammonium sulfate precipitate was obtained as discussed above.
2. The resolubilized precipitate was dialyzed and applied to a DEAE-Sepharose CL-6B or QAE-Sepharose column and eluted with NaCl. Collected fractions containing the 58 KD Protein eluted at approximately 400 mM NaCl.
3. Collected fractions were then applied to a Sepharose CL-6B size fractionation column. The protein eluted at approximately 670-700,000 Daltons.
4. The eluted protein was applied to a thiopropyl-sepharosecolumn. The homogeneous 58 KD protein eluted at approximately 250-350 mM 2-mercaptoethanol. The eluted protein was monitored using SDS-PAGE and exhibited the single band shown in Fig. 1 A, col. 2.

### E. 45 KD Extracellular Product

1.
   a. A 0-25% ammonium sulfate cut (1 hour at 0°C) was discarded.
   b. The 25-60% ammonium sulfate cut (overnight at 0°C) was retained.
2.
   a. A DEAE CL-6B column (Pharmacia) was charged with 2.5 mM Tris, pH 8.7 containing 1 M NaCl and equilibrated with 25 mM Tris, 10 mM NaCl, pH 8.7.
   b. The protein sample was dialyzed against 25 mM Tris, 10 mM NaCl, pH 8.7 and applied to column. The column was then washed overnight with the same buffer.
   c. The column was eluted with a salt gradient (10 mM to 200 mM) in 25 mM Tris, pH 8.7 buffer. The 45 KD protein eluted at approximately 40 mM NaCl.
3.
   a. A Q-Sepharose HP (Pharmacia) column was charged with 25 mM Tris, pH 8.7 containing 1 M NaCl and re-equilibrated with 25 mM Tris, 10 mM NaCl, pH 8.7.
   b. The protein sample was dialyzed against 25 mM Tris, 10 mM NaCl, pH 8.7 and applied to column with subsequent washing using the same buffer.
   c. The column was eluted with 10-150 mM NaCl in 25 mM Tris, pH 8.7.
4.
   a. Fractions containing the 45 KD product were collected, pooled and dialyzed against 25 mM Tris, 10 mM NaCl, pH 8.7, before concentration to 1 ml in a Speed Vac concentrator.
   b. Concentrate was Applied to Superdex 75 column equilibrated with 25 mM Tris 150 mM NaCl, pH 8.7. The product eluted as a homogeneous protein. The eluted protein was monitored using SDS-PAGE and resulted in the single band shown in Fig. 1B, co 1. 2.

### F. 32 KD Extracellular Product (A)

1.
   a. A 0-25% ammonium sulfate cut (1 hour at 0°C) was discarded.
   b.The 25-60% ammonium sulfate cut (overnight at 0°C) was retained.
2.
   a. A DEAE CL-6B column (Pharmacia) was charged with 25 mM Tris, pH 8.7 containing 1 M NaCl and then equilibrated with 25 mM Tris, 10 mM NaCl, pH 8.7.
   b. The protein sample was dialyzed against 25 mM Tris, 10 mM NaCl, pH 8.7 and applied to the column with subsequent washing overnight with same buffer.
   c. The column was eluted with a salt gradient (10 mM to 200 mM) in 25 mM Tris, pH 8.7 buffer. The 32 KD protein eluted at approximately 70 mM NaCl.
3.
   a. Fractions containing the 32 KD product were collected, pooled and dialyzed against 25 mM Tris, 10 mM NaCl, pH 8.7, before concentrating the protein sample to 1 ml in a Speed-Vac Concentrator.
   b. The concentrate was then Applied to a Superdex 75 column equilibrated with 25 mM Tris, 150 mM NaCl, pH 8.7 and eluted with this buffer. The 32 KD product eluted as homogeneous protein.
4.
   a. A Q-Sepharose HP column (Pharmacia) was charged with 25 mM Tris, pH 8.7 containing 1 M NaCl, and re-equilibrated with 25 mM Tris, 10mM NaCl, pH 8.7.
   b. The protein sample was dialyzed against 25 mM Tris, 10 mM NaCl, pH 8.7 and applied to the column with subsequent washing in the same buffer.
   c. The column was eluted with a 100-300 mM NaCl gradient. Labeled 32A, the homogeneous protein elutes at approximately 120 mM NaCl and is shown as a single band in Fig. 1 B, co 1. 4.

### G. 32 KD ExtracellularProduct (B)

1.
   a. A 0-25% ammonium sulfate cut (1 hour at 0°C) was discarded.
   b. The 25-60% ammonium sulfate cut (overnight at 0°C) was retained.
2.
   a. A DEAE CL-6B column (Pharmacia) was charged with 25 mM Tris, pH 8.7 containing 1 M NaCl and then equilibrated with 25 mM Tris, 10 mM NaCl, pH 8.7.
   b. The protein sample was dialyzed against 25 mM Tris, 10 mM NaCl, pH 8.7 and applied to the column with subsequent washing overnight with same buffer.
   c. A preliminary salt gradient of 10 mM to 200 mM NaCl in 25 mM Tris, pH 8.7 was run, eluting various proteins. Following column equilibration, a second salt gradient (200 to 300 mM NaCl) was run. The 32 KD protein eluted at approximately 225 mM NaCl.
3. a.
   a. A Q-Sepharose HP column (Pharmacia) was charged with 25 mM Tris, pH 8.7 containing 1 M NaCl, and re-equilibrated with 25 mM Tris, 10 mM NaCl, pH 8.7.
   b. The protein sample was dialyzed against 25 mM Tris, 10 mM NaCl, pH 8.7 and applied to the column with subsequent washing in the same buffer.
   c. The column was eluted with a 200-300 mM NaCl gradient in the same buffer.
4.
   a. Fractions containing the 32 KD product were collected, pooled and dialyzed against 25 mM Tris, 10 mM NaCl, pH 8.7, before concentrating the protein sample to 1 ml in a Speed-Vac Concentrator.
   b. The concentrate was then applied to a Superdex 75 column equilibrated with 25 mM Tris, 150 mM NaCl, pH 8.7 and eluted with the same buffer. The 32 KD product, labeled 32B to distinguish it from the protein of 32 KD separated using protocol H, eluted as homogeneous protein and is shown as a single band on Fig. 1 B, co 1. 3.

### H. 30 KD Extracellular Product

1.
   a. A 0-25% ammonium sulfate cut (1 hour at 0°C) was discarded.
   b. The 25-60% ammonium sulfate cut (overnight at 0°C) was retained.
2.
   a. A DEAE CL-6B column (Pharmacia) was charged with 25 mM Tris, pH 8.7 containing 1 M NaCl and then equilibrated with 25 mM Tris, 10 mM NaCl, pH 8.7.
   b. The protein sample was dialyzed against 25 mM Tris, 10 mM NaCl, pH 8.7 and applied to the column with subsequent washing overnight with same buffer.
   c. The column was eluted with a salt gradient (10 mM to 200 mM) in 25 mM Tris, pH 8.7 buffer. The 30 KD protein eluted at approximately 140 mM NaCl.
3.
   a. Fractions containing the 30 KD product were collected, pooled and dialyzed against 25 mM Tris, 10 mM NaCl, pH 8.7, before concentrating the protein sample to 1 ml in a Speed-Vac Concentrator.
   b. The concentrate was then Applied to a Superdex 75 column equilibrated with 25 mM Tris, 150 mM NaCl, pH 8.7 and eluted with this buffer. The 30 KD product eluted as homogeneous protein and is shown as a single band on Fig. 1B, col. 5.

### I. 24 KD Extracellular Product

1.
   a. A 0-25% ammonium sulfate cut (1 hour at 0°C) was discarded.
   b. The 25-60% ammonium sulfate cut (overnight at 0°C) was retained.
2.
   a. A DEAE CL-6B column (Pharmacia) was charged with 25 mM Tris, pH 8.7 containing 1 M NaCl and then equilibrated with 25 mM Tris, 10 mM NaCl, pH 8.7.
   b. The protein sample was dialyzed against 25 mM Tris, 10 mM NaCl, pH 8.7 and applied to the column with subsequent washing overnight with same buffer.
   c. A preliminary salt gradient of 10 mM to 200 mM NaCl in 25 mM Tris, pH 8.7 was run, eluting various proteins. Following column equilibration a second salt gradient (200 to 300 mM NaCl) was run. The 24 KD elutes at approximately 250 mM NaCl.
3.
   a. A Q-Sepharose HP column (Pharmacia) was charged with 25 mM Tris, pH 8.7 containing 1 M NaCl, and re-equilibrated with 25 mM Tris, 10 mM NaCl, pH 8.7.
   b. The protein sample was dialyzed against 25 mM Tris, 10 mM NaCl, pH 8.7 and applied to the column with subsequent washing in the same buffer.
   c. The column was eluted with a 200-300 mM NaCl gradient in the same buffer.
4.
   a. Fractions containing the 24 KD product were collected, pooled and dialyzed against 25 mM Tris, 10 mM NaCl, pH 8.7, before concentrating the protein sample to 1 ml in a Speed-Vac Concentrator.
   b. The concentrate was then applied to a Superdex 75 column equilibrated with 25 mM Tris, 150 mM NaCl, pH 8.7 and eluted with the same buffer. The 24 KD product eluted as homogeneous protein and is shown as a single band on Fig. 1 B, col 7.

### J. 23.5 KD Extracellular Product

1.
   a. A 0-25% ammonium sulfate cut (1 hour at 0°C) was discarded.
   b. The 25-60% ammonium sulfate cut (overnight at 0°C) was retained.
2.
   a. A DEAE CL-6B column (Pharmacia) was charged with 25 mM Tris, pH 8.7 containing 1 M NaCl and then equilibrated with 25 mM Tris, 10 mM NaCl, pH 8.7.
   b. The protein sample was dialyzed against 25 mM Tris, 10 mM NaCl, pH 8.7 and applied to the column prior to subsequent washing overnight with same buffer.
   c. The column was eluted with a salt gradient (10 mM to 200 mM) in 25 mM Tris, pH 8.7 buffer. The 23.5 KD protein eluted at approximately 80 mM NaCl.
3.
   a. A Q-Sepharose HP column was charged with 25 mM Tris, pH 8.7 containing 1 M NaCl, and re-equilibrated with 25 mM Tris, 10 mM NaCl, pH 8.7.
   b. The protein sample was dialyzed against 25 mM Tris, 10 mM NaCl, pH 8.7 and applied to the column with subsequent washing in the same buffer.
   c. The column was eluted with 100-300 mM NaCl in 25 mM Tris, pH 8.7.
   d. Steps 3a to 3c were repeated.
a. Fractions containing 23.5 KD product were collected, pooled and dialyzed against 25 mM Tris, 10 mM NaCl, pH 8.7. before concentrating the protein sample to 1 ml in a Speed-Vac Concentrator.
   b. The concentrate was then applied to a Superdex 75 column equilibrated with 25 mM Tris, 150 mM NaCl, pH 8.7 and eluted with the same buffer. The 23.5 KD product eluted as homogeneous protein. The eluted protein was monitored using SDS-PAGE and resulted in the single band shown in Fig. 1B, col 6.

### K. 23 KD Extracellular Product

1.
   a. Ammonium sulfate cuts of 0-25% (1h at 0°C) and 25-60% (overnight at 0°C) were discarded.
   b. A 60-95% ammonium sulfate cut was retained.
2.
   a. A DEAE CL-6B column (Pharmacia) was charged with 50 mM Bis-Tris pH 7.0 containing 1 M NaCl and equilibrated with 50 mM Bis-Tris, 100 mM NaCl, pH 7.0.
   b. The protein sample was dialyzed against 50 mM Bis-Tris, pH 7.0, 100 mM NaCl buffer and applied to the column before washing the column overnight with the same buffer.
   c. The column was eluted with a 100 to 300 mM NaCl linear gradient in 50 mM Bis-Tris pH 7.0.
   d. Fractions were collected containing the 23 KD protein which eluted at approximately 100-150 mM NaCl.
3.
   a. The protein fractions were dialyzed against 25 mM Tris, pH 8.7, 10 mM NaCl and concentrated to 1-2 ml on a Savant Speed Vac Concentrator.
   b. The concentrate was applied to a Superdex 75 column equilibrated with 25 mM Tris, 150 mM NaCl, pH 8.7. The product elutes as a homogeneous protein as is shown in Fig. 1B co 1. 8.

### L. 16 KD Extracellular Product

1.
   a. A 0-25% ammonium sulfate cut (1 hour at 0°C) was discarded.
   b. The 25-60% ammonium sulfate cut (overnight at 0°C) was retained.
2.
   a.A DEAE CL-6B column (Pharmacia) was charged with 2.5 mM Tris, pH 8.7 containing 1 M NaCl and then equilibrated with 25 mM Tris, 10 mM NaCl, pH 8.7.
   b. The protein sample was dialyzed against 25 mM Tris, 10 mM NaCl, pH 8.7 and applied to the column with subsequent washing overnight in the same buffer.
   c. The column was eluted with a salt gradient (10 mM to 200 mM) in 25 mM Tris, pH 8.7 buffer. The 16 KD protein eluted at approximately 50 mM NaCl.
3.
   a. Fractions containing 16 KD product were collected, pooled and dialyzed against 25 mM Tris, 10 mM NaCl, pH 8.7, before concentrating the protein sample to 1 ml in a Speed-Vac Concentrator.
   b. The concentrate was then applied to a Superdex 75 column equilibrated with 25 mM Tris, 150 mM NaCl, pH 8.7 and eluted with the same buffer. A 16 KD product eluted as homogeneous protein. The eluted protein was monitored using SDS-PAGE and resulted in the single band shown in Fig. 1 B, col. 9.

### M. 14 KD ExtracellularProduct

1.
   a. A 0-25% ammonium sulfate cut (1 hour at 0°C) was discarded.
   b. The 25-60% ammonium sulfate cut (overnight at 0°C) was retained.
2.
   a. A DEAE CL-6B column (Pharmacia) was charged with 25 mM Tris, pH 8.7 containing 1 M NaCl and then equilibrated with 25 mM Tris, 10 mM NaCl, pH 8.7.
   b. The protein sample was dialyzed against 25 mM Tris, 10 mM NaCl, pH 8.7 and applied to the column with subsequent washing overnight in the same buffer.
   c. The column was eluted with a salt gradient (10 mM to 200 mM) in 25 mM Tris, pH 8.7 buffer. The 14 KD protein eluted at approximately 60 mM NaCl.
3.
   a. A Q-Sepharose HP column was charged with 25 mM Tris, pH 8.7 containing 1 M NaCl, and re-equilibrated with 25 mM NaCl, pH 8.7.
   b. The protein sample was dialyzed against 25 mM Tris, 10 mM NaCl, pH 8.7 and applied to the column with subsequent washing in the same buffer.
   c. The column was eluted with 10-150 mM NaCl in 25 mM Tris, pH 8.7.
   d. Steps 3a through 3c were repeated.
4.
   a. Fractions containing 14 KD product were collected, pooled and dialyzed against 25 mM Tris, 10 mM NaCl, pH 8.7, before concentrating the protein sample to 1 ml in a Speed-Vac Concentrator.
   b. The concentrate was then applied to a Superdex 75 column equilibrated with 25 mM Tris, 150 mM NaCl, pH 8.7 and eluted with this buffer. The 14 KD product eluted as homogeneous protein. The eluted protein was monitored using SDS-PAGE and resulted in the single band shown in Fig. 1C, col 2.

### N. 12 KD ExtracellularProducts

1. A 0-10% ammonium sulfate precipitate was obtained (overnight at 4°C).
2. The resolubilized precipitate was applied to a S200 Sephacryl size fractionation column eluting the protein as a 12 KD molecule.
3. The protein fractions were applied to a DEAE-Sepharose CL-6B or QAE-Sepharose ion exchange column and eluted with an NaCl gradient as previously described. Fractions containing two homogeneous proteins having molecular weights of approximately 12 KD eluted at approximately 300-350 mM NaCl and were collected. The proteins were labeled 12A and 12B and purified as a doublet shown in Fig. 1 D, co 1. 2.

As illustrated in the SDS-PAGE profile of Fig. 1, the principal or majorly abundant extracellular proteins of *M. tuberculosis* were purified to homogeneity through the use of the protocols detailed in Examples 2A-2N above. More particularly, Fig. 1 illustrates four exemplary 12.5% acrylamide gels developed using SDS-PAGE and labeled 1 A, 1 B, 1 C, and 1D. The standard in lane 1 of gels 1 A-1C has proteins with molecular weights of 66, 45, 3 6, 29, 24, 20, and 14 KD. In gel 1D the standard in lane 1 contains proteins with molecular weights of 68, 45, 31, 29, 20, and 14 KD. The lanes containing the respective purified extracellular products show essentially one band at the reported molecular weight of the individual protein. It should be noted that in gel 1 D the 12 KD protein runs as a doublet visible in lane 2. Sequence analysis shows that the lower 12 KD (or 12B KD band) is equivalent to the upper 12 KD (or 12A KD) band except that it lacks the first 3 N-terminal amino acids.

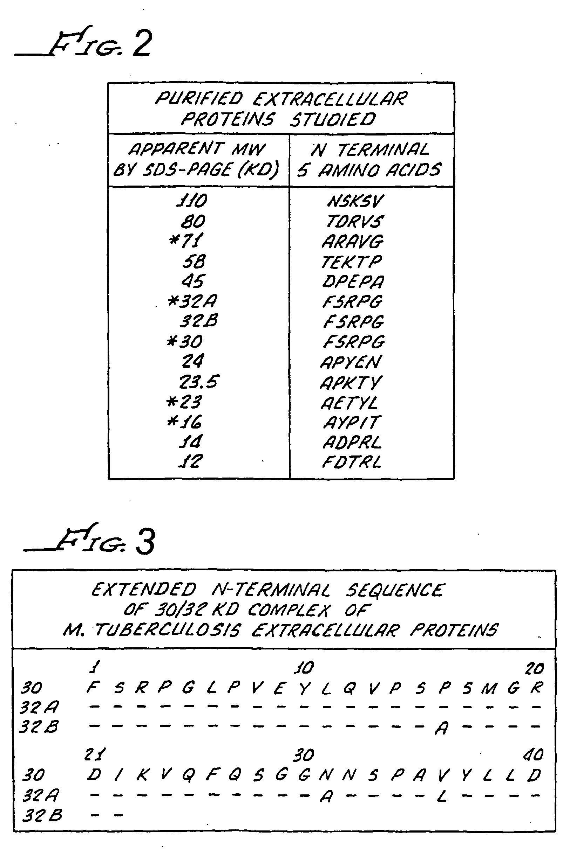

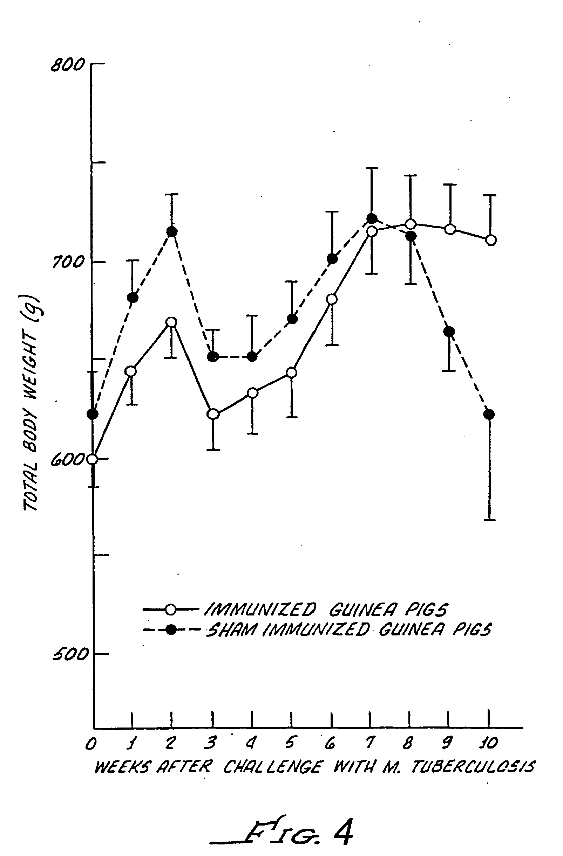

Further analysis of these individual exemplary majorly abundant extracellular products is provided in Fig. 2. More particularly Fig. 2 is a tabular compilation of N-terminal sequence data obtained from these purified extracellularproducts showing that the majority of the isolated products are indeed distinct (Sequence ID Nos. 1-14). Proteins 32A, 32B and 30 all had the same 5 N-terminal amino acids therefore further sequencing was necessary to fully characterize and differentiate them. Fig. 3 shows the extended N-terminal amino acid sequences for these three purified secretory products (Sequence ID Nos. 15-17). Different amino acids at positions 16 (Sequence ID No. 17), 31 (Sequence ID No. 16) and 36 (Sequence ID No. 16) demonstrate that these isolated proteins are distinct from one another despite their similarity in molecular weight.

In addition to proteins 30, 32A and 32B, extended N-terminal amino acid sequences of other majorly abundant extracellular products were determined to provide primary structural data and to uncover possible relationships between the proteins. Sequencing was performed on the extracellular products purified according to Example 2 using techniques well known in the art. Varying lengths of the N-terminal amino acid sequence, determined for each individual extracellular product, are shown below identified by the apparent molecular weight of the intact protein, and represented using standard one letter abbreviations for the naturally occurring amino acids. In keeping with established rules of notation, the N-terminal sequences are written left to right in the direction of the amino terminus to the carboxy terminus. Those positions where the identity of the determined amino acid is less than certain are underlined. Where the amino acid at a particular position is unknown or ambiguous, the position in the sequence is represented by a dash. Finally, where two amino acids are separated by a slash, the correct constituent has not been explicitly identified and either one may occupy the position in that sequence.

| **Protein** | N-terminal Amino Acid Sequence |
|---|---|
| **12 KD** | |
| **14 KD** | |
| **16 KD** | |
| **23 KD** | |
| **23.5 KD** | |
| **24 KD** | |
| **30 KD** | |
| **32A KD** | |
| **32B KD** | |
| **45 KD** | |
| **58 KD** | |
| **71 KD** | |
| **80 KD** | |
| **110 KD** | |

DNA sequencing was performed on the 30, 32A, 16, 58, 23.5, and 24 KD proteins using techniques well known in the art. These DNA sequences, and the corresponding amino acids, including upstream and downstream sequences, are shown below identified by the apparent molecular weight of the intact protein and represented using standard abbreviations and rules of notation.

This sequence data, combined with the physical properties ascertained using SDS-PAGE, allow these representative majorly abundant extracellular products of the present invention to be characterized and distinguished. The analysis described indicates that these proteins constitute the majority of the extracellular products of *M tuberculosis* with the 71 KD, 30 KD, 32A KD, 23 KD and 16 KD products comprising approximately 60% by weight of the total available extracellularproduct. It is further estimated that the 30 KD protein may constitute up to 25% by weight of the total products released by *M. tuberculosis.* Thus, individual exemplary majorly abundant extracellular products of *M. tuberculosis* useful in the practice of the present invention may range anywhere from approximately 0.5% up to approximately 25% of the total weight of the extracellular products.

As previously discussed, following the inability of traditional Western blot analysis to consistently identify the most immunogenically specific extracellularproducts, the present inventor decided to analyze the immunogenicity of the majorly abundant extracellular products based upon their abundance and consequent ease of identification and isolation. Surprisingly, it was found that these maj orly abundant extracellular products induce unexpectedly effective immune responses leading this inventor to conclude that they may function as vaccines. This surprising discovery led to the development of the nonlimiting functional theory of this invention discussed above.

To demonstrate the efficacy of the present invention, additional experiments were conducted using individual majorly abundant extracellular products and combinations thereof at various exemplary dosages to induce protective immunity in art accepted laboratory models. More specifically, purified individual majorly abundant extracellularproducts were used to induce protective immunity in guinea pigs which were then challenged with *M. tuberculosis.* Upon showing that these proteins were capable of inducing protective immunity, combinations of five purified majorly abundant extracellularproducts was similarly tested using differing routes of administration. Results of the various studies of these exemplary vaccines of the present disclosure follow.

Specific pathogen-free male Hartley strain guinea pigs (Charles River Breeding Laboratories, North Wilmington, Massachusetts) were used in all experiments involving immunogenic or aerosol challenges with *M. tuberculosis.* The animals were housed two or three to a stainless steel cage and allowed free access to standard guinea pig chow and water. After arrival at the animal facility, the guinea pigs were observed for at least one week prior to the start of each experiment to ensure that they were healthy.

Initial experiments were conducted using individual majorly abundant extracellular products believed to comprise between 3% to 25% of the total extracellular proteins normally present. These experiments demonstrate that majorly abundant extracellular products elicit an effective immune response.

### Example 3

### Protocol for purified Protein Skin Testing for Cell-Mediated Immunity of 30 KD Immunized Guinea Pigs

To illustrate that a measurable immune response can be induced by purified forms of abundant extracellular products, a cutaneous hypersensitivity assay was performed. Guinea pigs were immunized with a exemplary majorly abundant *M*. *tuberculosis* secretory product purified according to Example 2. In three independent experiments, guinea pigs were immunized three times three weeks apart with 100 mg of substantially purified protein in SAF adjuvant. Control animals were similarly injected with buffer in SAF. Three weeks after the last immunization the guinea pigs were challenged with the protein in a cutaneous hypersensitivity assay.

Guinea pigs were shaved over the back and injections of 0.1, 1 and 10 mg of protein were administered intradermally with resulting erythema (redness of the skin) and induration measured after 24 hours. Data are reported in terms of mean measurement values for the group ± standard error (SE) as determined using traditional methods. ND indicates that this particular aspect of the invention was not done.

### Example 4

### Protocol for Protein Testing for Cell-Mediated Immune Responses of Guinea Pigs Infected With M. tuberculosis

To obtain bacteria for use in experiments requiring the infection of guinea pigs, *M. tuberculosis* was first cultured on 7H11 agar and passaged once through a guinea pig lung to insure that they were virulent. For this purpose, guinea pigs were challenged by aerosol with a 10 ml suspension of bacteria in 7H9 broth containing approximately 5 x 10⁴ bacteria/ml. After the guinea pigs became ill, the animals were sacrificed and the lungs, containing prominent *M. tuberculosis* lesions, were removed. Each lung was ground up and cultured on 7H11 agar for 7 days to 10 days. The bacteria were scraped from the plates, diluted in 7H9 broth containing 10% glycerol, sonicated in a water bath to obtain a single cell suspension, and frozen slowly at-70°C at a concentration of approximately 2 x 10⁷ viable bacteria/ml. Viability of the frozen cells was measured by thawing the bacterial suspension and culturing serial dilutions of the suspension on 7HII agar. Just before a challenge, a vial of bacterial cells was thawed and diluted to the desired concentration in 7H9 broth.

The guinea pigs were exposed to aerosols of the viable *M tuberculosis* in a specially designed lucite aerosol chamber. The aerosol chamber measured 14 by 13 by 24 in. and contained two 6 inch diameter portals on opposite sides for introducing or removing guinea pigs. The aerosol inlet was located at the center of the chamber ceiling. A vacuum pump (Gast Mfg. Co., Benton Harbor, Michigan) delivered air at 30 lb/in² to a nebulizer-venturi unit (Mes Inc., Burbank, California), and an aerosol was generated from a 10-ml suspension of bacilli. A 0.2 µm breathing circuit filter unit (Pall Biomedical Inc., Fajardo, Puerto Rico) was located at one end of the chamber to equilibrate the pressure inside and outside of the assembly. Due to safety considerations, the aerosol challenges were conducted with the chamber placed completely within a laminar flow hood.

The animals were exposed to pathogenic aerosol for 30 minutes during which time the suspension of bacilli in the nebulizer was completely exhausted. Each aerosol was generated from the 10 ml suspension containing approximately 5.0 x 10⁴ bacterial particles per ml. Previous studies have shown that guinea pig exposure to this concentration of bacteria consistently produces infections in nonprotected animals. Following aerosol infection, the guinea pigs were housed in stainless steel cages contained within a laminar flow biohazard safety enclosure (Airo Clean Engineering Inc., Edgemont, Pennsylvania) and observed for signs of illness. The animals were allowed free access to standard guinea pig chow and water throughout the experiment.

In this experiment, the infected guinea pigs were sacrificed and splenic lymphocyte proliferation was measured in response to various concentrations of the 30 KD protein. More specifically, splenic lymphocytes were obtained and purified as described by Brieman and Horwitz (J. Exp. Med. 64:799-811). The lymphocytes were adjusted to a final concentration of 10⁷/ml in RPMI 1640 (GIBCO Laboratories, Grand Island, New York) containing penicillin (100 U/ml), streptomycin (100 mg/ml), and 10% fetal calf serum (GIBCO) and incubated with various concentrations of purified 30 KD secretory product in a total volume of 100 ml in microtest wells (96-well round-bottom tissue culture plate; Falcon Labware, Oxnard, California) for 2 days at 37°C in 5% CO₂-95% air and 100% humidity. Noninfected animals were used as negative controls. At the end of the incubation period, 0.25 mCi of [³H]thymidine (New England Nuclear, Boston, Massachusetts) was added to each well and the cells were further incubated for 2 hours at 37°C in 5% CO₂-95% air at 100% humidity. A multisample automated cell harvester (Skatron Inc., Sterling, Virginia) was used to wash each well, and the effluent was passed through a filtermat (Skatron). Filtermat sections representing separate microtest wells were placed in scintillation vials, and 2 ml of Ecoscint H liquid scintillation cocktail (National Diagnostics, Manville, New Jersey) was added. Beta particle emission was measured in a beta-scintillation counter (Beckman Instruments Inc., Fullerton, California).

Tissue samples from the infected and noninfected guinea pigs were assayed against 1 and 10 mg/ml of isolated 30 KD secretory protein. Samples were then monitored for their ability to incorporate [³H] thymidine.

### Example 5

### Protocol for challenge of protein Immunized Guinea Pig with Aerosolized M. tuberculosis

As before, the animals were immunized three times at three week intervals with 100mg of the protein in SAF. Control guinea pigs were immunized with 120mg of bulk EP in SAF or sham-immunized with buffer in the same adj uvant. Three weeks after the last immunization, the animals were challenged with aerosolized *M. tuberculosis as* described in Example 4.

Weight retention of the immunized animals was also monitored to further illustrates the prophylactic of vaccines incorporating majorly abundant extracellular products produced by pathogenic bacteria as taught by the present invention.

Following conclusion of the weight monitoring study, the surviving animals were sacrificed and the right lung and spleen of each animal was assayed for viable *M. tuberculosis* The animals were soaked in 2% amphyl solution (National Laboratories, Montvale, New Jersey), and the lungs and spleen were removed aseptically. The number of macroscopic primary surface lesions in the lungs were enumerated by visual inspection. Colony forming units (CFU) of *M. tuberculosis* in the right lung and spleen were determined by homogenizing each organ in 10 ml of 7H9 with a mortar and pestle and 90-mesh Norton Alundum (Fisher), serially diluting the tissue homogenate in 7H9, and culturing the dilutions on duplicate plates of 7HII agar by using drops of 0.1 ml/drop. All plates were kept in modular incubator chambers and incubated 12 to 14 days at 37°C in 5% CO₂, 95% air at 100% humidity. It should again be emphasized that the purified majorly abundant or principal extracellular product is free of the problems associated with prior art or bulk compositions and is more readily adaptable to synthetic and commercial production making the vaccines of the present invention superior to the prior art.

More particularly the bulk preparation cannot be manufactured easily on a large scale through modem biomoleculartechniques. Any commercial production of these unrefined bulk preparations containing all extracellularproducts would involve culturing vast amounts of the target pathogen or a closely related species and harvesting the resultant supernatant fluid. Such production methodology is highly susceptible to contamination by the target pathogen, toxic byproducts or other parasitic agents. Further, the large number of immunogenic determinants in such a preparation is far more likely to provoke a toxic immune reaction in a susceptible segment of the immunized population. Using these unrefined bulk preparations also negates the use of the most popular skin tests currently used for tuberculosis screening and control.

In direct contrast, the vaccines of the present invention can be mass-produced in relative safety using high yield transformed hosts. Similarly, the vaccines of the present invention can be produced in identical, easy to standardize batches as opposed to the wider variable production of bulk extracellular products. Moreover, as the number of immunogenic determinants presented to the host immune system is relatively small, toxic reactions and the chance of invalidating popular screening tests are greatly reduced.

Studies were performed in order to ascertain whether combinations of majorly abundant extracellular products of *M. tuberculosis* would provide protective immunity as well. In general, these studies utilized guinea pigs which were immunized either intradermally or subcutaneously with various dosages of vaccines comprising combinations of 5 purified extracellularproteins of *M. tuberculosis* in SAF three times, 3 or 4 weeks apart.

The first protein combination used for the immunization procedure, labeled Combination I, was comprised of 71 KD, 32A KD, 30 KD, 23 KD, and 16 KD proteins purified according to the protocols described in Example 2. This combination is believed to comprise up to 60% of the total extracellular protein normally present in *M. tuberculosis* culture supernatants. These proteins selected for use in Combination I, are identified with an asterisk in Fig. 2. Combination I vaccine containing 100 µg, 20 µg, or 2 µg of each protein was administered intradermally with the adjuvant SAF. Combination I vaccine containing 20 µg of each protein was also administered subcutaneously in similar experiments. Negative control guinea pigs were sham-immunized with equivalent volumes of SAF and buffer on the same schedule while positive controls were immunized using 120 µg of the bulk extracellular protein preparation from Example 1 in SAF. All injection volumes were standardized using buffer.

### Example 6

### Response of Combination I Immunized Guinea Pigs To a Challenge With Combination I Vaccine

To determine if the animals had developed a measurable immune response following vaccination with the Combination I mixture of principal extracellular products, a cutaneous hypersensitivity assay was performed. Guinea pigs were shaved over the back and injected intradermally with 1.0 µg and 10.0 mg of the same combination of the five purified extracellularproteins. 10.0 mg of buffer was used as a control and all injections were performed using a total volume of 0.1 ml. The diameters of erythema and induration at skin tests sites were measured at 24 hours after injection.

The results of the measurements are presented in Table L below. Data are again reported in terms of mean measurement values for the group ± standard error (SE) as determined using traditional methods. ND indicates that this particular aspect of the experiment was not done.

**Table L**

| | | | Erythema (mm) (Mean + SE) | |
|---|---|---|---|---|
| Guinea Pig Status | n | PD | 1.0 µg | 10.0 µg |
| Immunized | 6 | 0 | 11.4 ± 4.6 | 17.4 ± 2.6 |
| Controls | 6 | 0 | ND | 6.0 ± 0.5 |

| | | | Induration (mm) (Mean + SE) | |
|---|---|---|---|---|
| | n | PD | 1.0 µg | 10.0 µg |
| Immunized | 6 | 0 | 7.3 ± 0.8 | 11.6 ± 1.2 |
| Controls | 6 | 0 | ND | 4.2 ± 0.3 |

The data clearly demonstrate that a strong cell-mediated immune response to the Combination I extracellular proteins was generated by the vaccinated animals. The immunized guinea pigs show erythema and induration measurements almost three times greater than the control animals.

### Example 7

### Immunoprotective-Analysis of Combination I Vaccine against Aerosolized M. tuberculosis

Three weeks after the last immunization, the guinea pigs used for the preceding hypersensitivity assay were challenged with aerosolized *M. tuberculosis,* Erdman strain and weighed weekly for 10 weeks. This aerosol challenge was performed using the protocol of Example 4. Six animals immunized with 100 µg of the principal extracellular products of Combination I, along with equal sized groups of positive and negative controls, were challenged simultaneously with aerosolized *M. tuberculosis* Positive controls were immunized three times with 120 µg EP in SAF.

Guinea pigs that died before the end of the observation period were autopsied and examined for evidence of gross tuberculosis lesions. Such lesions were found in all animals which expired during the study.

Differences between immunized and control animals in mean weight profiles after aerosol challenge were analyzed by repeated measures analysis of variance (ANOVA). Differences between immunized and control guinea pigs in survival after challenge were analyzed by the two-tailed Fisher exact test. Data are the mean weights ± standard error (SE) for each group of six guinea pigs.

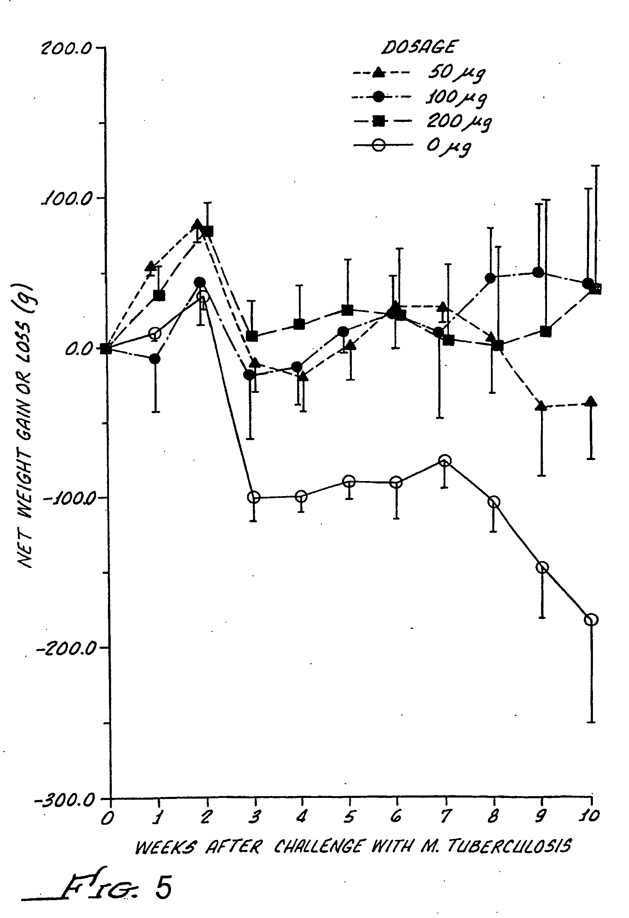

Results of the weekly weight determinations following challenge are shown in Fig. 4. Compared with guinea pigs immunized with the combination of extracellular products, sham-immunized animals lost 15.9% of their total body weight. Weights of the positive controls were similar to those of animals immunized with the combination of five purified extracellular proteins. Body weights were normalized immediately before challenge. The difference between animals immunized with Combination I and sham-immunized controls was highly significant with p <.0000001 by repeated measures ANOVA.

Mortality was determined ten and one-half weeks after challenge. All three of the sham-immunizedanimals died within three days of each other between ten and ten and one-half weeks after challenge. The mortality results of the experiment are provided in Table M below.

**Table M**

| Status of Guinea Pigs | Survivors/ Challenged | Percent Survival |
|---|---|---|
| Combination Immunized | 6/6 | 100% |
| EP-Immunized | 5/6 | 83% |
| Sham-Immunized | 3/6 | 50% |

Following the conclusion of the weight monitoring study, the surviving animals were sacrificed by hypercarbia and the right lung and spleen of each animal was assayed for viable *M. tuberculosis using* the protocol of Example 5. The results of the counts, including the 3 animals that died the last week of the experiment, are presented in Table N below in terms of mean colony forming units (CFU) ± standard error (SE).

**Table N**

| | | Mean CFU + SE | |
|---|---|---|---|
| Guinea Pig Status | n | Right Lung | Spleen |
| Sham-immunized | 6 | 8.9 ± 5.4 x 10⁷ | 1.3 ± 0.7 x 10⁷ |
| Immunized | 6 | 3.4 ± 1.7 x 10⁶ | 1.8 ± 0.6 x 10⁶ |
| EP-immunized | 6 | 1.7 ± 0.7 x 10⁷ | 5.0 ± 2.8 x 10⁶ |

The log difference between the concentration of bacilli in the lung of the animals immunized with the combination of purified proteins and that of the sham-immunized animals was 1.4 while the log difference of bacilli in the spleen was 0.9. Parallelling this, on gross inspection at autopsy immunized animals had markedly decreased lung involvement with tuberculosis compared with sham-immunized controls. Positive control animals immunized with the bulk extracellular preparation (EP) of Example I showed 0.7 log more bacilli in the lung and 5 log more bacilli in the spleen than animals immunized with the Combination I mixture of purified extracellularproteins.

### Example 8

### Immunoprotection Analysis of Combination I Vaccine At Low Doses Through Intradermal and Subcutaneous Delivery

While Example 7 confirmed that Combination I proteins demonstrated immunoprotection in animals immunized intradermally with 100 µg of each protein (30 + 32A + 16 + 23 + 71) 3 times, 4 weeks apart, an alternative study was conducted to demonstrate the immunoprotectivecapacity of lower doses of Combination I proteins, specifically 20 µg or 2 µg of each protein. As in Example 7, guinea pigs (6 animals per group) were immunized with Combination I proteins (30 + 32A + 16 + 23 + 71) intradermally in SAF 4 times, 3 weeks apart. Animals received either 20 µg or each protein per immunization or 2 µg of each protein per immunization. Control animals were sham-immunized utilizing the previous protocol. Three weeks later, the animals were challenged with aerosolized *M. tuberculosis* and weights were measured weekly for 9 weeks. All immunized animals survived to the end of the experiment while one sham-immunized animal died before the end of the experiment. As the following results illustrate, doses 5 fold and even 50 fold lower than those of Example 7 protected immunized animals from aerosolized *M tuberculosis* and that delivery by both the intradermal and subcutaneous route was effective.

Compared with guinea pigs immunized with 20 µg of each protein of Combination I, sham-immunized animals lost 12 % of their total body weight during the 9 weeks of the experiment (weights were normalized to just before challenge). Compared with guinea pigs immunized with 2 µg of each protein of Combination I, sham-immunized animals lost 11% of their normalized total body weight. Thus, guinea pigs immunized intradermally with low doses of Combination I proteins were protected against weight loss after aerosol challenge with *M. tuberculosis*.

Similarly, guinea pigs immunized intradermally with low doses of Combination I proteins also were protected against splenomegaly associated with dissemination of *M tuberculosis* to the spleen. As shown in Table O, whereas animals immunized with 20 µg or 2 µg of each protein of Combination I had spleens weighing an average of 4.6 ± 1.2g and 4.0 ± 0.8g (Mean ± SE), respectively, sham-immunized animals had spleens weighing an average of 9.6 ± 1.8g (Table 1), or more than twice as much.

**Table O**

| Status of Guinea Pigs | n | Spleen Weight (g) Mean + SE |
|---|---|---|
| Sham-Immunized | 5 | 9.6 ± 1.8 |
| Immunized (20 *µ*g) | 6 | 4.6 ± 1.2 |
| Immunized (2 *µ*g) | 6 | 4.0 ± 0.8 |

Guinea pigs immunized intradermally with low doses of Combination I proteins also had fewer CFU of *M. tuberculosis* in their spleens. As shown in Table P, when compared with sham-immunized animals, guinea pigs immunized with 20 µg or 2 ug of each protein of Combination I had an average of 0.6 and 0.4 log fewer CFU, respectively, in their spleens.

**Table P**

| Guinea Pig Status | n | CFU in Spleen Mean + SE | Log Difference |
|---|---|---|---|
| Sham-Immunized | 5 | 3.1 ± 2.3 x 10⁶ | |
| Immunized (20 *µ*g) | 6 | 8.1 ± 2.4 x 10⁵ | -0.6 |
| Immunized (2 µg) | 6 | 1.2 ± 0.6 x 10⁶ | -0.4 |

Moreover, guinea pigs immunized subcutaneously with Combination I proteins were also protected against weight loss, splenomegaly, and growth of *M tuberculosis* in the spleen. In the same experiment described in Example 8, guinea pigs were also immunized subcutaneously rather than intradermally with 20 µg of Combination I proteins, 4 times, 3 weeks apart. These animals were protected from challenge almost as much as the animals immunized intradermally with 20 µg of Combination I proteins.

### Example 9

### Response of Combination I and Combination II Immunized Guinea Pigs To Challenge with Combination I and Combination II

Additional studies were performed to ascertain whether other combinations of majorly abundant extracellular products of *M. tuberculosis would* provide protective immunity as well. One study utilized guinea pigs which were immunized with a vaccine comprising two combinations- Combination I (71,32A, 30, 23, and 16) and Combination II (32A, 30,24,23, and 16). Combination II is believed to comprise up to 62% of the total extracellular protein normally present in *M. tuberculosis* supernatants. Animals (6 per group) were immunized four times with 100 µg of each protein in Combination I or II in SAF, 3 weeks apart. Negative control animals were sham-immunized with equivalent volumes of SAF and buffer on the same schedule.

As in Example 6, the animals were tested for cutaneous delayed-type hypersensitivity to determine if the animals developed a measurable immune response following vaccination. Animals immunized with Combination II had 16.8 ± 1.3mm (Mean ± SE) erythema and 12.8 ± 1.2mm induration in response to skin-testing with Combination II whereas sham-immunized animals had only 1.3 ± 0.8mm erythema and 0.3 ± 3mm induration in response to Combination II. Thus, animals immunized with Combination II had greater than 12 fold more erythema and greater than 40 fold more induration than controls. By way of comparison, animals immunized with Combination I had 21.3 ± 2.0mm erythema and 15.8 ± 0.1 mm induration in response to skin-testing with Combination I, whereas sham-immunized animals had only 6.4 ± 0.8mm erythema and 2.6 ± 0.7mm induration in response to Combination I. Thus, animals immunized with Combination I had greater than 3 fold more erythema and greater than 6 fold more induration than controls. The difference from controls for Combination II proteins was even greater than that for Combination I proteins.

In the same experiment, animals immunized with a lower dose of Combination II proteins (20 µg of each protein vs. 100 µg) also developed strong cutaneous hypersensitivity to Combination II. They had 21.0 ± 2.0mm erythema and 15.3 ± 0.9mm induration in response to Combination II, whereas the sham-immunized animals had only 1.3 ± 0.8mm erythema and 0.3 ± 0.3mm induration, as noted above. Thus, animals immunized with a lower dose of Combination II proteins had greater than 16 fold erythema and greater than 50 fold more induration than controls, a difference that was even greater than for animals immunized with the higher dose of Combination II proteins.

### Example 10

### Immunoprotective Analysis of Combination I and II Vaccine Against Aerosolized M. tuberculosis

Three weeks after the last immunization, the guinea pigs used for the preceding hypersensitivity assay were challenged with aerosolized *M*. *tuberculosis,* Erdman strain as in Example 7 and weighed weekly for 7 weeks. As in Example 17, 6 animals were in each group. During the first 7 weeks after challenge, sham-immunized animals lost an average of 19.5g. In contrast, animals immunized with Combination II (100 µg of each protein) gained 52.4 g and animals immunized with Combination II at a lower dose (20 µg of each protein) gained an average of 67.2g. By way of contrast, animals immunized with Combination I gained 68g. Thus, compared with guinea pigs immunized with CombinationII (100 µg), sham-immunized animals lost 11 % of their total body weight. Compared with guinea pigs immunized with Combination II at a lower dose (20 µg), sham-immunized animals lost 14% of their total body weight. Compared with animals immunized with Combination 1, sham-immunized animals also lost 14% of their total body weight.

### Example 11

### Response of Guinea Pigs Immunized with Combinations III through XII To a Challenge with the Same Vaccine or Its Components

Additional experiments were performed to demonstrate the effectiveness of various combinations of *M*. *tuberculosis* majorly abundant extracellular products. In these studies, Hartley type guinea pigs were immunized intradermally with vaccines comprising combinations of 2 or more majorly abundant extracellular products purified as in Example 2. The purified extracellularproducts are identifiedusing their apparent molecular weight as determined by SDS-PAGE. The guinea pigs were immunized with the following combinations of majorly abundant extracellularproducts.

| Combination | Protein Constituents |
|---|---|
| III | 30+32A+32B+ 16+23 |
| IV | 30 + 32A |
| V | 30 + 32B |
| VI | 30+16 |
| VII | 30 + 23 |
| VIII | 30 + 71 |
| IX | 30 + 23.5 |
| X | 30 + 12 |
| XI | 30+24 |
| XII | 30 + 58 |

Each combination vaccine included 100 mg of each listed protein. The combination vaccines were volumetrically adjusted and injected intradermally in the adjuvant SAF. As before the guinea pigs were immunized four times, three weeks apart.

A cutaneous hypersensitivity assay was performed to determine if the animals had developed a measurable immune response following vaccination with the Combinations III to XII. Groups of six guinea pigs were shaved over the back and injected intradermally with the same combination of purified extracellularproducts to which they were immunized. For this challenge 10 mg of each of the proteins in the combination were injected. All injections were performed using a total volume of 0.1 ml. Sham-immunized controls, which had been immunized with SAF only were also skin-tested with Combinations III to XII, again using 10 mg of each protein in the respective combination. The diameters of erythema and induration at skin tests sites were measured 24 hours after injection as described in Example 3.

The results of these measurements are presented in Table Q below. Data are again reported in terms of mean measurement values for the group ± standard error (SE) as determined using traditional methods.

**Table Q**

| Vaccine Combination | Skin Test Combination | Diameter of Skin Reaction (mm) | |
|---|---|---|---|
| | | Erythema | Induration |
| III | III | 12.2 ± 2.0 | 6.8 ± 0.8 |
| IV | IV | 9.9 ± 0.5 | 6.3 ± 0.2 |
| V | V | 13.0 ± 1.1 | 8.1 ± 0.7 |
| VI | VI | 19.2 ± 1.2 | 12.4 ± 0.5 |
| VII | VII | 14.3 ± 1.0 | 8.7 ± 0.4 |
| VIII | VIII | 18.9 ± 1.1 | 12.6 ± 0.8 |
| IX | IX | 17.0 ± 0.9 | 12.1 ± 0.9 |
| X | X | 19.3 ± 1.4 | 13.6 ± 1.2 |
| XI | XI | 18.3 ± 1.2 | 12.4 ± 0.8 |
| XII | XII | 17.7 ± 0.9 | 14.0 ± 1.2 |
| | | | |
| Sham | III | 4.8 ± 0.9 | 2.0 ± 0.0 |
| Sham | IV | 4.3 ± 1.1 | 2.0 ± 0.0 |
| Sham | V | 5.0 ± 0.5 | 2.0 ± 0.0 |
| Sham | VI | 4.5 ± 0.3 | 2.0 ± 0.0 |
| Sham | VII | 4.5 ± 0.3 | 2.0 ± 0.0 |
| Sham | VIII | 3.3 ± 0.3 | 2.3 ± 0.3 |
| Sham | IX | 3.7 ± 0.3 | 2.0 ± 0.0 |
| Sham | X | 3.7 ± 0.4 | 2.0 ± 0.0 |
| Sham | XI | 3.7 ± 0.2 | 2.0 ± 0.0 |
| Sham | XII | 3.8 ± 0.2 | 2.0 ± 0.0 |

The results clearly demonstrate that a strong cell-mediated immune response was generated to each of the combinations of purified extracellular proteins. The immunized guinea pigs showed erythema at least twice and usually 3 fold or more that of controls for all combinations. Further, the immunized guinea pigs showed induration at least 3 fold that of controls for all combinations.

### Example 12

### Immunoprotective Analysis of Combinations III-XII against Aerosolized M. tuberculosis

To demonstrate the prophylactic efficacy of these exemplary combinations of purified extracellular products, guinea pigs immunized with Combinations III through XII were challenged with *M. tuberculosis* three weeks after the last immunization using the protocol of Example 4.

Consistent with earlier results guinea pigs immunized with Combinations III through XII were all protected against death after challenge. At 4 weeks after challenge, 2 of 6 sham-immunized animals (33%) died compared with 0 animals in groups immunized with Combinations IV-XII and I of 6 animals (17%) in the group immunized with Combination III. At 10 weeks after challenge, 50% of the sham-immunized animals had died compared with 0 deaths in the animals in groups immunized with Combinations IX and XII (0%),1of 6 deaths (17%) in the animals in the groups immunized with Combination III, IV, V, VI, X, and XI, 1 of 5 deaths (20%) in the animals immunized with Combination VIII, and 2 of 6 deaths (33%) in the animals immunized with Combination VII.

Guinea pigs that died before the end of the observation period were autopsied and examined for evidence of gross tuberculosis lesions. Lesions were found in all animals which expired during the study.

Following the conclusion of the mortality study, the surviving animals were sacrificed by hypercarbia and the spleen of each animal was assayed for viable *M tuberculosis* using the protocol of Example 5. The results are presented in Table R below in terms of mean colony forming units (CFU) along with the log decrease from the sham immunized animals. An asterisk next to the CFU value indicates that spleen counts were zero on one animal in each group. For purposes of calculation, zero counts were treated as 10³ CFU per spleen or 3 logs.

**Table R**

| Vaccine Group | CFU in Spleen (Mean Log) | Log Decrease from Sham |
|---|---|---|
| III | 5.99 | 0.5 |
| IV | 5.41 | 1.1 |
| V | 6.27 | 0.3 |
| VI | <5.80* | >0.7 |
| VII | <5.61* | >0.9 |
| VIII | 6.47 | 0.1 |
| IX | <5.85* | >0.7 |
| XXI | <5.74* | >0.8 |
| XII | 5.93 | 0.6 |
| | 6.03 | 0.5 |
| | | |
| Sham | 6.53 | - - |

Animals immunized with Combinations III, IV, VI, VII, IX, X, XI, and XII had at least 0.5 log fewer colony forming units of *M*. *tuberculosis* in their spleens on the average than the sham-immunized controls. In particular, combinations IV and VII proved to be especially effective, reducing the average number of colony forming units by roughly a factor of ten. Animals immunized with Combinations V and VIII had 0.3 and 0.1 log fewer colony forming units (CFU), respectively, in their spleens on average, than sham-immunized controls. This dramatic reduction in colony forming units in the animals immunized in accordance with the teachings of the present invention once again illustrates the immunoprotective operability of the present invention.

### Example 13

### Response of Guinea Pigs Immunized with 3 Different Dosages Of Combination XIII to a Challenge with Combination XIII

To further define the operability and scope of the present invention as well as to demonstrate the efficacy of additional combinations of purified extracellular products, guinea pigs were immunized as before using alternative vaccination dosages. Specifically, 50 mg, 100 mg and 200 mg of an alternative combination of 3 majorly abundant extracellular products identified as Combination XIII and comprising the 30 KD, 32(A) KD, and 16 KD proteins. As with the preceding examples, groups of animals were immunized intradermally 4 times, 3 weeks apart with the alternative dosages of Combination XIII in SAF.

A cutaneous hypersensitivity assay was performed to determine if the animals had developed a measurable immune response following vaccination. The animals were shaved over the back and injected intradermally with Combination XIII containing 10.0 mg of each of the purified extracellular products. All injections were performed using a total volume of 0.1 ml. Sham-immunized controls were also skin-tested with the same dosage of CombinationXIII. The diameters of erythema and induration at skin- test sites were measured 24 hours after injection.

The results are presented in Table S below in terms of mean measurement values for the group ± standard error (SE) as determined using traditional methods

**Table S**

| Vaccine Combination | Vaccine Dose (mg) | Diameter of Skin Reaction (mm) | |
|---|---|---|---|
| | | Erythema | Induration |
| XIII | 50 | 17.8 ± 1.3 | 13.2 ± 1.0 |
| XIII | 100 | 11.2 ± 0.9 | 7.3 ± 0.4 |
| XIII | 200 | 10.0 ± 0.7 | 7.0 ± 0.4 |
| Sham | 0 | 5.7 ± 0.5 | 0.2 ± 0.2 |

Once again, these results clearly demonstrate that a strong cell-mediated immune response to Combination XIII was generated in animals immunized with each of the three dosages of Combination XIII. The immunized animals exhibited erythema about two to three times that of controls. Even more strikingly, the immunized animals exhibited induration at least 35 fold that of control animals which exhibited a minimal response in all cases.

### Example 14

### Immunoprotective Analysis of Combination XIII In Three Different Dosages Against Aerosolized M. tuberculosis

To further demonstrate the protective immunity aspects of the vaccines of the present invention at various dosages, the immunized guinea pigs (6 per group) used for the preceding cutaneous hypersensitivity assay were challenged with aerosolized *M. tuberculosis* three weeks after the last immunization. The aerosol challenge was performed using the protocol detailed in Example 4. A control group of 12 sham-immunized animals was challenged simultaneously.

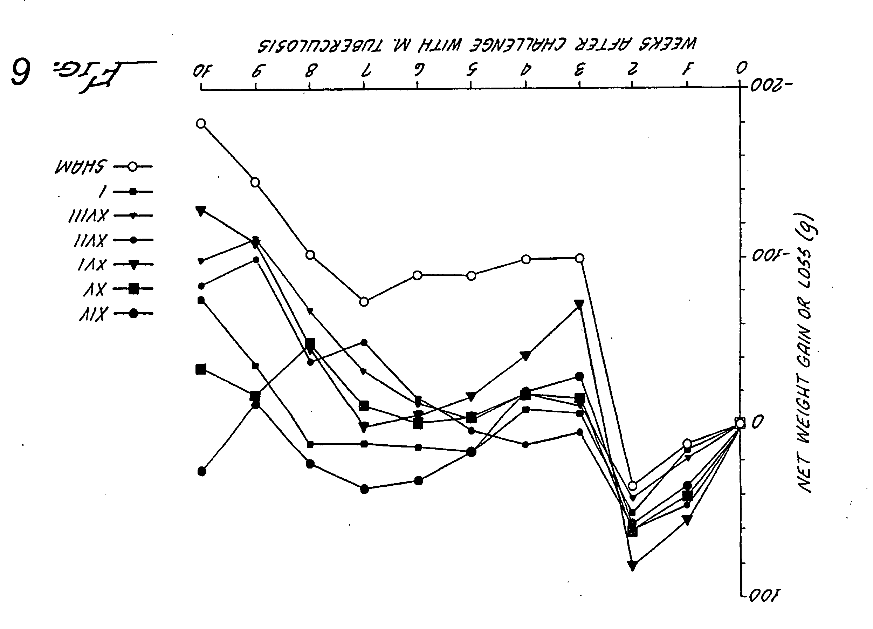

Results of the weekly weight determinations following challenge are graphically represented in Fig. 5 and distinctly show guinea pigs immunized with each of the three dosages of Combination XIII were protected from weight loss. Animals immunized with the higher dosages of Combination XIII (100 and 200 mg) actually showed a net gain in weight and animals immunized with the lower dosage (50 mg) showed a relatively small loss in weight. In contrast, the sham immunized animals lost approximately 22% of their total body weight in the weeks immediately after challenge and averaged a loss of 182 g over the 10 week observation period.

Table U below illustrates the percent weight change for immunized and control animals as determined by taking the mean weight at the end of the challenge, subtracting the mean weight at the start of the challenge and dividing the result by the mean weight at the start of the challenge. Similarly, the percent protection was determined by subtracting the mean percent weight loss of the controls from the mean percent weight gain or loss of the immunized animals.

**Table U**

| Immunogen | Dosage | % Weight Change | % Protection from Weight Loss |
|---|---|---|---|
| Combination XIII | 50 | -4% | 18% |
| Combination XIII | 100 | +7% | 29% |
| Combination XIII | 200 | +5% | 27% |
| | | | |
| Sham | Sham | -22% | - |

Table U shows that the sham-immunizedanimals lost a considerable amount of weight (18% - 29%) over the monitoring period compared with the immunized animals. Fig. 5 provides a more graphic illustration of the net weight loss for each group of immunized animals versus sham-control animals plotted at weekly intervals over the ten week monitoring period. As loss of body mass or "consumption" is one of the classical side effects of tuberculosis, these results indicate that the growth and proliferation of tubercle bacilli in the immunized animals was inhibited by the three different dosages of the exemplary combination vaccine of the present invention.

### Example 15

### Immunoprotective Analysis of Combinations XIV-XVIII Against Challenge with Combinations XIV-XVIII

To further demonstrate the scope of the present invention and the broad range of effective vaccines which may be formulated in accordance with the teachings thereof, five additional combination vaccines, Combinations XIV through XVIII, were tested in guinea pigs. Identified by the apparent molecular weight of the purified extracellular products determined using SDS-PAGE, the composition of each of the combination vaccines is given below.

| Combination | Protein Constituents |
|---|---|
| XIV | 30, 32A, 16, 32B, 24, 23, 45 |
| XV | 30, 32A, 16, 32B, 24, 23, 45, 23.5, 12 |
| XVI | 30, 32A, 16, 32B, 24, 23 |
| XVII | 30, 32A, 16, 32B, 24, 71 |
| XVIII | 30, 32A, 32B |
| I | 30, 32A, 16, 23, 71 |

In addition to the new combination vaccines and appropriate controls, Combination I was also used in this series of experiments. Guinea pigs were immunized intradermally with 50 mg of each protein of Combination XIV or XV and with 100 mg of each protein of Combinations I, XVI, XVII, and XVIII all in SAF adjuvant. The animals were immunized a total of four times, with each injection three weeks apart.

A cutaneous hypersensitivity assay was performed to determine if the animals had developed a measurable immune response following vaccination using the previously discussed protocol. Guinea pigs were shaved over the back and injected intradermally with the same combination of purified extracellular proteins to which they were immunized. For each challenge the appropriate combination vaccine containing 10 mg of each protein was injected. All injections were performed using a total volume of 0.1 ml. Sham-immunized controls were also skin-tested with the same dosage of each combination. The diameters of erythema and induration at skin test sites were measured at 24 hours after injection as described in Example 3.

The results of these measurements are presented in Table V below, reported in terms of mean measurement values for the group ± standard error (SE) as determined using traditional methods.

**Table V**

| Vaccine Combination | Skin Test Combination | Diameter of Skin Reaction (mm) | |
|---|---|---|---|
| | | Erythema | Induration. |
| XIV | XIV | 13.3 ± 0.7 | 9.1 ± 0.4 |
| XV | XV | 10.4 ± 0.4 | 6.5 ± 0.4 |
| XVI | XVI | 8.0 ± 1.8 | 5.1 ± 1.0 |
| XVII | XVII | 9.4 ± 0.9 | 6.1 ± 1.1 |
| XVIII | XVIII | 13.6 ± 1.2 | 8.7 ± 0.7 |
| I | I | 10.0 ± 0.3 | 6.7 ± 0.2 |
| | | | |
| Sham | XIV | 5.5 ± 1.6 | 0.4 ± 0.2 |
| Sham | XV | 6.1 ± 0.5 | 0.4 ± 0.2 |
| Sham | XVI | 4.6 ± 1.4 | 0.4 ± 0.2 |
| Sham | XVII | 5.7 ± 1.2 | 0.2 ± 0.2 |
| Sham | XVIII | 2.1 ± 1.1 | 0 ± 0 |
| Sham | I | 6.0 ± 1.2 | 0.6 ± 0.2 |

These results clearly demonstrate that a strong cell-mediated immune response was generated to Combinations XIV through XVIII, and, as before, to Combination I. Immunized animals exhibited erythema about twice that of controls. Even more strikingly, the immunized animals exhibited induration at least 10 fold greater than the sham-immunized controls which exhibited a minimal response in all cases.

### Example 16

### Immunoprotective Analysis of Combinations XIV-XVIII And Combination I Against Aerosolized M. tuberculosis

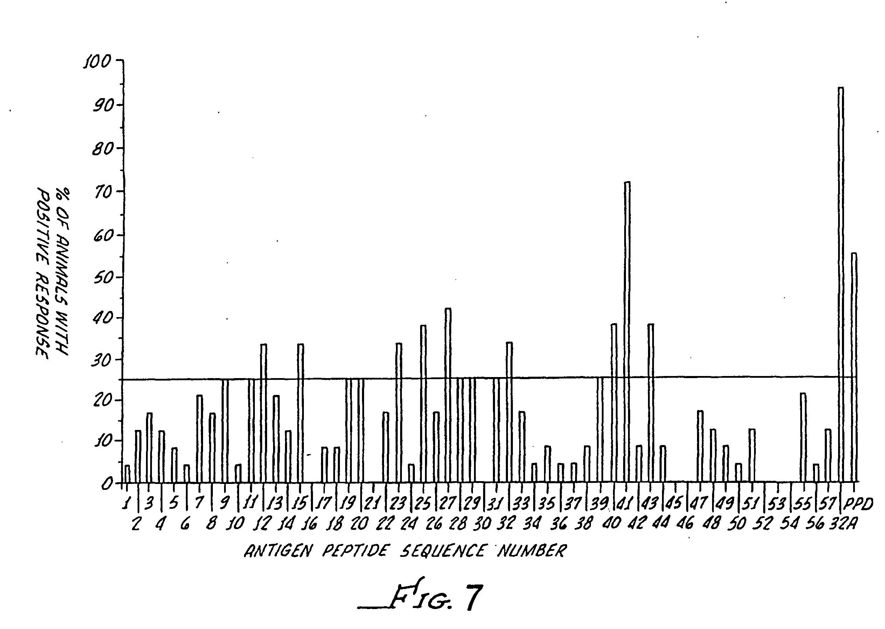

To confirm the immunoreactivity of the combination vaccines of Example 15 and to demonstrate their applicability to infectious tuberculosis, the immunized guinea pigs used for the preceding cutaneous hypersensitivity assay were challenged with aerosolized *M. tuberculosis* three weeks after the last immunization and monitored using the protocol of Example 4. A control group of 12 sham-immunized animals, the same as used in Example 14, was similarly challenged. The results of these challenge are graphically represented in Fig. 6 and shown in Table W directly below.

Percent weight change was determined by taking the mean weight at the end of the challenge, subtracting the mean weight at the start of the challenge and dividing the result by the mean weight at the start of the challenge. Similarly, the percent protection was determined by subtracting the mean percent weight loss of the controls from the mean percent weight gain or loss of the immunized animals.

**Table W**

| Immunogen | % Weight Change | % Protection from Weight Loss |
|---|---|---|
| Combination XIV | 3% | 25% |
| Combination XV | - 4% | 18% |
| Combination XVI | -15% | 7% |
| Combination XVII | -11% | 11% |
| Combination XVIII | -12% | 10% |
| Combination I | -11% | 11% |
| | | |
| Sham | -22% | |

As shown in Table W, guinea pigs immunized with each of the combination vaccines were protected from weight loss. Sham-immunizedanimals lost approximately 22% of their total combined body weight. In contrast the prophylactic effect of the combination vaccines resulted in actual weight gain for one of the test groups and a reduced amount of weight loss in the others. Specifically, animals immunized with Combination XIV evidenced a 3% weight gain while those animals immunized with the other combinations lost only 4% to 15% of their total combined weight.

These results are shown graphically in Fig. 6 which plots weekly weight determinations in terms of net weight gain or loss for each group of animals following aerosolized challenge. This statistically significant difference between the net weight loss for the immunized animals and the sham-immunized controls shown in Fig. 6 provides further evidence for the immunoprophylactic response generated by the combination vaccines of the present invention.

### Example 17

### Cell-Mediated Immunity in Guinea Pigs Immunized with Three Different Adjuvants

Immunogenic studies were conducted using different adjuvants. Specifically three different immunogens, purified 30 KD protein, Combination I (30, 32A, 16, 23, 71) and Combination XIII (30, 32A, 16) were each formulated using three different adjuvants, Syntex Adjuvant Formulation I (SAF), incomplete Freunds adjuvant (IFA) and Monophosphoryl Lipid A containing adjuvant (MPL). Such adjuvants are generally known to enhance the immune response of an organism when administered with an immunogen.

Guinea pigs were immunized intradermally with 100 mg of each protein comprising Combinations I and XIII and approximately 100 mg of purified 30 KD protein in each of the three different adjuvant formulations. The guinea pigs were immunized with each formulation a total of three times with injections three weeks apart.

Following immunization, a cutaneous hypersensitivity assay was performed to determine if the guinea pigs had developed a measurable immune response. Guinea pigs were shaved over the back and injected intradermally with the same immunogen to which they had been immunized. For the challenge, 10 mg of each protein in Combinations I and XIII or 10 mg of purified 30 KD protein was injected in a total volume of 100 ml. Sham-immunized guinea pigs, vaccinated with one of the three adjuvants, were skin-tested with each of the immunogen formulations containing the same adjuvant. The diameters of erythema and induration at skin test sites were measured 24 hours after challenge as described in Example 3.

The results of these measurements are presented in Table X below. As previously discussed data are reported in terms of mean measurement values for the group ± standard error as determined using accepted statistical techniques.

**Table X**

| Vaccine | Adjuvant | Skin Test Reagent | Diameter of Skin Reaction (mm) | |
|---|---|---|---|---|
| | | | Erythema | Induration |
| 30 | SAF | 30 | 10.7 ± 1.6 | 5.8 ± 1.5 |
| 30 | IFA | 30 | 8.8 ± 0.7 | 4.6 ± 0.7 |
| 30 | MPL | 30 | 10.2 ± 1.7 | 5.3 ± 1.5 |
| | | | | |
| XIII | SAF | XIII | 7.3 ± 0.5 | 4.1 ± 0.5 |
| XIII | IFA | XIII | 6.8 ± 0.9 | 3.5 ± 0.5 |
| XIII | MPL | XIII | 6.3 ± 0.4 | 3.4 ± 0.3 |
| | | | | |
| I | SAF | I | 6.9 ± 0.6 | 4.0 ± 0.3 |
| I | IFA | I | 6.8 ± 0.2 | 3.6 ± 0.3 |
| I | MPL | I | 7.4 ± 0.4 | 3.9 ± 0.5 |
| | | | | |
| Sham | SAF | 30 | 0.7 ± 0.7 | 1.0 ± 0 |
| Sham | IFA | 30 | 0 ± 0 | 0 ± 0 |
| Sham | MPL | 30 | 0 ± 0 | 0 ± 0 |
| | | | | |
| Sham | SAF | XIII | 1.0 ± 1.0 | 1.0 ± 0 |
| Sham | IFA | XIII | 0 ± 0 | 0.3 ± 0.3 |
| Sham | MPL | XIII | 0 ± 0 | 0 ± 0 |
| | | | | |
| Sham | SAF | I | 4.7 ± 0.3 | 1.0 ± 0 |
| Sham | IFA | I | 2.0 ± 1.0 | 0.7 ± 0.3 |
| Sham | MPL | I | 1.0 ± 1.0 | 0.7 ± 0.3 |

As shown in the data presented in Table X, the combination vaccines and purified extracellularproducts of the present invention provide a strong cell-mediated immunogenic response when formulated with different adjuvants. Moreover, each one of the three adjuvants provided about the same immunogenic response for each respective immunogen. In general, the immunized guinea pigs exhibited erythema diameters approximately seven to ten times that of the sham-immunizedguinea pigs while indurations were approximately four to six times greater than measured in the control animals.

The ability of the present invention to provoke a strong immunogenic response in combination with different adjuvants facilitates vaccine optimization. That is, adjuvants used to produce effective vaccine formulations in accordance with the teachings herein may be selected based largely on considerationof secondary criteria such as stability, lack of side effects, cost and ease of storage. These and other criteria, not directly related to the stimulation of an immune response, are particularly important when developing vaccine formulations for widespread use under relatively primitive conditions.

### Example 18

### Immunoprotective Analysis of Combinations XIX-XXVIII Against Challenge with Combinations XIX-XXVIII

The generation of an immune response using ten additional combination vaccines, Combinations XIX through XXVIII was further demonstrated. In addition to the new combination vaccines and appropriate controls, Combinations IV and XIII were also used as positive controls to provoke an immune response in guinea pigs. Identified by the apparent molecular weight of the purified extracel lularproducts determined using SDS-PAGE, the composition of each of the combination vaccines is given below.

| Combination | Protein Constituents |
|---|---|
| XIX | 30, 32A, 23 |
| XX | 30, 32A, 23.5 |
| XXI | 30, 32A, 24 |
| XXII | 30, 32A, 71 |
| XXIII | 30, 32A, 16,23 |
| XXIV | 30, 32A, 16,23.5 |
| XXV | 30, 32A, 16,24 |
| XXVI | 30, 32A, 16, 71 |
| XXVII | 30, 32A, 16, 32B |
| XXVIII | 30, 32A, 16,45 |
| IV | 30, 32A |
| XIII | 30, 32A, 16 |

The guinea pigs were immunized a total of four times, with each injection three weeks apart. Each combination vaccine used to immunize the animals consisted of 100 mg of each protein in SAF adjuvant to provide a total volume of 0.1 ml.

Using the protocol discussed in Example 3, a cutaneous hypersensitive assay was performed to determine if the animals had developed a measurable immune response following vaccination with the selected combination vaccine. The guinea pigs were shaved over the back and injected intradermally with the same combination of purified extracellular proteins with which they were immunized. The protein combinations used to challenge the animals consisted of 10 mg of each protein. Sham immunized controls were also skin-tested with the same dosage of each combination. As in Example 3, the diameters of erythema and induration at the skin test sites were measured at 24 hours after injection.

The results of these measurements are presented in Table Y below, reported in terms of mean measurement values for the group of animals ± standard error.

**Table Y**

| | | | |
|---|---|---|---|
| Vaccine Combination | Skin Test Combination | Diameter of Skin Reaction (mm) | |
| | | Erythema | Induration |
| XIX | XIX | | 3.9 ± 0.3 |
| XX | XX | 8.5 ± 0.6 | 3.7 ± 0.3 |
| XXI | XXI | 8.2 ± 0.3 | 4.5 ± 0.4 |
| XXII | XXII | 11.1 ± 1.1 | 4.3 ± 0.4 |
| XXIII | XXIII | 9.4 ± 0.8 | 3.0 ± 0.3 |
| XXIV | XXIV | 8.3 ± 1.1 | 3.4 ± 0.5 |
| XXV | XXV | 8.5 ± 0.9 | 3.2 ± 0.4 |
| XXVI | XXVI | 7.9 ± 0.5 | 3.3 ± 0.5 |
| XXVII | XXVII | 8.9 ± 0.7 | 2.8 ± 0.5 |
| XXVIII | XXVIII | 7.2 ± 1.0 | 2.8 ± 0.3 |
| IV | IV | B.5 ± 0.5 | 4.1 ± 0.3 |
| XIII | XIII | 9.0 ± 0.9 | 4.3 ± 0.3 |
| | | 9.4 ± 0.9 | |
| Sham | XIX | | 1.0 ± 0 |
| Sham | XX | 4.0 ± 2.6 | 1.0 ± 0 |
| Sham | XXI | 1.3 ± 1.3 | 1.3 ± 1.3 |
| Sham | XXII | 3.5 ± 1.0 | 1.0 ± 1.0 |
| Sham | XXIII | 1.3 ± 1.3 | 1.0 ± 0 |
| Sham | XXIV | 0 ± 0 | 1.0 ± 0 |
| Sham | XXV | 0 ± 0 | 1.0 ± 0 |
| Sham | XXVI | 0 ± 0 | 2.0 ± 1.0 |
| Sham | XXVII | 2.3 ± 2.3 | 1.0 ± 0 |
| Sham | XXVIII | 0 ± 0 | 1.0 ± 0 |
| Sham | IV | 2.0 ± 1.2 | 1.0 ± 0 |
| Sham | XIII | 2.8 ± 1.6 | 1.0 ± 0 |
| | | 1.5 ± 1.5 | |

The results presented in Table Y explicitly show that a strong cell-mediated immune response was generated to Combinations XIX through XXVIII when challenged with the same immunogens. As before, a strong cell-mediated immune response was also provoked by Combinations IV and XIII. The erythema exhibited by the immunized guinea pigs was at least twice, and generally proved to be and more then four fold greater than, the reaction provoked in the corresponding sham immunized control animals. Similarly, the induration exhibited by the immunized animals was at least twice, and generally three to four times greater than, that of the nonimmunized controls. The substantially stronger immune response generated among the animals immunized in accordance with the teachings of the present disclosure once again illustrates the immunoprotective operability of the combination vaccines of the present disclosure.

Those skilled in the art will also appreciate additional benefits of the vaccines and methods of the present disclosure. For example, because individual compounds or selected combinationsof highly purified molecular species are used for the subject vaccines rather than whole bacteria or components thereof, the vaccines of the present invention are considerably less likely to provoke a toxic response when compared with prior art attenuated or killed bacterial vaccines. Moreover, the molecular vaccines of the present disclosure are not life threatening to immunocompromised individuals. In fact, the compositions of the present invention may be used therapeutically to stimulate a directed immune response to a pathogenic agent in an infected individual.

Selective use of majorly abundant extracellular products or their immunogenic analogs also prevents the development of an opsonizing humoral response which can increase the pathogenesis of intracellular bacteria. As the protective immunity generated by this invention is directed against unbound proteins, any opsonic response will simply result in the phagocytosis and destruction of the majorly abundant extracellular product rather than the expedited inclusion of the parasitic bacteria. Moreover, the selective use of purified extracellular products reduces the potential for generating a response which precludes the use of widely used screening and control techniques based on host recognition of immunogenic agents. Unlike prior art vaccines, the screening tests could still be performed using an immunoreactive molecule that is expressed by the pathogen but not included in the vaccines made according to the present invention. The use of such an immunogenic determinant would only provoke a response in those individuals which had been exposed to the target pathogen allowing appropriate measures to be taken.

Another advantage of the present disclosure that purified extracellular products are easily obtained in large quantities and readily isolated using techniques well known in the art as opposed to the attenuated bacteria and bacterial components of prior art vaccines. Since the immunoreactive products of the present disclosure are naturally released extracellularly into the surrounding media for most organisms of interest, removal of intracellular contaminants and cellular debris is simplified. Further, as the most prominent or majorly abundant extracellular products or immunogenic analogs thereof are used to stimulate the desired immune response, expression levels and culture concentrations of harvestable product is generally elevated in most production systems. Accordingly, whatever form of production is employed, large scale isolation of the desired products is easily accomplished through routine biochemical procedures such as chromatography or ultrafiltration. These inherent attributes and molecular characteristics of the immunogenic determinants used in the present invention greatly facilitate the production of a consistent, standardized, high quality composition for use on a large scale.

Alternatively, the use of purified molecular compounds based on the immunogenic properties of the most prominent or majorly abundant extracellular products of target pathogens also makes the large scale synthetic generation of the immunoactive vaccine components of the present disclosure relatively easy. For instance, the extracellular products of interest or their immunogenic analogs may be cloned into a nonpathogenic host bacteria using recombinant DNA technology and harvested in safety. Molecular cloning techniques well known in the art may be used for isolating and expressing DNA corresponding to the extracellular products of interest, their homologs or any segments thereof in selected high expression vectors for insertion in host bacteria such as *Escherichia coli.* Exemplary techniques may be found in II R. Anon, Synthetic Vaccines 31-77 (1987), Tam et al., Incorporation of T and B Epitopes of the Circumsporozoite Protein in a Chemically Defined Synthetic Vaccine Against Malaria. 171 J. Exp. Med. 299-306 (1990), and Stover et al., Protective Immunity Elicited by Recombinant Bacille Calmette-Guerin (BCG) Expressing Outer Surface Protein A (OspA) Lipoprotein: A Candidate Lyme Disease Vaccine, 178 J. Exp. Med.197-209 (1993).

The present disclosure involves a process for using a host cell to produce a majorly abundant extracellular product selected from the group consisting of *M. tuberculosis* 110 KD protein, 80 KD protein, 71 KD protein, 58 KD protein, 45 KD protein, 32A KD protein, 32B KD protein, 30 KD protein, 24 KD protein, 23.5 KD protein, 23 KD protein, 16 KD protein, 14 KD protein, 12 KD protein and respective analogs, homologs, and subunits thereof. Examples of practice demonstrating preferred methods for expressing the extracellular proteins of the present invention are as follows:

### Example 19

### Expression of Soluble. Processed. Extracellular. M. tuberculosis 32A KD Major Secretory Protein Using the Plasmid pSMT3 in Mycobacterium smegmatis and Mycobacterium vaccae

This example is directed to demonstrating the expression and secretion of the *M. tuberculosis* 32A KD major secretory protein in a mycobacterium. We used the pSMT3 plasmid (Dr. Douglas B. Young, Dept. Medical Microbiology, St. Mary's Hospital Medical School, Norfolk Place, London, W2 1PG, United Kingdom, a 5.7 kb (kilo basepairs) plasmid with both *E. coli* (col E1 ori) and mycobacterium *(Mycobacterium fortuitum* plasmid pAL5000 ori) origins of replication.

Successful expression in pET22b of the 30 and 32A KD *M. tuberculosis* proteins was obtained by adding the respective leader sequence of the protein in front of the structural gene. For both proteins, this resulted in expression of both the full-length and processed protein. These constructs were relatively stable in *E. coli,* i.e. they expressed the recombinant proteins after 2 or 3 subcultures but not after additional subculturing.

Successful expression in pET22b of the 30 and 32A KD *M. tuberculosis proteins* was also obtained by adding the E. coli-derived pelB leader sequence in front of the structural gene for each protein. Expression levels in these constructs were even higher than in those utilizing the respective leader sequences of the 30 or 32A KD proteins. However, a drawback of the pelB constructs was their instability. These constructs lost their ability to express any recombinant protein after one subculture

Previously, expression and secretion of the *M. tuberculosis* 32A KD protein had been achieved in *Mycobacterium smegmatis* and *Mycobacterium vaccae* using pSMT3 based expression constructs containing a ~4.5 kb DNA fragment which was flanked by *Eco*RV restriction sites and which encoded the full-length 32A protein gene. However, in contrast to the case with the *M. tuberculosis* 30 KD major extracellular protein and the *M. tuberculosis* 16 KD major extracellular protein, use of these constructs resulted in the intracellular accumulation of *M*. *tuberculosis* 32A KD protein and did not yield high-level secretion of the *M.* tuberculosis 32A KD protein.

The present disclosure functions by culturing the recombinant strains of *Mycobacterium smegmatis* containing DNA fragments encoding the full-length 32A KD major extracellular protein at 28°C rather than 37°C, the usual temperature for culturing these bacteria. When cultured at 28°C, the recombinant mycobacterium expresses and secretes large quantities of the 32A KD protein. The secreted protein can then be purified from the culture supernatant fluid by conventional purification techniques.

Prior to the present disclosure, only small amounts of recombinant *M. tuberculosis* 32A KD protein could be obtained from recombinant mycobacteria expressing the protein. The invention allows large amounts of recombinant 32A protein to be obtained from the culture filtrate of recombinant mycobacteria, which secrete the 32A KD protein into the culture medium. Thus, the cost-effective production of recombinant 32A KD protein becomes possible.

### Example 20

The gene from the *M tuberculosis* 32A KD major extracellular protein was cloned into *Mycobacterium smegmatis* using a pSMT3-based expression construct containing an ~4.5 kb DNA fragment, flanked by *Eco*RV restriction sites, that encoded the full-length 32A KD protein (Construct A in Fig. 19). The recombinant *M. smegmatis* was cultured in 7H9 medium containing 2% glucose at 37°C and 28°C. The culture filtrate of each organism was then collected and subjected to SDS-PAGE analysis. When *M. smegmatis* containing the pSMT3 vector with the full-length 32A KD protein gene was cultured at 37°C, virtually no 32A KD protein was present in the culture filtrate (Lane 2 of Fig. 20). However, when the same *M smegmatis* strain was cultured at 28°C, a large amount of 32A KD protein was secreted and present in the culture filtrate (Lane 3, arrowhead). Lane 1 shows molecular weight standards, the molecular weight x 10⁻³ being noted to the left of the standards. Lane 2 shows filtrate from *M smegmatis* containing the pSMT3 vector with the full-length *M tuberculosis* 32A KD protein gene that was cultured at 37°C. Lane 3 shows filtrate from *M smegmatis* containing pSMT3 vector with the full-length *M. tuberculosis 32A* KD protein gene that was cultured at 28°C.

*M smegmatis* containing constructs B, D, E, and G also expressed and secreted large amounts of recombinant *M*. *tuberculosis* 32A KD protein when cultured at 28°C.

An N-terminal amino acid analysis of recombinant *Mycobacterium tuberculosis* 32A KD protein expressed and secreted by *Mycobacterium smegmatis* at 28°C gives the following sequence:

Two recombinant *M. smegmatis* strains (Constructs A and D) containing the full length *M tuberculosis* 32A KD protein gene were cultured at 28°C. The culture filtrate of each organism was obtained and an aliquot subjected to denaturing SDS-PAGE analysis. The proteins were transferred to polyvinylidine fluoride (PVDF) membranes and the Coomassie blue R stained band migrating at 32 KD was cut out and subjected to automated amino acid sequence determination. The sequence shown above is in the conventional one-letter code.

The N-terminal sequence of the recombinant protein secreted by *M smegmatis* containing either Construct A or D is identical to the native *M tuberculosis* 32A KD protein, confirming that the recombinant *M tuberculosis* 32A KD protein in *M smegmatis* is processed the same way as the native *M tuberculosis* 32A KD in *M tuberculosis.*

Similarly, the extracellularproteins, their analogs, homologs or immunoreactive protein subunits may be chemically synthesized on a large scale in a relatively pure form using common laboratory techniques and automated sequencer technology. This mode of production is particularly attractive for constructing peptide subunits or lower molecular weight analogs corresponding to antigenic determinants of the extracellular products. Exemplary techniques for the production of smaller protein subunits are well known in the art and may be found in II R. Anon, Synthetic Vaccines 15-30 (1987), and in A. Streitwieser, Jr., Introduction to Organic Chemistry 953-55 (3rd ed. 1985). Alternative techniques may be found in Gross et al., "Nonenzymatic Cleavage of Peptide Bonds: The Methionine Residues in Bovine Pancreatic Ribonuclease," 237 The Journal of Biological Chemistry No. 6 (1962), Mahoney, "High-Yield Cleavage of Tryptophanyl Peptide Bonds by o-Iodosobenzoic Acid," 18 Biochemistry No. 17 (1979), and Shoolnik et al., "Gonococcal Pili," 159 Journal of Experimental Medicine (1984). Other immunogenic techniques such as anti-idiotyping or directed molecular evolution using peptides, nucleotides or other molecules such as mimetics can also be employed to generate effective, immunoreactive compounds capable of producing the desired prophylactic response.

Nucleic acid molecules useful for the practice of the present disclosure may be expressed from a variety of vectors, including, for example, viral vectors such as herpes viral vectors (e.g., U.S. Patent No. 5,288,641), retroviruses (e.g., EP 0,415,731; WO 90/07936, WO 91/0285, WO 94/03622; WO 93/25698; WO 93/25234; U.S. Patent No. 5,219,740; WO 89/09271; WO 86/00922; WO 90/02797; WO 90/02806; U.S. Patent No. 4,650,764; U.S. Patent No. 5,124,263; U.S. Patent No. 4,861,719; WO 93/11230; WO 93/10218; Vile and Hart, Cancer Res. 53:3860-3864, 1993; Vile and Hart, Cancer Res. 53:962-967, 1993; Ram et al., Cancer Res. 53:83-88, 1993; Takamiya et al., J. Neurosci. Res. 33:493-503, 1992; Baba et al., J. Neurosurg. 79:729-735, 1993), pseudotyped viruses, adenoviral vectors (e.g., WO 94/26914, WO 93/9191; Kolls et al., PNAS 91(1):215-219, 1994; Kass-Eisleret al., PNAS 90(24):11498-502, 1993; Guzman et al., Circulation 88(6):2838-48, 1993; Guzman et al., Cir. Res. 73(6):1202-1207, 1993; Zabneret al., Cell 75(2):207-216, 993; Li et al., Hum. Gene Ther. 4(4):403-409, 1993; Caillaud et al., Eur. J. Neurosci. 5(10:1287-1291, 1993; Vincent et al., Nat. Genet. 5(2):130=134, 1993; Jaffe et al., Nat. Genet. 1(5):372-378, 1992; and Levrero et al., Gene 101(2):195-202,1991), adenovirus-associated viral vectors (Flotte et al., PNAS 90(22): 10613-10617, 1993), parvovirus vectors (Koering et al., Hum. Gene Therap. 5:457-463, 1994), and pox virus vectors (Panicali and Paoletti, PNAS 79:4927-4931, 1982).

The nucleic acid molecules (or vectors. i.e., an assembly capable of directing the expression of a sequence of interest) may be introduced into host cells by a wide variety of mechanisms, including, for example, transfection, including, for example, DNA linked to killed adenovirus (Michael et al., J. Biol. Chem. 268(10:6866-6869, 1993; and Curiel et al., Hum. Gene Ther. 3(2):147-154. 1992), cytofectin=mediated introduction (DMRIE-DOPE, Vical, Calif.), direct DNA injection (Acsadi et al., Nature 352:815-818, 1991); DNA ligand (Wu et al., J. of Biol. Chem. 264:16985-16987,1989); lipofection (Felgner et al., Proc. Natl. Acad Sci. USA 84:7413-7417, 1989); liposomes(Pickeringet al., Circ. 89(1):13-21, 1994; and Wang et al., PNAS 84:7851-7855. 1987); microprojectile bombardment (Williams et al., PNAS 88:2726-2730, 1991); and direct delivery of nucleic acids which encode the enzyme itself, either alone (Vile and hart. Cancer Res. 53:3860-3864, 1993), or utilizing PEG-nucleic acid complexes (see also WO 93/18759; WO 93/04701; WO 93/07283 and WO 93/07282).

As an additional alternative, DNA or other genetic material encoding one or more genes capable of inducing the expression of one or more of the extracellular products, homologs, analogs, or subunits of the present disclosure can be directly injected into a mammalian host utilizing so called "naked DNA" techniques. Following the *in vivo* introduction and the resultant uptake of the genetic construct by the host's cells the host will begin the endogenous production of the one or more encoded immunoreactive products engendering an effective immune response to subsequent challenge. As those skilled in the art will appreciate, coupling the genetic construct to eucaryotic promoter sequences and/or secretion signals may facilitate the endogenous expression and subsequent secretion of the encoded immunoreactive product or products. Exemplary techniques for the utilization of naked DNA as a vaccine can be found in International Patent No. WO 9421797 A (Merck & Co. Inc. and ViCal Inc.), International Patent Application No. WO 9011092 (ViCal Inc.), and Robinson. Protection Against a Lethal Influenza Virus Challenge by Immunization with a Hemagglutinin-Expressing Plasmid DNA.11 Vaccine 9 (1993), and in Ulmer et al., Heterologous Protection Against Influenza by Injection of DNA Encoding a Viral Protein, 259 Science (1993).

Alternatively, techniques for the fusion of a strongly immunogenic protein tail have been disclosed in Tao et al., Idiotype/Granulocyte-MacrophargeColony-Stimulating Factor Fusion Protein as a Vaccine for B-Ceo Lymphoma, 362 Nature (1993), and for T-cell epitope mapping in Good et al., Human T-Cell Recognition of the Circumsporozoite Protein of Plasmodium falciparum: Immunodominant T-Cell Domains Map to the Polymorphic Regions of the Molecule, 85 Proc. Natl. Acad. Sci. USA (1988), and Gao et al., Identification and Characterization of T Helper Epitopes in the Nucleoprotein of Influenza A Virus, 143 The Journal of Immunology No. 9 (1989).

As many bacterial genera exhibit homology, the foregoing examples are provided for the purposes of illustration.

It should also be reemphasized that the prevalence of interspecies homology in the DNA and corresponding proteins of microorganisms enables the vaccines of the present invention to induce cross-reactive immunity. Because the immunodominant epitopes of the majorly abundant extracellular products may provide cross-protective immunity against challenge with other serogroups and species of the selected genera those skilled in the art will appreciate that vaccines directed against one species may be developed using the extracellular products or immunogenic analogs of another species.

For example. *M*. *bovis* is between 90% and 100% homologous with *M*. *tuberculosis* and is highly cross-reactive in terms of provoking an immune response. Accordingly, vaccines based on abundant extracellular products of *M. bovis* or other *Mycobacterium* can offer various degrees of protection against infection by *M. tuberculosis* and vice versa. Thus, it is contemplated as being within the scope of the present disclosure to provide an immunoprophylactic response against several bacterial species of the same genera using an highly homologous immunogenic determinantof an appropriate majorly abundant extracellular product.

It should also be emphasized that the immunogenic determinant selected to practice the present disclosure may be used in many different forms to elicit an effective protective or immunodiagnostic immune response. Thus the mode of presentation of the one or more immunogenic determinants of selected maj orly abundant extracellular products to the host immune system is not critical and may be altered to facilitate production or administration. For example, the vaccines of the present disclosure may be formulated using whole extracellular products or any immunostimulating fraction thereofincluding peptides, protein subunits, immunogenic analogs and homologs as noted above.

In accordance with the teachings of the present disclosure, effective protein subunits of the majorly abundant extracellular products of *M*. *tuberculosiscan* be identified in a genetically diverse population of a species of mammal. The resultant immunodominant T-cell epitopes identified should be recognized by other mammals including humans and cattle. These immunodominant T-cell epitopes are therefore useful for vaccines as well as for immunodiagnostic reagents. An exemplary study identifying the immunodominant T-cell epitopes of the 30 KD major secretory protein of *M*. *tuberculosis* was conducted as follows.

### Example 21

### Protocol for Immunodominant Epitose Mapping of a Protein

Synthetic peptides (15-mers) covering the entire native protein and overlapping by 10 amino acids were used for splenic lymphocyte proliferation assays to identify the immunodominant T-cell epitopes of a major secretory protein of *M. tuberculosis* 55. Control animals received phosphate buffered saline in SAF. Cell mediated immune responses were evaluated by skin testing as described above. Lymphocytes were seeded in 96-well tissue culture plates (Falcon Labware) and incubated in triplicate with the synthetic 15-mer peptides at 20 mg ml⁻¹, purified protein at 20 mg ml⁻¹, purified protein derivative [(PPD); Connaught Laboratories LTD] at 20 mg ml⁻¹, or concanavalin A at 10 mg ml⁻¹ for 2 days in the presence of 10 U polymyxin B. Subsequently, cells were labeled for 16 h with 1 mCi [³H) thymidine (New England Nuclear) and then harvested (Breiman and Horwitz, 1987). A positive proliferative response was defined as follows: (dpm of antigen) - (dpm of medium)³ 1 500 and (dpm of antigen)/(dpm of medium)³ 1.2.

Previously, two types of studies aimed at identifying epitopes of the M. tuberculosis 32A KD protein have been conducted. The first type examined the T-cell responses to peptides (overlapping the entire sequence of the protein) of humans who were a) tuberculin (PPD) positive; in part as a result of vaccination with BCG; b) lepromin positive; c) had TB; or d) had leprosy. (P. Launois, R. Deleys, M.N. Niang, A. Drowart, M. Adrien. P. Deirckx, J.-L. Cartel, J.-L. Sarthou, J.-P. van Vooren, and K. Huygen, 1994, "T-Cell epitope mapping of the major secreted mycobacterial antigen AG85A in tuberculosis and leprosy", Infect. and Immun. 62:3679-3687.) The response to this protein in these people is often weak, making it difficult to map epitopes accurately. In order to overcome these difficulties, outbred guinea pigs were immunized with purified protein, focusing the immune system on this single protein and producing a very strong cell-mediated immune response. The response was studied at the peak of T-cell responsiveness, i.e., within a few weeks of immunization.

The second type of previous study examined T-cell responses to peptides of inbred mice infected with BCG. (K. Huygen, E. Lozes, B. Gilles, A. Drowart, K. Palfliet, F. Jurion, I. Roland, M. Art, M. DuFaux, J. Nyabenda, J. De Bruyn, J.-P. van Vooren, and R. Deleys, 1994, "Mapping of TH1 helper T-cell epitopes on major secreted mycobacterial antigen 85A in mice infected with live Mycobacterium bovis BCG", Infect. and Immun., 62:363-370.) Such a study is unlikely to yield results predictive of the diverse responses of multiple MHC types found in humans.

To obtain predictive results, in accordance with the present invention, a large number of outbred guinea pigs with a wide diversity of HLA types were studied. The epitopes of the 32A KD protein recognized by 25% or more of the guinea pigs therefore are strongly immunodominantand recognized by multiple HLA types. Hence, they are likely to be generally recognized by HLA molecules of other species including humans, cattle, and other mammals. Such study should be highly predictive of immunodominant epitopes recognized by humans and other mammals. In addition, the mouse study referred to above relied on responses of BCG-infected animals. Such animals may a) not reflect *M. tuberculosis-*infected animals and b) not develop as strong a response to a specific protein-in this case, the 32A KD protein-as animals without active disease.

### Example 22

### Immunodominant Epitope Mapping of the 32A KD Protein

The procedure of Example 21 was carried out using the fifty-seven synthetic peptides (15-mers) covering the entire native 32A KD proteins. The sequence of each 15-mer targeted is shown in Table AB:

**Table AB**

| No. | Residues | Peptide Sequence | Seq. ID No. |
|---|---|---|---|
| 1 | 1-15 | F S R P G L P V E Y L Q V P S | 96 |
| 2 | 6-20 | L P V E Y L Q V P S P S M G R | 97 |
| 3, | 11-25 | L Q V P S P S M G R D I K V Q | 98 |
| 4 | 16-30 | P S M G R D I K V Q F Q S G G | 99 |
| 5 | 21-35 | D I K V Q F Q S G G A N S P A | 100 |
| 6 | 26-40 | F Q S G G A N S P A L Y L L D | 101 |
| 7 | 31-45 | A N S P A L Y L L D G L R A Q | 102 |
| 8 | 36-50 | L Y L L D G L R A Q D D F S G | 103 |
| 9 | 41-55 | G L R A Q D D F S G W D I N T | 104 |
| 10 | 46-60 | D D F S G W D I N T P A F E W | 105 |
| 11 | 51-65 | W D I N T P A F E W Y D Q S G | 106 |
| 12 | 56-70 | P A F E W Y D Q S G L S V V M | 107 |
| 13 | 61-75 | Y D Q S G L S V V M P V G G Q | 108 |
| 14 | 66-80 | L S V V M P V G G Q S S F Y S | 109 |
| 15 | 71-85 | P V G G Q S S F Y S D W Y Q P | 110 |
| 16 | 76-90 | S S F Y S D W Y Q P A C G K A | 111 |
| 17 | 81-95 | D W Y Q P A C G K A G C Q T Y | 112 |
| 18 | 86-100 | A C G K A G C Q T Y K W E T F | 113 |
| 19 | 91-105 | G C Q T Y K W E T F L T S E L | 114 |
| 20 | 96-110 | K W E T F L T S E L P G W L Q | 115 |
| 21 | 101-115 | L T S E L P G W L Q A N R H V | 116 |
| 22 | 106-120 | P G W L Q A N R H V K P T G S | 117 |
| 23 | 111-125 | A N R H V K P T G S A V V G L | 118 |
| 24 | 116-130 | K P T G S A V V G L S M A A S | 119 |
| 25 | 121-135 | A V V G L S M A A S S A L T L | 120 |
| 26 | 126-140 | S M A A S S A L T L A I Y H P | 121 |
| 27 | 131-145 | S A L T L A I Y H P Q Q F V Y | 122 |
| 28 | 136-150 | A I Y H P Q Q F V Y A G A M S | 123 |
| 29 | 141-155 | Q Q F V Y A G A M S G L L D P | 124 |
| 30 | 146-160 | A G A M S G L L D P S Q A M G | 125 |
| 31 | 151-165 | G L L D P S Q A M G P T L I G | 126 |
| 32 | 156-170 | S Q A M G P T L I G L A M G D | 127 |
| 33 | 161-175 | P T L I G L A M G D A G G Y K | 128 |
| 34 | 166-180 | L A M G D A G G Y K A S D M W | 129 |
| 35 | 171-185 | A G G Y K A S D M W G P K E D | 130 |
| 36 | 176-190 | A S D M W G P K E D P A W Q R | 131 |
| 37 | 181-195 | G P K E D P A W Q R N D P L L | 132 |
| 38 | 186-200 | P A W Q R N D P L L N V G K L | 133 |
| 39 | 191-205 | N D P L L N V G K L I A N N T | 134 |
| 40 | 196-210 | N V G K L I A N N T R V W V Y | 135 |
| 41 | 201-215 | I A N N T R V W V Y C G N G K | 136 |
| 42 | 206-220 | R V W V Y C G N G K P S D L G | 137 |
| 43 | 211-225 | C G N G K P S D L G G N N L P | 138 |
| 44 | 216-230 | P S D L G G N N L P A K F L E | 139 |
| 45 | 221-235 | G N N L P A K F L E G F V R T | 140 |
| 46 | 226-240 | A K F L E G F V R T S N I K F | 141 |
| 47 | 231-245 | G F V R T S N I K F Q D A Y N | 142 |
| 48 | 236-250 | S N I K F Q D A Y N A G G G H | 143 |
| 49 | 241-255 | Q D A Y N A G G G H N G V F D | 144 |
| 50 | 246-260 | A G G G H N G V F D F P D S G | 145 |
| 51 | 251-265 | N G V F D F P D S G T H S W E | 146 |
| 52 | 256-270 | F P D S G T H S W E Y W G A Q | 147 |
| 53 | 261-275 | T H S W E Y W G A Q L N A M K | 148 |
| 54 | 266-280 | Y W G A Q L N A M K P D L Q R | 149 |
| 55 | 271-285 | L N A M K P D L Q R A L G A T | 150 |
| 56 | 276-290 | P D L Q R A L G A T P N T G P | 151 |
| 57 | 281-295 | A L G A T P N T G P A P Q G A | 152 |

As shown in the following table, for technical reasons, synthetic peptide numbers 1 A, 5A, 15A, 26A, 29A, 43A, and 56A differed slightly from the corresponding 15-mers targeted.

**Table AC**

| No. | Residues | Peptide Sequence | Seq. ID No. |
|---|---|---|---|
| 1A | 1-18 | F S R P G L P V E Y L Q V P S P S M | 153 |
| 5A | 21-36 | D I K V Q F Q S G G A N S P A L | 154 |
| 15A | 71-87 | P V G G Q S S F Y S D W Y Q P A C | 155 |
| 26A | 126-142 | S M A A S S A L T L A I Y H P Q Q | 156 |
| 29A | 140-157 | P Q Q F V Y A G A M S G L L D P S Q | 157 |
| 43A | 211-227 | C G N G K P S D L G G N N L P A K | 158 |
| 49A | 240-255 | F Q D A Y N A G G G H N G V F D | 159 |
| 56A | 276-289 | P D L Q R A L G A T P N T G | 160 |

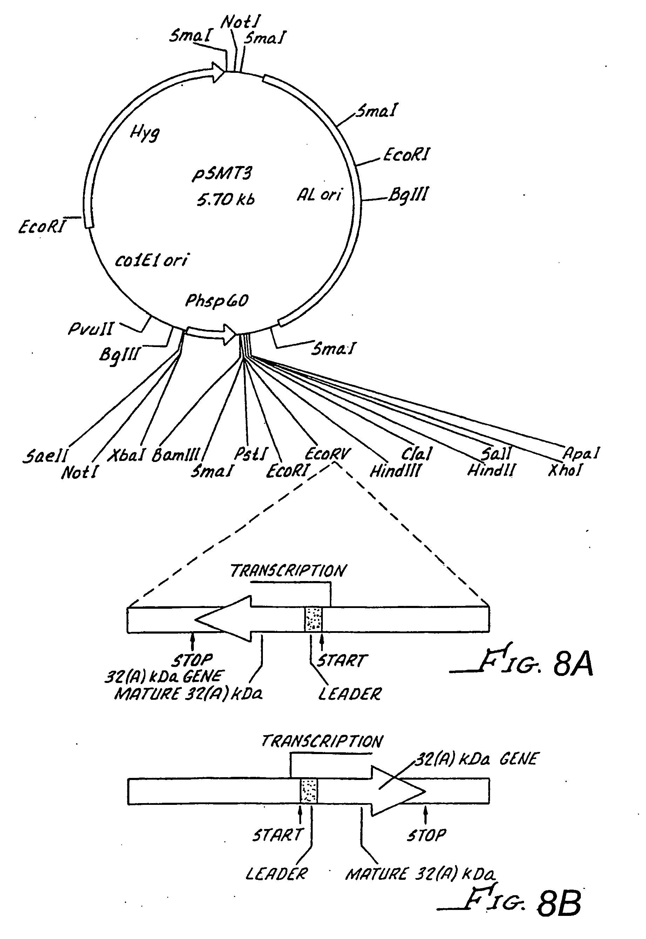

Immunodominant T-cell epitopes of the 32A KD major secretory protein of *M tuberculosis* recognized by greater than 25% of the guinea pigs immunized with purified *M. tuberculosis32A* KD protein are presented in Table AD below and graphically illustrated in Fig. 7.

**Table AD**

| Peptide* | Inclusive Amino Acids for Mature Protein | Sequence ID No. |
|---|---|---|
| 9 | 41 - 55 | 104 |
| 11 | 51 - 65 | 106 |
| 12 | 56 - 70 | 107 |
| 15 | 71 - 85 | 110 |
| 19 | 91 - 105 | 114 |
| 20 | 96 - 110 | 115 |
| 23 | 111 - 125 | 118 |
| 25 | 121 - 135 | 120 |
| 27 | 131 - 145 | 122 |
| 28 | 136 - 150 | 123 |
| 29 | 141 - 155 | 124 |
| 31 | 151 - 165 | 126 |
| 32 | 156 - 170 | 127 |
| 39 | 191 - 205 | 134 |
| 40 | 196 - 210 | 135 |
| 41 | 201 - 215 | 136 |
| 43 | 211 - 225 | 138 |

In accordance with the teachings of the present invention one or more of the immunodominant epitopes identified above can be incorporated into a vaccine against tuberculosis. For example, individual immunodominant epitopes can be synthesized and used individually or in combination in a multiple antigen peptide system. Alternatively, two or more immunodominant epitopes can be linked together chemically. The peptides, either linked together or separately, can be combined with an appropriate adjuvant and used in subunit vaccines for humans or other mammals. In addition, the immunodominant epitopes can be used in new immunodiagnostic reagents such as new skin tests.

Specific exemplary adjuvants used in the instant invention to enhance the activity of the selected immunogenic determinants are SAF, adjuvants containing Monophosphoryl Lipid A (MPL), Freund's incomplete adjuvant, Freund's complete adjuvant containing killed bacteria, gamma interferons (Radford et al., American Society of Hepatology 2008-2015, 1991; Watanabe et al., PNAS 86:9456-9460, 1989; Gansbacher et al., Cancer Research 50:7820-7825, 1990; Maio et al., Can. Immunol. Immunother. 30:34-42, 1989; U.S. Patent Nos. 4,762,791 and 4,727,138), IL-12, IL-15 (Grabstein et al., Science 264:965-968, 1994), MF 59, MF 59 plus MTP, MF 59 plus IL-12, MPL plus TDM (trehalose dimycolate), QS-21, QS-21 plus IL-12, IL-2 (American Type Culture Collection Nos. 39405, 39452 and 39516; see also U.S. Patent No. 4,518,584), dimethyldioctadecyl ammonium (ddA), ddA plus dextran, alum, Quil A, ISCOMS, (Immunostimulatory Complexes), Liposomes, Lipid Carriers, Protein Carriers, and Microencapsulation techniques. Additional adjuvants that may be useful in the present invention are water-in-oil emulsions, mineral salts (for example, alum), nucleic acids, block polymer surfactants, and microbial cell walls (peptido glycolipids). While not limiting the scope of the invention it is believed that adjuvants may magnify immune responses by allowing the slow release of antigens from the site of injection and/or modulation of the milieu at the site of injection including the cellular and cytokine constituents.

Particularly preferred is IL-12 either alone or in conjunction with another adjuvant. It has been found that immunization of guinea pigs with purified major *M. tuberculosis* extracellular proteins in the presence of IL-12 alone, or in the presence of IL-12 plus another adjuvant, for example, MF 59, enhances protective immunity over that obtained without IL-12. By increasing the capacity of the vaccine to induce protective immunity, IL-12 renders the vaccine more efficacious.

It was known that murine IL-12 stimulates both murine lymphoblasts and human lymphoblasts, and that human IL-12 stimulates human lymphoblasts, but it was not known if either mouse or human IL-12 stimulates guinea pig lymphoblasts. To determine if murine or human IL-12 stimulates guinea pig lymphoblasts, IL-12 activity was assayed using the protocol described in Current Protocols in Immunology, 1993, Cytokines and their Receptors entitled "Lymphoblasts Proliferation Assay for IL-12 Activity" Alternate Protocol), pages 6.16.3 through 6.16.5.

### Example 23

Spleen cells were isolated from a male Hartley strain guinea pig, incubated at a concentration of 10⁷ cells/75cm² culture flask containing 20 ml supplemented medium for 3 days with PHA at a concentration of 8 µg/ml, diluted 1:1 with supplemental medium, and incubated for 1 day with IL-2 at a concentration of 50 IU/ml. The lymphoblasts were washed, counted, dispensed into 96 well flat-bottom, microtiter plates at a cell density of 2x10⁴ lymphoblasts/well, and incubated for 24h with 0 to 5 µg/ml IL-12 (R&D Systems, Minneapolis, MN). ³H-thymidine (0.25 µCi) was added to each well for an additional 18h, and the cells then were harvested and assayed for incorporated ³H-thymidine.

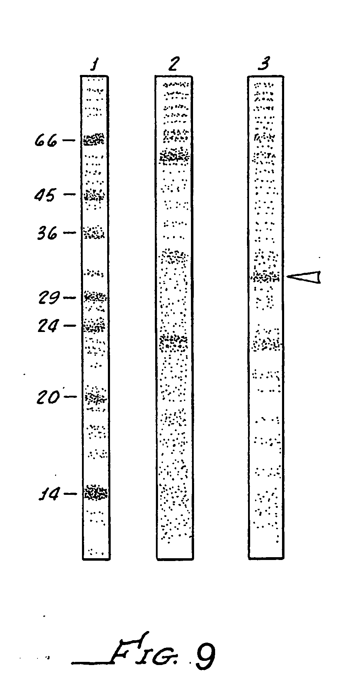

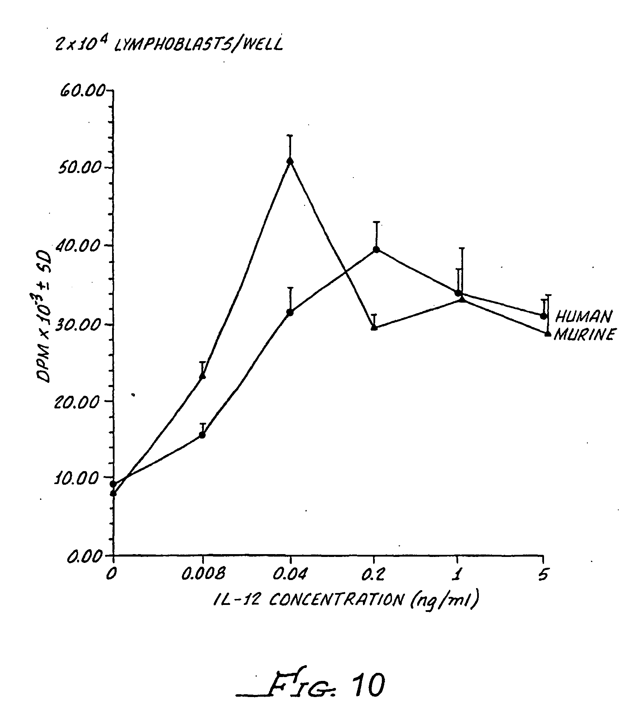

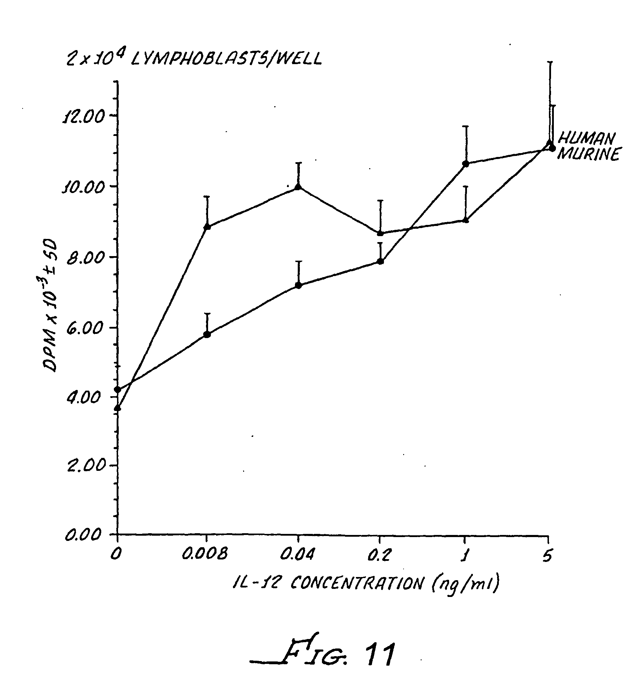

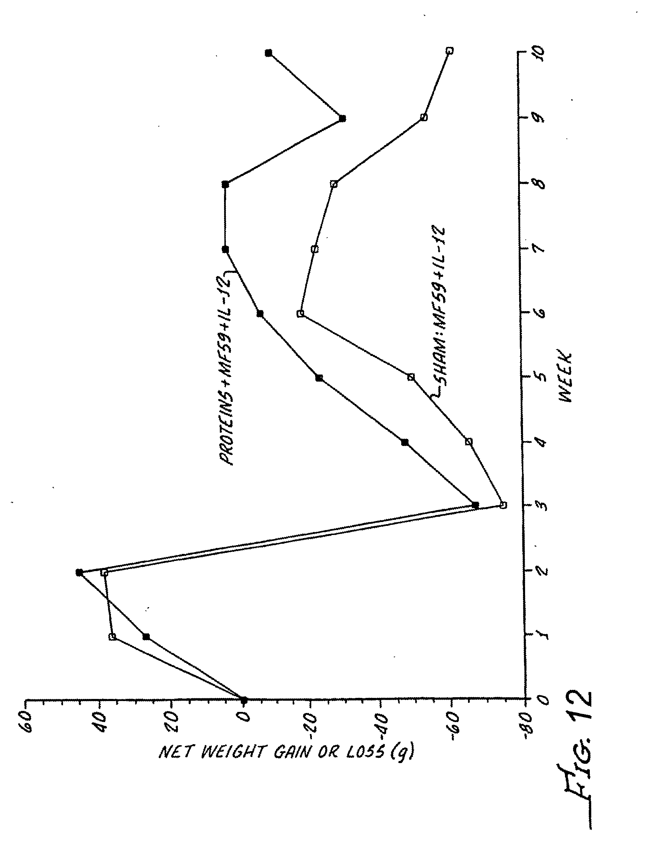

The results of two independent experiments using spleen cells from different outbred guinea pigs are shown in Fig. 10 and Fig. 11. Data are the mean DPM ± S.D. for sextuplicate wells.

In each experiment, both murine and human IL-12 strongly stimulated proliferation of guinea pig lymphoblasts in a dose-dependent fashion.

To determine if IL-12 would enhance the efficacy of a vaccine consisting of purified *M tuberculosis* 30, 32A, and 16KD major extracellular proteins, guinea pigs were immunized with the vaccine in the presence or absence of IL-12 and challenged with virulent *M tuberculosis* by aerosol. IL- 12 was found to enhance the capacity of the vaccine to protect the animals against weight loss and growth of *M. tuberculosis* in the lungs of challenged animals.

### Example 24

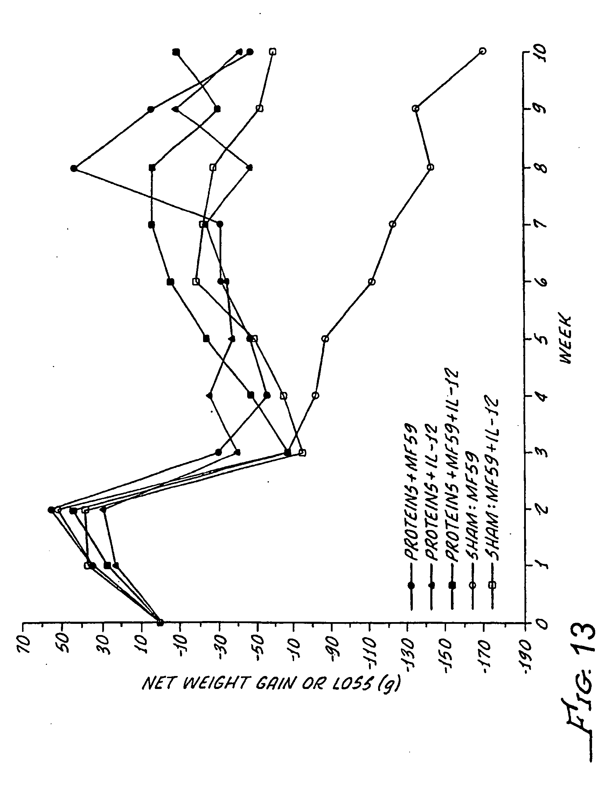

Guinea pigs (6 per group) were immunized intradermally three times with 100 µg of purified *M tuberculosis* 30, 32A and 16 KD major extracellular proteins in the presence of adjuvant (MF 59) and recombinant mouse IL-12 purchased from R&D Systems (Minneapolis, MN). Control animals (6 per group) were sham-immunized with adjuvant and IL-12 only. The animals were then challenged with an aerosol of virulent *M. tuberculosis* Erdman strain and weighed weekly thereafter until killed at 10 weeks. Sham-immunized animals exhibited greater weight loss than animals immunized with the proteins in the presence of adjuvant plus IL-12. The results are shown in Fig. 12.

### Example 25

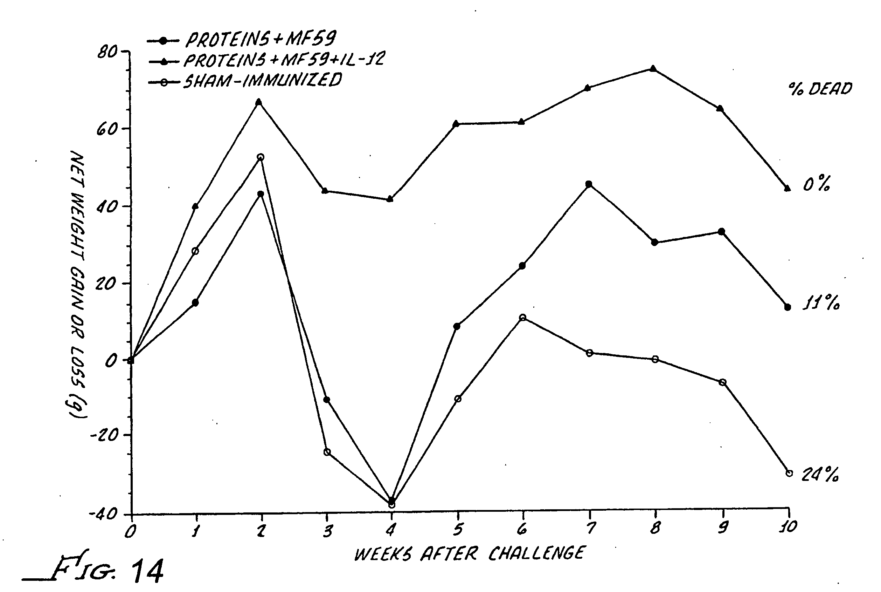

Guinea pigs (6 per group) were immunized three times intradermally with 100 µg of purified *M tuberculosis* 30, 32A, and 16 KD major extracellular proteins in the presence of adjuvant (MF 59) alone, IL-12 alone, or adjuvant +IL-12 or sham-immunized with adjuvant alone, or adjuvant +IL-12. IL-12 was purchased from R&D Systems. The animals were then challenged with virulent *M. tuberculosis* Erdman strain and weighed weekly thereafter until killed at 10 weeks. Fig. 13 shows the mean net weight gain or loss of each group from the weight on the.day of the challenge. Animals immunized with the proteins exhibited less weight loss than both groups of sham-immunized controls. Of the sham-immunized animals, animals immunized with adjuvant in the presence of IL-12 lost less weight than animals immunized with adjuvant only. Of the protein-immunized animals, animals immunized with proteins in the presence of adjuvant plus IL-12 exhibited less weight loss over the course of the experiment than animals immunized with the proteins in the presence of either IL-12 only or adjuvant only. Thus, during the critical period of disease from 4 to 10 weeks after challenge, animals immunized with proteins in the presence of both adjuvant and IL-12 exhibited the least amount of weight loss at weeks 5, 6, 7, and 10 and the second least amount of weight loss at weeks 4 and 8.

### Example 26

Animals were immunized three times intradermally with 100 µg of *M. tuberculosis* 30, 32A, and 16 KD major extracellular proteins in the presence of MF 59 or MF 59 + IL-12, or sham-immunized with MF 59 only, IL-12 only or MF 59 + IL-12. The animals were challenged with virulent *M tuberculosis* by aerosol, observed for 10 weeks, and killed. The right lung was removed and cultured for *M. tuberculosis* on 7H11 agar plates. Data shown in Table AE are the mean CFU per right lung.

**Table AE**

| | | |
|---|---|---|
| CFU in Lungs of Animals Immunized with 3 Purified Major Extracellular *M. tuberculosis* Proteins (30. 32A. 16 KD) in the Presence of Various Adjuvants | | |
| | | Mean log₁₀CFU |
| A. | Sham - Immunized (MF 59 only) | 7.7 |
| B. | Sham - Immunized (MF 59 + IL-12) | 7.0 |
| C. | Protein - Immunized (MF 59 only) | 7.0 |
| D. | Protein - Immunized (IL-12 only) | 6.9 |
| E. | Protein - Immunized (MF 59 + IL-12) | 6.6 |

Animals immunized with proteins had fewer CFU than animals sham-immunized without proteins but with the same adjuvant preparation. Thus, animals immunized with proteins plus MF 59 (C) had 0.7 log fewer CFU in the lungs than animals immunized with MF 59 only (A); and animals immunized with proteins with MF 59+IL-12 (E) had 0.4 log fewer CFU in the lungs than animals immunized with MF 59 + IL-12 only (B), and 1.1 log fewer CFU in the lungs than animals immunized with MF 59 only.

Animals immunized with proteins in the presence of IL-12 had fewer CFU than animals immunized with proteins in the absence of IL-12. Thus, animals immunized with proteins in MF 59 + IL-12 (E) had 0.4 log fewer CFU in the lungs than animals immunized with proteins in the presence of MF 59 only.

### Example 27

Guinea pigs were immunized intradermally three times with 100µg of purified *M*. *tuberculosis* 30, 32A, and 16KD major extracellular proteins in the presence of adjuvant (MF59) only (9 animals), or in the presence of adjuvant (MF59) plus recombinant mouse IL-12 (9 animals) purchased from R&D Systems (Minneapolis, MN). Control animals were sham-immunizedwith various adj uvants only (17 animals). The animals were then challenged with an aerosol of virulent *M. tuberculosis* Erdman strain and weighed weekly thereafter until killed at 10 weeks. Sham-immunized animals exhibited greater weight loss than animals immunized with the proteins in the presence of either adjuvant only or adjuvant plus IL-12. Of the animals immunized with proteins, animals immunized with proteins in the presence of adjuvant only lost more weight than animals immunized with proteins in the presence of adjuvant plus IL-12. Furthermore, sham-immunizedanimals had a higher mortality during the course of the experiment - 24% of the sham-immunized animals died vs. only 6% of animals immunized with proteins. Of the animals immunized with proteins, animals immunized with proteins in the presence of adjuvant only had a higher mortality (11 %) than animals immunized with proteins in the presence of adjuvant plus IL-12 (0%). The results are shown in Fig. 14.

Those skilled in the art will also appreciate that DNA encoding the peptides can be synthesized and used to express the peptides, individually or collectively, or can be combined in a DNA vaccine injected directly into humans or other mammals. A construct consisting of only the immunogenic epitopcs (or the DNA coding therefor) would focus the immune response on the protective portions of the molecule. By avoiding irrelevant or even immunosuppressiveepitopes such a construct may induce a stronger and more protective immune response.

Smaller protein subunits of the majorly abundant extracellularproducts, molecular analogs thereof, genes encoding therefore, and respective combinations thereof are within the scope of the present invention as long as they provoke effective immunoprophylaxisor function as an immunodiagnostic reagent. Moreover, recombinant protein products such as fusion proteins or extracellularproducts modified through known molecular recombinant techniques are entirely compatible with the teachings of the present invention. In addition, immunogenically generated analogs of the selected immunoactive determinants or peptides and nucleotides derived using directed evolution are also within the scope of the invention. Moreover, the selected immunoactive determinants can be modified so as to bind more tightly to specific MHC molecules of humans or other species or be presented more efficiently by antigen presenting cells. Further, the selected immunoactive determinants can be modified so as to resist degradation in the vaccinated host.

Similarly, the formulation and presentation of the immunogenic agent to the host immune system is not limited to solutions of proteins or their analogs in adjuvant. For example, the immunogenic determinant derived from the appropriate extracellularproteins may be expressed by *M. tuberculosis,* different species of *Mycobacteria,* different species of bacteria, phage, mycoplasma or virus that is nonpathogenic and modified using recombinant technology. In such cases the whole live organism may be formulated and used to stimulate the desired response. Conversely, large scale vaccination programs in hostile environments may require very stable formulations without complicating adjuvants or additives. Further, the vaccine formulation could be directed to facilitate the stability or immunoreactivity of the active component when subjected to harsh conditions such as lyophilizationor oral administration or encapsulation. Accordingly, the present invention encompasses vastly different formulations of the immunogenic determinants comprising the subject vaccines depending upon the intended use of the product.

Those skilled in the art will appreciate that vaccine dosages should be determined for each pathogen and host utilizing routine experimentation. At present, it is believed that the lowest practical dosage will be on the order of 0.1 µg though dosages of 2.0 µg, 20.0 µg, 100 µg and even 1 mg may be optimum for the appropriate system. The proper dosage can be administered using any conventional immunization technique and sequence known in the art.

## Claims

1. A vaccinating agent for use in promoting an effective immune response in a mammalian host, comprising a peptide consisting of one or more immunodominant epitopes selected from the group consisting of the amino acid sequences:
| Peptide Sequence | SEQ ID |
|---|---|
| G L R A Q D D F S G W D I N T | 104 |
| W D I N T P A F E W Y D Q S G | 106 |
| P A F E W Y D Q S G L S V V M | 107 |
| P V G G Q S S F Y S D W Y Q P | 110 |
| G C Q T Y K W E T F L T S E L | 114 |
| K W E T F L T S E L P G W L Q | 115 |
| A N R H V K P T G S A V V G L | 118 |
| A V V G L S M A A S S A L T L | 120 |
| S A L T L A I Y H P Q Q F V Y | 122 |
| A I Y H P Q Q F V Y A G A M S | 123 |
| Q Q F V Y A G A M S G L L D P | 124 |
| G L L D P S Q A M G P T L I G | 126 |
| S Q A M G P T L I G L A M G D | 127 |
| N D P L L N V G K L I A N N T | 134 |
| N V G K L I A N N T R V W V Y | 135 |
| I A N N T R V W V Y C G N G K | 136 |
| C G N G K P S D L G G N N L P | 138 |

2. The vaccinating agent of claim 1 wherein said agent further comprises M. *tuberculosis* 30kDa protein.

3. The vaccinating agent of claim 1 or 2, further comprising IL-12 or a mixture of IL-12 and MF 59.

4. An agent according to any preceding claim for use in a method of immunizing a mammalian host against an infectious pathogen of the genus *Mycobacterium,* said method comprising the step of introducing said vaccinating agent into said mammalian host to induce an effective immune response to subsequent infection by said infectious pathogen.

5. An immunodiagnosticagent for use in promoting a detectable immune response in a mammalian host for identifying an infections pathogen from the genus Mycobacterius, said agent comprising a peptide consisting of one or more immunodominantepitopes selected from the amino acid sequences
| Pepetide Sequence | SED ID |
|---|---|
| G L R A Q D D F S G W D I N T | 104 |
| W D I N T P A F E W Y D Q S G | 106 |
| P A F E W Y D Q S G L S V V M | 107 |
| P V G G Q S S F Y S D W Y Q P | 110 |
| G C Q T Y K W E T F L T S E L | 114 |
| K W E T F L T S E L P G W L Q | 115 |
| A N R H V K P T G S A V V G L | 118 |
| A V V G L S M A A S S A L T L | 120 |
| S A L T L A I Y H P Q Q F V Y | 122 |
| A I Y H P Q Q F V Y A G A M S | 123 |
| Q Q F V Y A G A M S G L L D P | 124 |
| G L L D P S Q A M G P T L I G | 126 |
| S Q A M G P T L I G L A M G D | 127 |
| N D P L L N V G K L I A N N T | 134 |
| N V G K L I A N N T R V W V Y | 135 |
| I A N N T R V W V Y C G N G K | 136 |
| C G N G K P S D L G G N N L P | 138 |

6. An agent according to claim 5 for use in a method for detecting the presence of an immune response in a mammal against a pathogen of the genus *Mycobacterium,* said method comprising the step of administering said agent to said mammal; and measuring the resultant immune response.

## Patentansprüche

1. Impfstoff zur Verwendung bei der Förderung einer wirksamen Immunreaktion in einem Säugerwirt, umfassend ein Peptid, welches aus einem oder mehr immunodominanten Epitopen besteht, welche aus der Gruppe, die aus den folgenden Aminosäuregruppen besteht, ausgewählt sind:
| Peptidsequenz | SEQ ID |
|---|---|
| G L R A Q D D F S G W D I N T | 104 |
| W D I N T P A F E W Y D Q S G | 106 |
| P A F E W Y D Q S G L S V V M | 107 |
| P V G G Q S S F Y S D W Y Q P | 110 |
| G C Q T Y K W E T F L T S E L | 114 |
| K W E T F L T S E L P G W L Q | 115 |
| A N R H V K P T G S A V V G L | 118 |
| A V V G L S M A A S S A L T L | 120 |
| S A L T L A I Y H P Q Q F V Y | 122 |
| A I Y H P Q Q F V Y A G A M S | 123 |
| Q Q F V Y A G A M S G L L D P | 124 |
| G L L D P S Q A M G P T L I G | 126 |
| D S Q A M G P T L I G L A M G D | 127 |
| N D P L L N V G K L I A N N T | 134 |
| N V G K L I A N N T R V W V Y | 135 |
| I A N N T R V W V Y C G N G K | 136 |
| C G N G K P S D L G G N N L P | 138 |

2. Impfstoff nach Anspruch 1, wobei der Stoff weiterhin das 30 kDa *M. tuber*culosis-Protein enthält.

3. Impfstoff nach Anspruch 1 oder 2, weiterhin enthaltend IL-12 oder eine Mischung aus IL-12 und MF 59.

4. Stoff gemäß einem der vorangehenden Ansprüche zur Verwendung in einem Verfahren zur Immunisierung eines Säugerwirts gegen ein infektiöses Pathogen der Gattung *Mycobacterium,* wobei das Verfahren den Schritt des Einführens des Impfstoffs in den Säugerwirt zur Erzeugung einer wirksamen Immunreaktion auf eine nachfolgende Infektion durch das infektiöse Pathogen umfasst.

5. Immunodiagnostisches Mittel zur Verwendung bei der Förderung einer nachweisbaren Immunreaktion in einem Säugerwirt zur Identifikation eines infektiösen Pathogens der Gattung Mycobacterius, wobei das Mittel ein Peptid enthält, welches aus einem oder mehr immunodominanten Epitopen besteht, welche aus der Gruppe, die aus den folgenden Aminosäuregruppen besteht, ausgewählt sind:
| Peptidsequenz | SEQ ID |
|---|---|
| G L R A O D D F S G W D I N T | 104 |
| W D I N T P A F E W Y D Q S G | 106 |
| P A F E W Y D Q S G L S V V M | 107 |
| P V G G Q S S F Y S D W Y Q P | 110 |
| G C Q T Y K W E T F L T S E L | 114 |
| K W E T F L T S E L P G W L Q | 115 |
| A N R H V K P T G S A V V G L | 118 |
| A V V G L S M A A S S A L T L | 120 |
| S A L T L A Y H P Q Q F V Y | 122 |
| A I Y H P Q Q F V Y A G A M S | 123 |
| Q Q F V Y A G A M S G L L D P | 124 |
| G L L D P S Q A M G P T L I G | 126 |
| S Q A M G P T L I G L A M G D | 127 |
| N D P L L N V G K L I A N N T | 134 |
| N V G K L I A N N T R V W V Y | 135 |
| I A N N T R V W V Y C G N G K | 136 |
| C G N G K P S D L G G N N L P | 138 |

6. Mittel gemäß Anspruch 5 zur Verwendung in einem Verfahren zum Nachweis des Vorhandenseins einer Immunreaktion gegen ein Pathogen der Gattung *Mycobacterium* in einem Säugerwirt, wobei das Verfahren den Schritt der Verabreichung des Mittels an den Säuger und Bestimmung der resultierenden Immunreaktion umfasst.

## Revendications

1. Agent vaccinant destiné à une utilisation pour favoriser une réponse immunitaire efficace chez un hôte mammalien, comprenant un peptide constitué d'un ou plusieurs épitopes immunodominants choisis dans le groupe constitué des séquences d'acides aminés :
| Séquence peptidique | SEQ ID |
|---|---|
| G L R A Q D D F S G W D I N T | 104 |
| W D I N T P A F E W Y D Q S G | 106 |
| P A F E W Y D Q S G L S V V M | 107 |
| P V G G Q S S F Y S D W Y O P | 110 |
| G C Q T Y K W E T F L T S E L | 114 |
| K W E T F L T S E L P G W L Q | 115 |
| A N R H V K P T G S A V V G L | 118 |
| A V V G L S M A A S S A L T L | 120 |
| S A L T L A I Y H P Q Q F V Y | 122 |
| A I Y H P Q Q F V Y A G A M S | 123 |
| Q Q F V Y A G A M S G L L O P | 124 |
| G L L D P S A M G P T L I G | 126 |
| S Q A M G P T L I G L A M G D | 127 |
| N D P L L N V G K L I A N N T | 134 |
| N V G K L I A N N T R V W V Y | 135 |
| I A N N T R V W V Y C G N G K | 136 |
| C G N G K P S D L G G N N L P | 138 |

2. Agent vaccinant selon la revendication 1, où ledit agent comprend en outre une protéine de 30 kDa de *M. tuberculosis.*

3. Agent vaccinant selon la revendication 1 ou 2, comprenant en outre de l'IL-12 ou un mélange d'IL-12 et de MF 59.

4. Agent selon l'une quelconque des revendication précédentes, pour une utilisation dans un procédé d'immunisation d'un hôte mammalien contre un agent pathogène infectieux du genre *Mycobacterium,* ledit procédé comprenant l'étape consistant à introduire ledit agent vaccinant chez ledit hôte mammalien pour provoquer une réponse immunitaire efficace contre une infection ultérieure par ledit agent pathogène infectieux.

5. Agent d'immunodiagnostic destiné à une utilisation pour favoriser une réponse immunitaire détectable chez un hôte mammalien pour identifier un agent pathogène infectieux du genre Mycobacterius, ledit agent comprenant un peptide constitué d'un ou plusieurs épitopes immunodominants choisis parmi les séquences d'acides aminés
| Séquence peptidique | SEQ ID |
|---|---|
| G L R A Q D D F S G W D I N T | 104 |
| W D I N T P A F E W Y D O S G | 106 |
| P A F E W Y D Q S G L S V V M | 107 |
| P V G G O S S F Y S D W Y O P | 110 |
| G C Q T Y K W E T F L T S E L | 114 |
| K W E T F L T S E L P G W L Q | 115 |
| A N R H V K P T G S A V V G L | 118 |
| A V V G L S M A A S S A L T L | 120 |
| S A L T L A I Y H P Q Q F V Y | 122 |
| A I Y H P Q Q F V Y A G A M S | 123 |
| Q O F V Y A G A M S G L L D P | 124 |
| G L L D P S Q A M G P T L I G | 126 |
| S Q A M G P T L T G L A M G D | 127 |
| N D P L L N V G K L I A N N T | 134 |
| N V G K L I A N N T R V W V Y | 135 |
| I A N N T R V W V Y C G N G K | 136 |
| C G N G K P S D L G G N N L P | 138 |

6. Agent selon la revendication 5 pour une utilisation dans un procédé de détection de la présence d'une réponse immunitaire chez un mammifère contre un agent pathogène du genre *Mycobacterium,* ledit procédé comprenant l'étape consistant à administrer ledit agent au dit mammifère ; et à mesurer la réponse immunitaire résultante.
